# EUROPEAN PATENT SPECIFICATION

(11) **EP 4 178 959 B1**
(45) Date of publication and mention of the grant of the patent: **10.09.2025**
(21) Application number: 20743578.5
(22) Date of filing: 13.07.2020
(51) Int. Cl.: C07D 473/34, A61P 35/00, A01N 43/54

(54) **NITROGEN HETEROCYCLIC CYTOKININ DERIVATIVES, COMPOSITIONS CONTAINING THESE DERIVATIVES AND USE THEREOF**
STICKSTOFFHETEROCYCLISCHE CYTOKININDERIVATE, DIESE ENTHALTENDE ZUSAMMENSETZUNGEN UND DEREN VERWENDUNG
DÉRIVÉS DE CYTOKININE HÉTÉROCYCLIQUES AZOTÉS, COMPOSITIONS CONTENANT CES DÉRIVÉS ET UTILISATION ASSOCIÉES

(43) Date of publication of application: 17.05.2023
(73) Proprietor: Univerzita Palackého v Olomouci, 779 00 Olomouc (CZ)
(72) Inventor: SKARUPA, Frantisek, 77900 Olomouc (CZ); PLIHALOVA, Lucie, 78302 Olomouc (CZ); ZATLOUKAL, Marek, 78701 Sumperk (CZ); DOLEZAL, Karel, 78365 Hlubocky (CZ); KOPRNA, Radoslav, 77900 Olomouc (CZ); GUCKY, Tomas, 78501 Mladejovice (CZ); PLIHAL, Ondrej, 78302 Olomouc (CZ); POSPISIL, Tomas, 77900 Olomouc (CZ); STRNAD, Miroslav, 77900 Olomouc (CZ)
(74) Representative: Harber IP s.r.o.
(86) International application number: PCT/CZ2020/050051
(87) International publication number: WO 2022/012702

(56) References cited:
- WO-A1-2010/130233
- WO-A1-2016/095881
- WO-A1-2017/036434
- WO-A1-2018/196893

## Description

### Technical Field

The invention relates to 2-fluoro-N⁶-substituted-9-(2-oxacycloalkyl)-9H-purine-6-amine derivatives, their use in agricultural and biotechnological applications, and compositions containing these derivatives.

### Background Art

Various adenine derivatives are known in the art to be versatile compounds with many interesting properties in medicine, cosmetics and agriculture (WO 2003/040144). Some of them occur naturally as plant hormones, e.g., kinetin, isopentenyladenine, *trans*-zeatin, dihydrozeatin, 6-benzylaminopurine, 6-(3-hydroxybenzylamino)purine (Strnad, M. Physiol. Plant. 1997, 101, 674; Wojtania and Gabryszewska, Biotechnologia 2004, 2, 162; Baroja-Fernández et al., Plant. Physiol. Biochem. 2002, 40, 217; Peixe et al., Scientia Horticulturae 2007, 113, 1). The application of these plant hormones to plants, however, brings significants undesired side effects, including problems with heterogeneity of the growth and rooting inhibition (Werbrouck et al., Physiol. Plant. 1996, 98, 291).

Substitution of purine ring at positions 2, 6, 8 and 9 by a wide range of substituents was carried out and these compounds were tested, e.g., substitution by benzyl and phenyl based substituents in position N6 (WO 03/040144), substitution by ribose at N9 (WO 2004/058791), substitution by less sterically demanding substituents at C2 (WO 2009/003428), substitution by 2-substituted benzyl substituents at N6 (WO 2009/043320). Most substitutions, however, did not result in removal of the undesirable effects.

WO 2008/008770 and WO 2009/086457 relate to 6,9-disubstituted adenine derivatives, bearing 2-tetrahydropyranyl or 2-tetrahydrofuranyl moiety at N9 atom, and to their use in cosmetics as antiinflammatory and antiaging components of skin care products. WO2018/196893 and WO2017/036434 disclose purine derivatives as agents for reducing the adverse effects of senescence in plants.

### Disclosure of the Invention

The present invention provides 2-fluoro-N⁶-substituted-9-(2-oxacycloalkyl)-9H-purine-6-amine derivatives bearing at N9 atom some 2-oxacycloalkyl moiety and their use for agricultural applications. Use of these compounds does not result in the undesired side effects such as root inhibition, which is the problem of many adenine-based compounds known heretofore.

The object of this invention are 2-fluoro-N⁶-substituted-9-(2-oxacycloalkyl)-9H-purine-6-amine derivatives of the general formula I, 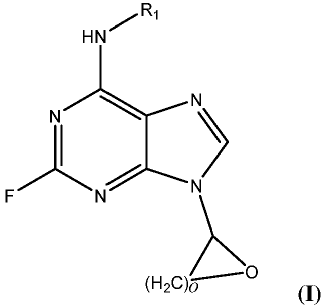 wherein
- *o* is an integer ranging from 2 to 5, i.e. 2 or 3 or 4 or 5, wherein one hydrogen in at least one methylene group (CH₂) in the substituent on N9 may be optionally replaced by a methyl and/or methoxy group, thus the substituent on N9 is selected from the group comprising oxetan-2-yl, tetrahydrofuran-2-yl, tetrahydro-2H-pyran-2-yl or oxepan-2-yl, optionally substituted with at least one methyl and/or methoxy group;
- R¹ is selected from the group containing
   - furfuryl,
   - furfuryl substituted with at least one, preferably exactly one, methyl or methoxy group,
   - benzyl,
   - benzyl substituted with at least one substituent, preferably with one or two or three substituents, independently selected from the group comprising methyl, trifluoromethyl, hydroxy, methoxy, trifluoromethoxy, halogen, amino, methoxycarbonyl and/or acetoxy,
   - 3-methylbut-2-en-1-yl,
   - 3-methylbut-3-en-1-yl,
   - 4-hydroxy-3-methylbut-2-en-1-yl, and
   - 4-hydroxy-3-methylbutyl,
or agriculturally acceptable salts thereof.

Agriculturally acceptable salts are in particular salts with alkali metals, ammonia or amines or addition salts with acids. The term "agriculturally acceptable salts" is intended to include agriculturally as well as biotechnologically acceptable salts.

"Halogen" refers to fluoro, chloro, bromo or iodo.

When chiral centers are present in the molecule, the present invention also includes optically active isomers, mixtures thereof and racemates.

In one preferred embodiment, R¹ is selected from the group containing furfuryl, 3-methylfurfuryl, 4-methylfurfuryl, 5-methylfurfuryl, 3-methoxyfurfuryl, 4-methoxyfurfuryl, 5-methoxyfurfuryl, benzyl, 2-methylbenzyl, 3-methylbenzyl, 4-methylbenzyl, 2-(trifluoromethyl)benzyl, 3-(trifluoromethyl)benzyl, 4-(trifluoromethyl)benzyl, 2-hydroxybenzyl, 3-hydroxybenzyl, 4-hydroxybenzylamino, 2-methoxybenzyl, 3-methoxybenzyl, 4-methoxybenzyl, 2-(trifluoromethoxy)benzyl, 3-(trifluoromethoxy)benzyl, 4-(trifluoromethoxy)benzyl, 2-fluorobenzyl, 3-fluorobenzyl, 4-fluorobenzyl, 2-chlorobenzyl, 3-chlorobenzyl, 4-chlorobenzyl, 2-bromobenzyl, 3-bromobenzyl, 4-bromobenzyl, 2-iodobenzyl, 3-iodobenzyl, 4-iodobenzyl, 2-aminobenzyl, 3-aminobenzyl, 4-aminobenzyl, 2-(methoxykarbonyl)benzyl, 3-(methoxykarbonyl)benzyl, 4-(methoxykarbonyl)benzyl, 2-acetoxybenzyl, 3-acetoxybenzyl, 4-acetoxybenzyl, 2,3-dihydroxybenzyl, 2,5-dihydroxybenzyl, 3,4-dihydroxybenzyl, 3,5-dihydroxybenzyl, 2,3-dimethoxybenzyl, 2,5-dimethoxybenzyl, 3,4-dimethoxybenzyl, 3,5-dimethoxybenzyl, 2,6-difluorobenzyl, 3,4-difluorobenzyl, 3,5-difluorobenzyl, 2,3-dichlorobenzyl, 2,4-dichlorobenzyl, 3,4-dichlorobenzyl, 3,5-dichlorobenzyl, 2-hydroxy-3-methylbenzyl, 2-hydroxy-5-methylbenzyl, 2-hydroxy-3-methoxybenzyl, 2-hydroxy-4-methoxybenzyl, 3-hydroxy-4-methoxybenzyl, 4-hydroxy-2-methoxybenzyl, 4-hydroxy-3-methoxybenzyl, 3-fluoro-4-hydroxybenzyl, 3-chloro-4-hydroxybenzyl, 4-fluoro-3-hydroxybenzyl, 4-chloro-3-hydroxybenzyl, 2-chloro-4-fluorobenzyl, 2-chloro-6-fluorobenzyl, 3,4,5-trihydroxybenzyl, 3,4,5-trimethoxybenzyl, 2,3,4-trifluorobenzyl, 2,3,6-trifluorobenzyl, 3,4,5-trifluorobenzyl, 4-hydroxy-3,5-dimethoxybenzyl, 3-methylbut-2-en-1-yl, 3-methylbut-3-en-1-yl, 4-hydroxy-3-methylbut-2-en-1-yl, -hydroxy-3-methylbutyl)amino.

In some embodiments, the substituent on N9 is selected from tetrahydro-2H-pyran-2-yl and tetrahydrofuran-2-yl, which may be optionally substituted with at least one methyl and/or methoxy group.

In some embodiments, the substituent on N9 is selected from tetrahydro-2H-pyran-2-yl and tetrahydrofuran-2-yl substituted with one methyl or methoxy group.

In the preferred embodiment, the 9-(2-oxacycloalkyl)-9*H*-purine-2-fluoro-6-amine derivatives of the general formula **I** are selected from the group comprising:
2-fluoro-6-furfurylamino-9-(oxetan-2-yl)-9H-purine, 2-fluoro-6-furfurylamino-9-(tetrahydrofuran-2-yl)-9H-purine, 2-fluoro-6-furfurylamino-9-(tetrahydro-2*H*-pyran-2-yl)-9H-purine, 2-fluoro-6-furfurylamino-9-(oxepan-2-yl)-9*H*-purine; 2-fluoro-6-[(3-methylfurfuryl)amino]-9-(oxetan-2-yl)-9-H-purine, 2-fluoro-6-[(3-methylfurfuryl)amino]-9-(tetrahydrofuran-2-yl)-9H-purine, 2-fluoro-6-[(3-methylfurfuryl)amino]-9-(tetrahydro-2*H*-pyran-2-yl)-9H-purine, 2-fluoro-6-[(3-methylfurfuryl)amino]-9-(oxepan-2-yl)-9*H*-purine; 2-fluoro-6-[(4-methylfurfuryl)amino]-9-(oxetan-2-yl)-9H-purine, 2-fluoro-6-[(4-methylfurfuryl)amino]-9-(tetrahydrofuran-2-yl)-9H-purine, 2-fluoro-6-[(4-methylfurfuryl)amino]-9-(tetrahydro-2*H*-pyran-2-yl)-9H-purine, 2-fluoro-6-[(4-methylfurfuryl)amino]-9-(oxepan-2-yl)-9*H*-purine, 2-fluoro-6-[(5-methylfurfuryl)amino]-9-(oxetan-2-yl)-9H-purine, 2-fluoro-6-[(5-methylfurfuryl)amino]-9-(tetrahydrofuran-2-yl)-9H-purine, 2-fluoro-6-[(5-methylfurfuryl)amino]-9-(tetrahydro-2*H*-pyran-2-yl)-9H-purine, 2-fluoro-6-[(5-methylfurfuryl)amino]-9-(oxepan-2-yl)-9*H*-purine; 2-fluoro-6-(3-methoxyfurfurylamino)-9-(oxetan-2-yl)-9H-purine, 2-fluoro-6-(3-methoxyfurfurylamino)-9-(tetrahydrofuran-2-yl)-9H-purine, 2-fluoro-6-(3-methoxyfurfurylamino)-9-(tetrahydro-2*H*-pyran-2-yl)-9H-purine, 2-fluoro-6-(3-methoxyfurfurylamino)-9-(oxepan-2-yl)-9*H*-purine; 2-fluoro-6-(4-methoxyfurfurylamino)-9-(oxetan-2-yl)-9H-purine, 2-fluoro-6-(4-methoxyfurfurylamino)-9-(tetrahydrofuran-2-yl)-9H-purine, 2-fluoro-6-(4-methoxyfurfurylamino)-9-(tetrahydro-2*H*-pyran-2-yl)-9H-purine, 2-fluoro-6-(4-methoxyfurfurylamino)-9-(oxepan-2-yl)-9*H*-purine; 2-fluoro-6-(5-methoxyfurfurylamino)-9-(oxetan-2-yl)-9H-purine, 2-fluoro-6-(5-methoxyfurfurylamino)-9-(tetrahydrofuran-2-yl)-9H-purine, 2-fluoro-6-(5-methoxyfurfurylamino)-9-(tetrahydro-2*H*-pyran-2-yl)-9H-purine, 2-fluoro-6-(5-methoxyfurfurylamino)-9-(oxepan-2-yl)-9*H*-purine; 2-fluoro-6-benzylamino-9-(oxetan-2-yl)-9H-purine, 2-fluoro-6-benzylamino-9-(tetrahydrofuran-2-yl)-9H-purine, 2-fluoro-6-benzylamino-9-(tetrahydro-2*H*-pyran-2-yl)9H-purine, 2-fluoro-6-benzylamino-9-(oxepan-2-yl)-9*H-*purine; 2-fluoro-6-[(2-methylbenzyl)amino]-9-(oxetan-2-yl)-9H-purine, 2-fluoro-6-[(2-methylbenzyl)amino]-9-(tetrahydrofuran-2-yl)-9H-purine, 2-fluoro-6-[(2-methylbenzyl)amino]-9-(tetrahydro-2*H*-pyran-2-yl)-9H-purine, 2-fluoro-6-[(2-methylbenzyl)amino]-9-(oxepan-2-yl)-9*H*-purine; 2-fluoro-6-[(3-methylbenzyl)amino]-9-(oxetan-2-yl)-9H-purine, 2-fluoro-6-[(3-methylbenzyl)amino]-9-(tetrahydrofuran-2-yl)-9H-purine, 2-fluoro-6-[(3-methylbenzyl)amino]-9-(tetrahydro-2*H*-pyran-2-yl)-9H-purine, 2-fluoro-6-[(3-methylbenzyl)amino]-9-(oxepan-2-yl)-9*H*-purine; 2-fluoro-6-[(4-methylbenzyl)amino]-9-(oxetan-2-yl)-9H-purine, 2-fluoro-6-[(4-methylbenzyl)amino]-9-(tetrahydrofuran-2-yl)-9H-purine, 2-fluoro-6-[(4-methylbenzyl)amino]-9-(tetrahydro-2*H*-pyran-2-yl)-9H-purine, 2-fluoro-6-[(4-methylbenzyl)amino]-9-(oxepan-2-yl)-9*H*-purine; 2-fluoro-6-{ [2-(trifluoromethyl)benzyl]amino }-9-(oxetan-2-yl)-9H-purine, 2-fluoro-6-{ [2-(trifluoromethyl)benzyl]amino }-9-(tetrahydrofuran-2-yl)-9H-purine, 2-fluoro-6-{[2-(trifluoromethyl)benzyl]amino}-9-(tetrahydro-2*H*-pyran-2-yl, oxepan-2-yl)-9*H*-purine; 2-fluoro-6-{[3-(trifluoromethyl)benzyl] amino }-9-(oxetan-2-yl)-9H-purine, 2-fluoro-6-{[3-(trifluoromethyl)benzyl]amino }-9-(tetrahydrofuran-2-yl)-9H-purine, 2-fluoro-6-{[3-(trifluoromethyl)benzyl]amino }-9-(tetrahydro-2*H*-pyran-2-yl)-9H-purine, 2-fluoro-6-{[3-(trifluoromethyl)benzyl]amino }-9-(oxepan-2-yl)-9*H*-purine; 2-fluoro-6-{ [4-(trifluoromethyl)benzyl] amino }-9-(oxetan-2-yl)-9H-purine, 2-fluoro-6-{[3-(trifluoromethyl)benzyl]amino }-9-(tetrahydrofuran-2-yl)-9H-purine, 2-fluoro-6-{[3-(trifluoromethyl)benzyl]amino}-9-(tetrahydro-2*H*-pyran-2-yl, oxepan-2-yl)-9*H*-purine; 2-fluoro-6-[(2-hydroxybenzyl)amino]-9-(oxetan-2-yl)-9H-purine, 2-fluoro-6-[(2-hydroxybenzyl)amino]-9-(tetrahydrofuran-2-yl)-9H-purine, 2-fluoro-6-[(2-hydroxybenzyl)amino]-9-(tetrahydro-2*H*-pyran-2-yl)-9H-purine, 2-fluoro-6-[(2-hydroxybenzyl)amino]-9-(oxepan-2-yl)-9*H*-purine; 2-fluoro-6-[(3-hydroxybenzyl)amino]-9-(oxetan-2-yl)-9H-purine, 2-fluoro-6-[(3-hydroxybenzyl)amino]-9-(tetrahydrofuran-2-yl)-9H-purine, 2-fluoro-6-[(3-hydroxybenzyl)amino]-9-(tetrahydro-2*H*-pyran-2-yl)-9H-purine, 2-fluoro-6-[(3-hydroxybenzyl)amino]-9-(oxepan-2-yl)-9*H*-purine; 2-fluoro-6-[(4-hydroxybenzyl)amino]-9-(oxetan-2-yl)-9H-purine, 2-fluoro-6-[(4-hydroxybenzyl)amino]-9-(tetrahydrofuran-2-yl)-9H-purine, 2-fluoro-6-[(4-hydroxybenzyl)amino]-9-(tetrahydro-2*H*-pyran-2-yl)-9H-purine, 2-fluoro-6-[(4-hydroxybenzyl)amino]-9-(oxepan-2-yl)-9*H*-purine; 2-fluoro-6-[(2-methoxybenzyl)amino]-9-(oxetan-2-yl)-9H-purine, 2-fluoro-6-[(2-methoxybenzyl)amino]-9-(tetrahydrofuran-2-yl)-9H-purine, 2-fluoro-6-[(2-methoxybenzyl)amino]-9-(tetrahydro-2*H*-pyran-2-yl)-9H-purine, 2-fluoro-6-[(2-methoxybenzyl)amino]-9-(oxepan-2-yl)-9*H*-purine; 2-fluoro-6-[(3-methoxybenzyl)amino]-9-(oxetan-2-yl)-9H-purine, 2-fluoro-6-[(2-methoxybenzyl)amino]-9-(tetrahydrofuran-2-yl)-9H-purine, 2-fluoro-6-[(2-methoxybenzyl)amino]-9-(tetrahydro-2*H*-pyran-2-yl, oxepan-2-yl)-9*H*-purine; 2-fluoro-6-[(4-methoxybenzyl)amino]-9-(oxetan-2-yl)-9H-purine, 2-fluoro-6-[(2-methoxybenzyl)amino]-9-(tetrahydrofuran-2-yl)-2H-purine, 2-fluoro-6-[(2-methoxybenzyl)amino]-9-(tetrahydro-2*H*-pyran-2-yl)-9H-purine, 2-fluoro-6-[(2-methoxybenzyl)amino]-9-(oxepan-2-yl)-9*H*-purine; 2-fluoro-6-{[2-(trifluoromethoxy)benzyl]amino}-9-(oxetan-2-yl)-9H-purine, 2-fluoro-6-{ [2-(trifluoromethoxy)benzyl]amino}-(tetrahydrofuran-2-yl)-9H-purine, 2-fluoro-6-{[2-(trifluoromethoxy)benzyl]amino}-(tetrahydro-2*H*-pyran-2-yl)-9H-purine, 2-fluoro-6-{[2-(trifluoromethoxy)benzyl]amino}-(oxepan-2-yl)-9*H*-purine; 2-fluoro-6-{ [3-(trifluoromethoxy)benzyl]amino}-9-(oxetan-2-yl)-9H-purine, 2-fluoro-6-{[3-(trifluoromethoxy)benzyl]amino}-9-(tetrahydrofuran-2-yl)-9H-purine, 2-fluoro-6-{[3-(trifluoromethoxy)benzyl]amino}-9-(tetrahydro-2*H*-pyran-2-yl)-9H-purine, 2-fluoro-6-{ [3-(trifluoromethoxy)benzyl]amino}-9-(oxepan-2-yl)-9*H*-purine; 2-fluoro-6-{[4-(trifluoromethoxy)benzyl] amino }-9-(oxetan-2-yl)-9H-purine, 2-fluoro-6-{[4-(trifluoromethoxy)benzyl]amino }-9-(tetrahydrofuran-2-yl)-9H-purine, 2-fluoro-6-{[4-(trifluoromethoxy)benzyl]amino}-9-(tetrahydro-2*H*-pyran-2-yl)-9H-purine, 2-fluoro-6-{[4-(trifluoromethoxy)benzyl]amino}-9-(oxepan-2-yl)-9*H*-purine; 2-fluoro-6-[(2-fluorobenzyl)amino]-9-(oxetan-2-yl)-9H-purine, 2-fluoro-6-[(2-fluorobenzyl)amino]-9-(tetrahydrofuran-2-yl)-9H-puirne, 2-fluoro-6-[(2-fluorobenzyl)amino]-9-(tetrahydro-2*H*-pyran-2-yl)-9H-purine, 2-fluoro-6-[(2-fluorobenzyl)amino]-9-(oxepan-2-yl)-9*H*-purine; 2-fluoro-6-[(3-fluorobenzyl)amino]-9-(oxetan-2-yl)-9H-purine, 2-fluoro-6-[(3-fluorobenzyl)amino]-9-(tetrahydrofuran-2-yl)-9H-purine, 2-fluoro-6-[(3-fluorobenzyl)amino]-9-(tetrahydro-2*H*-pyran-2-yl)-9H-purine, 2-fluoro-6-[(3-fluorobenzyl)amino]-9-(oxepan-2-yl)-9*H-*purine; 2-fluoro-6-[(4-fluorobenzyl)amino]-9-(oxetan-2-yl)-9H-purine, 2-fluoro-6-[(4-fluorobenzyl)amino]-9-(tetrahydrofuran-2-yl)-9H-purine, 2-fluoro-6-[(4-fluorobenzyl)amino]-9-(tetrahydro-2*H*-pyran-2-yl)-9H-purine, 2-fluoro-6-[(4-fluorobenzyl)amino]-9-(oxepan-2-yl)-9*H*-purine; 2-fluoro-6-[(2-chlorobenzyl)amino]-9-(oxetan-2-yl)-9H-purine, 2-fluoro-6-[(2-chlorobenzyl)amino]-9-(tetrahydrofuran-2-yl)-9H-purine, 2-fluoro-6-[(2-chlorobenzyl)amino]-9-(tetrahydro-2*H*-pyran-2-yl)-9H-purine, 2-fluoro-6-[(2-chlorobenzyl)amino]-9-(oxepan-2-yl)-9*H*-purine; 2-fluoro-6-[(3-chlorobenzyl)amino]-9-(oxetan-2-yl)-9H-purine, 2-fluoro-6-[(3-chlorobenzyl)amino]-9-(tetrahydrofuran-2-yl)-9H-purine, 2-fluoro-6-[(3-chlorobenzyl)amino]-9-(tetrahydro-2*H*-pyran-2-yl)-9H-purine, 2-fluoro-6-[(3-chlorobenzyl)amino]-9-(oxepan-2-yl)-9*H*-purine; 2-fluoro-6-[(4-chlorobenzyl)amino]-9-(oxetan-2-yl)-9H-purine, 2-fluoro-6-[(4-chlorobenzyl)amino]-9-(tetrahydrofuran-2-yl)-9H-purine, 2-fluoro-6-[(4-chlorobenzyl)amino]-9-(tetrahydro-2*H*-pyran-2-yl)-9H-purine, 2-fluoro-6-[(4-chlorobenzyl)amino]-9-(oxepan-2-yl)-9*H-*purine; 2-fluoro-6-[(2-bromobenzyl)amino]-9-(oxetan-2-yl)-9H-purine, 2-fluoro-6-[(2-bromobenzyl)amino]-9-(tetrahydrofuran-2-yl)-9H-purine, 2-fluoro-6-[(2-bromobenzyl)amino]-9-(tetrahydro-2*H*-pyran-2-yl)-9H-purine, 2-fluoro-6-[(2-bromobenzyl)amino]-9-(oxepan-2-yl)-9*H*-purine; 2-fluoro-6-[(3-bromobenzyl)amino]-9-(oxetan-2-yl)-9H-purine, 2-fluoro-6-[(3-bromobenzyl)amino]-9-(tetrahydrofuran-2-yl)-9H-purine, 2-fluoro-6-[(3-bromobenzyl)amino]-9-(tetrahydro-2*H*-pyran-2-yl)-9H-purine, 2-fluoro-6-[(3-bromobenzyl)amino]-9-(oxepan-2-yl)-9*H*-purine; 2-fluoro-6-[(4-bromobenzyl)amino]-9-(oxetan-2-yl)-9H-purine, 2-fluoro-6-[(4-bromobenzyl)amino]-9-(tetrahydrofuran-2-yl)-9H-purine, 2-fluoro-6-[(4-bromobenzyl)amino]-9-(tetrahydro-2*H*-pyran-2-yl)-9H-purine, 2-fluoro-6-[(4-bromobenzyl)amino]-9-(oxepan-2-yl)-9*H*-purine; 2-fluoro-6-[(2-iodobenzyl)amino]-9-(oxetan-2-yl)-9H-purine, 2-fluoro-6-[(2-iodobenzyl)amino]-9-(tetrahydrofuran-2-yl)-9H-purine, 2-fluoro-6-[(2-iodobenzyl)amino]-9-(tetrahydro-2*H*-pyran-2-yl)-9H-purine, 2-fluoro-6-[(2-iodobenzyl)amino]-9-(oxepan-2-yl)-9*H-*purine; 2-fluoro-6-[(3-iodobenzyl)amino]-9-(oxetan-2-yl)-9H-purine, 2-fluoro-6-[(3-iodobenzyl)amino]-9-(tetrahydrofuran-2-yl)-9H-purine, 2-fluoro-6-[(3-iodobenzyl)amino]-9-(tetrahydro-2*H*-pyran-2-yl)-9H-purine, 2-fluoro-6-[(3-iodobenzyl)amino]-9-(oxepan-2-yl)-9*H*-purine; 2-fluoro-6-[(4-iodobenzyl)amino]-9-(oxetan-2-yl)-9H-purine, 2-fluoro-6-[(4-iodobenzyl)amino]-9-(tetrahydrofuran-2-yl)-9H-purine, 2-fluoro-6-[(4-iodobenzyl)amino]-9-(tetrahydro-2*H*-pyran-2-yl, oxepan-2-yl)-9*H*-purine; 2-fluoro-6-[(2-aminobenzyl)amino]-9-(oxetan-2-yl)-9H-purine, 2-fluoro-6-[(2-aminobenzyl)amino]-9-(tetrahydrofuran-2-yl)-9H-purine, 2-fluoro-6-[(2-aminobenzyl)amino]-9-(tetrahydro-2*H*-pyran-2-yl)-9H-purine, 2-fluoro-6-[(2-aminobenzyl)amino]-9-(oxepan-2-yl)-9*H*-purine; 2-fluoro-6-[(3-aminobenzyl)amino]-9-(oxetan-2-yl)-9H-purine, 2-fluoro-6-[(3-aminobenzyl)amino]-9-(tetrahydrofuran-2-yl)-9H-purine, 2-fluoro-6-[(3-aminobenzyl)amino]-9-(tetrahydro-2*H*-pyran-2-yl)-9H-purine, 2-fluoro-6-[(3-aminobenzyl)amino]-9-(oxepan-2-yl)-9*H*-purine; 2-fluoro-6-[(4-aminobenzyl)amino]-9-(oxetan-2-yl)-9H-purine, 2-fluoro-6-[(4-aminobenzyl)amino]-9-(tetrahydrofuran-2-yl)-9H-purine, 2-fluoro-6-[(4-aminobenzyl)amino]-9-(tetrahydro-2*H*-pyran-2-yl)-9H-purine, 2-fluoro-6-[(4-aminobenzyl)amino]-9-(oxepan-2-yl)-9*H-*purine; 2-fluoro-6-{[2-(methoxycarbonyl)benzyl]amino}-9-(oxetan-2-yl)-9H-purine, 2-fluoro-6-{[2-(methoxycarbonyl)benzyl]amino}-9-(tetrahydrofuran-2-yl)-9H-purine, 2-fluoro-6-{ [2-(methoxycarbonyl)benzyl]amino} -9-(tetrahydro-2*H*-pyran-2-yl)-9H-purine, 2-fluoro-6-{[2-(methoxycarbonyl)benzyl]amino}-9-(oxepan-2-yl)-9*H*-purine; 2-fluoro-6-{ [3-(methoxycarbonyl)benzyl]amino } -9-(oxetan-2-yl)-9H-purine, 2-fluoro-6-{ [3-(methoxycarbonyl)benzyl]amino}-9-(tetrahydrofuran-2-yl)-9H-purine, 2-fluoro-6-{[3-(methoxycarbonyl)benzyl]amino}-9-(tetrahydro-2*H*-pyran-2-yl)-9H-purine, 2-fluoro-6-{[3-(methoxycarbonyl)benzyl]amino}-9-(oxepan-2-yl)-9*H*-purine; 2-fluoro-6-{[4-(methoxycarbonyl)benzyl]amino}-9-(oxetan-2-yl)-9H-purine, 2-fluoro-6-{[4-(methoxycarbonyl)benzyl]amino}-9-(tetrahydrofuran-2-yl)-9H-purine, 2-fluoro-6-{[4-(methoxycarbonyl)benzyl]amino}-9-(tetrahydro-2*H*-pyran-2-yl)-9H-purine, 2-fluoro-6-{[4-(methoxycarbonyl)benzyl]amino}-9-(oxepan-2-yl)-9*H*-purine; 2-fluoro-6-[(2-acetoxybenzyl)amino]-9-(oxetan-2-yl)-9H-purine, 2-fluoro-6-[(2-acetoxybenzyl)amino]-9-(tetrahydrofuran-2-yl)-9H-purine, 2-fluoro-6-[(2-acetoxybenzyl)amino]-9-(tetrahydro-2*H*-pyran-2-yl)-9H-purine, 2-fluoro-6-[(2-acetoxybenzyl)amino]-9-(oxepan-2-yl)-9*H*-purine; 2-fluoro-6-[(3-acetoxybenzyl)amino]-9-(oxetan-2-yl)-9H-purine, 2-fluoro-6-[(3-acetoxybenzyl)amino]-9-(tetrahydrofuran-2-yl)-9H-purine, 2-fluoro-6-[(3-acetoxybenzyl)amino]-9-(tetrahydro-2*H*-pyran-2-yl)-9H-purine, 2-fluoro-6-[(3-acetoxybenzyl)amino]-9-(oxepan-2-yl)-9*H-*purine; 2-fluoro-6-[(4-acetoxybenzyl)amino]-9-(oxetan-2-yl)-9H-purine, 2-fluoro-6-[(4-acetoxybenzyl)amino]-9-(tetrahydrofuran-2-yl)-9H-purine, 2-fluoro-6-[(4-acetoxybenzyl)amino]-9-(tetrahydro-2*H*-pyran-2-yl)-9H-purine, 2-fluoro-6-[(4-acetoxybenzyl)amino]-9-(oxepan-2-yl)-9*H*-purine; 2-fluoro-6-[(2,3-dihydroxybenzyl)amino]-9-(oxetan-2-yl)-9H-purine, 2-fluoro-6-[(2,3-dihydroxybenzyl)amino]-9-(tetrahydrofuran-2-yl)-9H-purine, 2-fluoro-6-[(2,3-dihydroxybenzyl)amino]-9-(tetrahydro-2*H*-pyran-2-yl)-9H-purine, 2-fluoro-6-[(2,3-dihydroxybenzyl)amino]-9-(oxepan-2-yl)-9*H-*purine; 2-fluoro-6-[(2,5-dihydroxybenzyl)amino]-9-(oxetan-2-yl)-9H-purine, 2-fluoro-6-[(2,5-dihydroxybenzyl)amino]-9-(tetrahydrofuran-2-yl)-9H-purine, 2-fluoro-6-[(2,5-dihydroxybenzyl)amino]-9-(tetrahydro-2*H*-pyran-2-yl)-9H-purine, oxepan-2-yl)-9*H*-purine; 2-fluoro-6-[(3,4-dihydroxybenzyl)amino]-9-(oxetan-2-yl)-9H-purine, 2-fluoro-6-[(3,4-dihydroxybenzyl)amino]-9-(tetrahydrofuran-2-yl)-9H-purine, 2-fluoro-6-[(3,4-dihydroxybenzyl)amino]-9-(tetrahydro-2*H*-pyran-2-yl)-9H-purine, 2-fluoro-6-[(3,4-dihydroxybenzyl)amino]-9-(oxepan-2-yl)-9*H*-purine; 2-fluoro-6-[(3,5-dihydroxybenzyl)amino]-9-(oxetan-2-yl)-9H-purine, 2-fluoro-6-[(3,5-dihydroxybenzyl)amino]-9-(tetrahydrofuran-2-yl)-9H-purine, 2-fluoro-6-[(3,5-dihydroxybenzyl)amino]-9-(tetrahydro-2*H*-pyran-2-yl)-9H-purine, 2-fluoro-6-[(3,5-dihydroxybenzyl)amino]-9-(oxepan-2-yl)-9*H*-purine; 2-fluoro-6-[(2,3-dimethoxybenzyl)amino]-9-(oxetan-2-yl)-9H-purine, 2-fluoro-6-[(2,3-dimethoxybenzyl)amino]-9-(tetrahydrofuran-2-yl)-9H-purine, 2-fluoro-6-[(2,3-dimethoxybenzyl)amino]-9-(tetrahydro-2*H*-pyran-2-yl)-9H-purine, 2-fluoro-6-[(2,3-dimethoxybenzyl)amino]-9-(oxepan-2-yl)-9*H*-purine; 2-fluoro-6-[(2,5-dimethoxybenzyl)amino]-9-(oxetan-2-yl)-9H-purine, 2-fluoro-6-[(2,5-dimethoxybenzyl)amino]-9-(tetrahydrofuran-2-yl)-9H-purine, 2-fluoro-6-[(2,5-dimethoxybenzyl)amino]-9-(tetrahydro-2*H*-pyran-2-yl)-9H-purine, 2-fluoro-6-[(2,5-dimethoxybenzyl)amino]-9-(oxepan-2-yl)-9*H*-purine; 2-fluoro-6-[(3,4-dimethoxybenzyl)amino]-9-(oxetan-2-yl)-9H-purine, 2-fluoro-6-[(3,4-dimethoxybenzyl)amino]-9-(tetrahydrofuran-2-yl)-9H-purine, 2-fluoro-6-[(3,4-dimethoxybenzyl)amino]-9-(tetrahydro-2*H*-pyran-2-yl)-9H-purine, 2-fluoro-6-[(3,4-dimethoxybenzyl)amino]-9-(oxepan-2-yl)-9*H*-purine; 2-fluoro-6-[(3,5-dimethoxybenzyl)amino]-9-(oxetan-2-yl)-9H-purine, 2-fluoro-6-[(3,5-dimethoxybenzyl)amino]-9-(tetrahydrofuran-2-yl)-9H-purine, 2-fluoro-6-[(3,5-dimethoxybenzyl)amino]-9-(etrahydro-2*H*-pyran-2-yl)-9H-purine, 2-fluoro-6-[(3,5-dimethoxybenzyl)amino]-9-(oxepan-2-yl)-9*H*-purine; 2-fluoro-6-[(2,6-difluorobenzyl)amino]-9-(oxetan-2-yl)-9H-purine, 2-fluoro-6-[(2,6-difluorobenzyl)amino]-9-(tetrahydrofuran-2-yl)-9H-purine, 2-fluoro-6-[(2,6-difluorobenzyl)amino]-9-(tetrahydro-2*H*-pyran-2-yl)-9H-purine, 2-fluoro-6-[(2,6-difluorobenzyl)amino]-9-(oxepan-2-yl)-9*H*-purine; 2-fluoro-6-[(3,4-difluorobenzyl)amino]-9-(oxetan-2-yl)-9H-purine, 2-fluoro-6-[(3,4-difluorobenzyl)amino]-9-(tetrahydrofuran-2-yl)-9H-purine, 2-fluoro-6-[(3,4-difluorobenzyl)amino]-9-(tetrahydro-2*H*-pyran-2-yl)-9H-purine, 2-fluoro-6-[(3,4-difluorobenzyl)amino]-9-(oxepan-2-yl)-9*H*-purine; 2-fluoro-6-[(3,5-difluorobenzyl)amino]-9-(oxetan-2-yl)-9H-purine, ; 2-fluoro-6-[(3,5-difluorobenzyl)amino]-9-(tetrahydrofuran-2-yl)-9H-purine, ; 2-fluoro-6-[(3,5-difluorobenzyl)amino]-9-(tetrahydro-2*H*-pyran-2-yl)-9H-purine, 2-fluoro-6-[(3,5-difluorobenzyl)amino]-9-(oxepan-2-yl)-9*H*-purine; 2-fluoro-6-[(2,3-dichlorobenzyl)amino]-9-(oxetan-2-yl)-9H-purine, 2-fluoro-6-[(2,3-dichlorobenzyl)amino]-9-(tetrahydrofuran-2-yl)-9H-purine, 2-fluoro-6-[(2,3-dichlorobenzyl)amino]-9-(tetrahydro-2*H*-pyran-2-yl)-9H-purine, 2-fluoro-6-[(2,3-dichlorobenzyl)amino]-9-(oxepan-2-yl)-9*H*-purine; 2-fluoro-6-[(2,4-dichlorobenzyl)amino]-9-(oxetan-2-yl)-9H-purine, 2-fluoro-6-[(2,4-dichlorobenzyl)amino]-9-(tetrahydrofuran-2-yl)-9H-purine, 2-fluoro-6-[(2,4-dichlorobenzyl)amino]-9-(tetrahydro-2*H*-pyran-2-yl)-9H-purine, 2-fluoro-6-[(2,4-dichlorobenzyl)amino]-9-(oxepan-2-yl)-9*H*-purine; 2-fluoro-6-[(3,4-dichlorobenzyl)amino]-9-(oxetan-2-yl)-9H-purine, 2-fluoro-6-[(3,4-dichlorobenzyl)amino]-9-(tetrahydrofuran-2-yl)-9H-purine, 2-fluoro-6-[(3,4-dichlorobenzyl)amino]-9-(tetrahydro-2*H*-pyran-2-yl)-9H-purine, 2-fluoro-6-[(3,4-dichlorobenzyl)amino]-9-(oxepan-2-yl)-9*H*-purine; 2-fluoro-6-[(3,5-dichlorobenzyl)amino]-9-(oxetan-2-yl)-9H-purine, 2-fluoro-6-[(3,5-dichlorobenzyl)amino]-9-(tetrahydrofuran-2-yl)-9H-purine, 2-fluoro-6-[(3,5-dichlorobenzyl)amino]-9-(tetrahydro-2*H*-pyran-2-yl)-9H-purine, 2-fluoro-6-[(3,5-dichlorobenzyl)amino]-9-(oxepan-2-yl)-9*H*-purine; 2-fluoro-6-[(2-hydroxy-3-methylbenzyl)amino]-9-(oxetan-2-yl)-9H-purine, 2-fluoro-6-[(2-hydroxy-3-methylbenzyl)amino]-9-(tetrahydrofuran-2-yl)-9H-purine, 2-fluoro-6-[(2-hydroxy-3-methylbenzyl)amino]-9-(tetrahydro-2*H-*pyran-2-yl)-9H-purine, 2-fluoro-6-[(2-hydroxy-3-methylbenzyl)amino]-9-(oxepan-2-yl)-9*H*-purine; 2-fluoro-6-[(2-hydroxy-5-methylbenzyl)amino]-9-(oxetan-2-yl)-9H-purine, 2-fluoro-6-[(2-hydroxy-5-methylbenzyl)amino]-9-(tetrahydrofuran-2-yl)-9H-purine, 2-fluoro-6-[(2-hydroxy-5-methylbenzyl)amino]-9-(tetrahydro-2*H*-pyran-2-yl)-9H-purine, 2-fluoro-6-[(2-hydroxy-5-methylbenzyl)amino]-9-(oxepan-2-yl)-9*H*-purine; 2-fluoro-6-[(2-hydroxy-3-methoxybenzyl)amino]-9-(oxetan-2-yl)-9H-purine, 2-fluoro-6-[(2-hydroxy-3-methoxybenzyl)amino]-9-(tetrahydrofuran-2-yl)-9H-purine, 2-fluoro-6-[(2-hydroxy-3-methoxybenzyl)amino]-9-(tetrahydro-2*H*-pyran-2-yl)-9H-purine, 2-fluoro-6-[(2-hydroxy-3-methoxybenzyl)amino]-9-(oxepan-2-yl)-9*H*-purine; 2-fluoro-6-[(2-hydroxy-4-methoxybenzyl)amino]-9-(oxetan-2-yl)-9H-purine, 2-fluoro-6-[(2-hydroxy-4-methoxybenzyl)amino]-9-(tetrahydrofuran-2-yl)-9H-purine, 2-fluoro-6-[(2-hydroxy-4-methoxybenzyl)amino]-9-(tetrahydro-2*H-*pyran-2-yl)-9H-purine, 2-fluoro-6-[(2-hydroxy-4-methoxybenzyl)amino]-9-(oxepan-2-yl)-9*H*-purine; 2-fluoro-6-[(3-hydroxy-4-methoxybenzyl)amino]-9-(oxetan-2-yl)-9H_purine, 2-fluoro-6-[(3-hydroxy-4-methoxybenzyl)amino]-9-(tetrahydrofuran-2-yl)-9H-purine, 2-fluoro-6-[(3-hydroxy-4-methoxybenzyl)amino]-9-(tetrahydro-2*H*-pyran-2-yl)-9H-purine, 2-fluoro-6-[(3-hydroxy-4-methoxybenzyl)amino]-9-(oxepan-2-yl)-9*H*-purine; 2-fluoro-6-[(4-hydroxy-2-methoxybenzyl)amino]-9-(oxetan-2-yl)-9H-purine, 2-fluoro-6-[(4-hydroxy-2-methoxybenzyl)amino]-9-(tetrahydrofuran-2-yl)-9H-purine, 2-fluoro-6-[(4-hydroxy-2-methoxybenzyl)amino]-9-(tetrahydro-2*H*-pyran-2-yl)-9H-purine, 2-fluoro-6-[(4-hydroxy-2-methoxybenzyl)amino]-9-(oxepan-2-yl)-9*H*-purine; 2-fluoro-6-[(4-hydroxy-3-methoxybenzyl)amino]-9-(oxetan-2-yl)-9H-purine, 2-fluoro-6-[(4-hydroxy-3-methoxybenzyl)amino]-9-(tetrahydrofuran-2-yl)-9H-purine, 2-fluoro-6-[(4-hydroxy-3-methoxybenzyl)amino]-9-(tetrahydro-2*H-*pyran-2-yl)-9H-purine, 2-fluoro-6-[(4-hydroxy-3-methoxybenzyl)amino]-9-(oxepan-2-yl)-9*H*-purine; 2-fluoro-6-[(3-fluoro-4-hydroxybenzyl)amino]-9-(oxetan-2-yl)-9H-purine, 2-fluoro-6-[(3-fluoro-4-hydroxybenzyl)amino]-9-(tetrahydrofuran-2-yl)-9H-purine, 2-fluoro-6-[(3-fluoro-4-hydroxybenzyl)amino]-9-(tetrahydro-2*H*-pyran-2-yl)-9H-purine, 2-fluoro-6-[(3-fluoro-4-hydroxybenzyl)amino]-9-oxepan-2-yl)-9*H*-purine; 2-fluoro-6-[(3-chloro-4-hydroxybenzyl)amino]-9-(oxetan-2-yl)-9H-purine, 2-fluoro-6-[(3-chloro-4-hydroxybenzyl)amino]-9-(tetrahydrofuran-2-yl)-9H-purine, 2-fluoro-6-[(3-chloro-4-hydroxybenzyl)amino]-9-(tetrahydro-2*H*-pyran-2-yl)-9H-purine, 2-fluoro-6-[(3-chloro-4-hydroxybenzyl)amino]-9-(oxepan-2-yl)-9*H*-purine; 2-fluoro-6-[(4-fluoro-3-hydroxybenzyl)amino]-9-(oxetan-2-yl)-9H-purine, 2-fluoro-6-[(4-fluoro-3-hydroxybenzyl)amino]-9-(tetrahydrofuran-2-yl)-9H-purine, 2-fluoro-6-[(4-fluoro-3-hydroxybenzyl)amino]-9-(tetrahydro-2*H-*pyran-2-yl)-9H-purine, 2-fluoro-6-[(4-fluoro-3-hydroxybenzyl)amino]-9-(oxepan-2-yl)-9*H*-purine; 2-fluoro-6-[(4-chloro-3-hydroxybenzyl)amino]-9-(oxetan-2-yl)-9H-purine, 2-fluoro-6-[(4-chloro-3-hydroxybenzyl)amino]-9-(tetrahydrofuran-2-yl)-9H-purine, 2-fluoro-6-[(4-chloro-3-hydroxybenzyl)amino]-9-(tetrahydro-2*H*-pyran-2-yl)-9H-purine, 2-fluoro-6-[(4-chloro-3-hydroxybenzyl)amino]-9-(oxepan-2-yl)-9H-purine; 2-fluoro-6-[(2-chloro-4-fluorobenzyl)amino]-9-(oxetan-2-yl)-9H-purine, 2-fluoro-6-[(2-chloro-4-fluorobenzyl)amino]-9-(tetrahydrofuran-2-yl)-9H-purine, 2-fluoro-6-[(2-chloro-4-fluorobenzyl)amino]-9-(tetrahydro-2*H*-pyran-2-yl)-9H-purine, 2-fluoro-6-[(2-chloro-4-fluorobenzyl)amino]-9-(oxepan-2-yl)-9*H*-purine; 2-fluoro-6-[(2-chloro-6-fluorobenzyl)amino]-9-(oxetan-2-yl)-9H-purine, 2-fluoro-6-[(2-chloro-6-fluorobenzyl)amino]-9-(tetrahydrofuran-2-yl)-9H-purine, 2-fluoro-6-[(2-chloro-6-fluorobenzyl)amino]-9-(tetrahydro-2*H*-pyran-2-yl)-9H-purine, 2-fluoro-6-[(2-chloro-6-fluorobenzyl)amino]-9-(oxepan-2-yl)-9*H*-purine; 2-fluoro-6-[(3,4,5-trihydroxybenzyl)amino]-9-(oxetan-2-yl)-9H-purine, 2-fluoro-6-[(3,4,5-trihydroxybenzyl)amino]-9-(tetrahydrofuran-2-yl)-9H-purine, 2-fluoro-6-[(3,4,5-trihydroxybenzyl)amino]-9-(tetrahydro-2*H*-pyran-2-yl)-9H-purine, 2-fluoro-6-[(3,4,5-trihydroxybenzyl)amino]-9-(oxepan-2-yl)-9*H*-purine; 2-fluoro-6-[(3,4,5-trimethoxybenzyl)amino]-9-(oxetan-2-yl)-9H-purine, 2-fluoro-6-[(3,4,5-trimethoxybenzyl)amino]-9-(tetrahydrofuran-2-yl)-9H-purine, 2-fluoro-6-[(3,4,5-trimethoxybenzyl)amino]-9-(tetrahydro-2*H*-pyran-2-yl)-9H-purine, 2-fluoro-6-[(3,4,5-trimethoxybenzyl)amino]-9-(oxepan-2-yl)-9*H*-purine; 2-fluoro-6-[(2,3,4-trifluorobenzyl)amino]-9-(oxetan-2-yl)-9H-purine, 2-fluoro-6-[(2,3,4-trifluorobenzyl)amino]-9- (tetrahydrofuran-2-yl)-9H-purine, 2-fluoro-6-[(2,3,4-trifluorobenzyl)amino]-9-(tetrahydro-2*H*-pyran-2-yl)-9H-purine, 2-fluoro-6-[(2,3,4-trifluorobenzyl)amino]-9-(oxepan-2-yl)-9*H*-purine; 2-fluoro-6-[(2,3,6-trifluorobenzyl)amino]-9-(oxetan-2-yl)-9H-purine, 2-fluoro-6-[(2,3,6-trifluorobenzyl)amino]-9-(tetrahydrofuran-2-yl)-9H-purine, 2-fluoro-6-[(2,3,6-trifluorobenzyl)amino]-9-(tetrahydro-2*H*-pyran-2-yl)-9H-purine, 2-fluoro-6-[(2,3,6-trifluorobenzyl)amino]-9-(oxepan-2-yl)-9*H*-purine; 2-fluoro-6-[(3,4,5-trifluorobenzyl)amino]-9-(oxetan-2-yl)-9H-purine, 2-fluoro-6-[(3,4,5-trifluorobenzyl)amino]-9-(tetrahydrofuran-2-yl)-9H-purine, 2-fluoro-6-[(3,4,5-trifluorobenzyl)amino]-9-(tetrahydro-2*H*-pyran-2-yl)-9H-purine, oxepan-2-yl)-9*H*-purine; 2-fluoro-6-[(4-hydroxy-3,5-dimethoxybenzyl)amino]-9-(oxetan-2-yl, tetrahydrofuran-2-yl)-9H-purine, 2-fluoro-6-[(4-hydroxy-3,5-dimethoxybenzyl)amino]-9-(tetrahydro-2*H*-pyran-2-yl)-9H-purine, 2-fluoro-6-[(4-hydroxy-3,5-dimethoxybenzyl)amino]-9-(oxepan-2-yl)-9*H*-purine; 2-fluoro-6-(2-methoxyanilino)-9-(oxetan-2-yl)-9H-purine, 2-fluoro-6-(2-methoxyanilino)-9-(tetrahydrofuran-2-yl)-9H-purine, 2-fluoro-6-(2-methoxyanilino)-9-(tetrahydro-2H-pyran-2-yl)-9H-purine, 2-fluoro-6-(2-methoxyanilino)-9-(oxepan-2-yl)-9H-purine, 2-fluoro-6-(3-methoxyanilino)-9-(oxetan-2-yl)-9H-purine, 2-fluoro-6-(3-methoxyanilino)-9-(tetrahydrofuran-2-yl)-9H-purine, 2-fluoro-6-(3-methoxyanilino)-9-(tetrahydro-2H-pyran-2-yl)-9H-purine, 2-fluoro-6-(3-methoxyanilino)-9-(oxepan-2-yl)-9H-purine, 2-fluoro-6-(4-methoxyanilino)-9-(oxetan-2-yl)-9H-purine, 2-fluoro-6-(4-methoxyanilino)-9-(tetrahydrofuran-2-yl)-9H-purine, 2-fluoro-6-(4-methoxyanilino)-9-(tetrahydro-2H-pyran-2-yl)-9H-purine, 2-fluoro-6-(4-methoxyanilino)-9-(oxepan-2-yl)-9H-purine, 2-fluoro-6-(2,3-dimethoxyanilino)-9-(oxetan-2-yl)-9H-purine, 2-fluoro-6-(2,3-dimethoxyanilino)-9-(tetrahydrofuran-2-yl)-9H-purine, 2-fluoro-6-(2,3-dimethoxyanilino)-9-(tetrahydro-2H-pyran-2-yl)-9H-purine, 2-fluoro-6-(2,3-dimethoxyanilino)-9-(oxepan-2-yl)-9H-purine, 2-fluoro-6-(2,4-dimethoxyanilino)-9-(oxetan-2-yl)-9H-purine, 2-fluoro-6-(2,4-dimethoxyanilino)-9-(tetrahydrofuran-2-yl)-9H-purine, 2-fluoro-6-(2,4-dimethoxyanilino)-9-(tetrahydro-2H-pyran-2-yl)-9H-purine, 2-fluoro-6-(2,4-dimethoxyanilino)-9-(oxepan-2-yl)-9H-purine, 2-fluoro-6-(2,5-dimethoxyanilino)-9-(oxetan-2-yl)-9H-purine, 2-fluoro-6-(2,5-dimethoxyanilino)-9-(tetrahydrofuran-2-yl)-9H-purine, 2-fluoro-6-(2,5-dimethoxyanilino)-9-(tetrahydro-2H-pyran-2-yl)-9H-purine, 2-fluoro-6-(2,5-dimethoxyanilino)-9-(oxepan-2-yl)-9H-purine, 2-fluoro-6-(3,5-dimethoxyanilino)-9- (oxetan-2-yl)-9H-purine, 2-fluoro-6-(3,5-dimethoxyanilino)-9-(tetrahydrofuran-2-yl)-9H-purine, 2-fluoro-6-(3,5-dimethoxyanilino)-9- (tetrahydro-2H-pyran-2-yl)-9H-purine, 2-fluoro-6-(3,5-dimethoxyanilino)-9- (oxepan-2-yl)-9H-purine, 2-fluoro-6-(2,3,4-trimethoxyanilino)-9-(oxetan-2-yl)-9H-purine, 2-fluoro-6-(2,3,4-trimethoxyanilino)-9-(tetrahydrofuran-2-yl)-9H-purine, 2-fluoro-6-(2,3,4-trimethoxyanilino)-9-(tetrahydro-2H-pyran-2-yl)-9H-purine, 2-fluoro-6-(2,3,4-trimethoxyanilino)-9-(oxepan-2-yl)-9H-purine, 2-fluoro-6-(2,3,5-trimethoxyanilino)-9-(oxetan-2-yl)-9H-purine, 2-fluoro-6-(2,3,5-trimethoxyanilino)-9-(tetrahydrofuran-2-yl)-9H-purine, 2-fluoro-6-(2,3,5-trimethoxyanilino)-9-(tetrahydro-2H-pyran-2-yl)-9H-purine, 2-fluoro-6-(2,3,5-trimethoxyanilino)-9-(oxepan-2-yl)-9H-purine, 2-fluoro-6-(4-hydroxy-2-methylanilino)-9-(oxetan-2-yl)-9H-purine, 2-fluoro-6-(4-hydroxy-2-methylanilino)-9-(tetrahydrofuran-2-yl)-9H-purine, 2-fluoro-6-(4-hydroxy-2-methylanilino)-9-(tetrahydro-2H-pyran-2-yl)-9H-purine, 2-fluoro-6-(4-hydroxy-2-methylanilino)-9-(oxepan-2-yl)-9H-purine, 2-fluoro-6-(4-hydroxy-3-methylanilino)-9-(oxetan-2-yl)-9H-purine, 2-fluoro-6-(4-hydroxy-3-methylanilino)-9-(tetrahydrofuran-2-yl)-9H-purine, 2-fluoro-6-(4-hydroxy-3-methylanilino)-9-(tetrahydro-2H-pyran-2-yl)-9H-purine, 2-fluoro-6-(4-hydroxy-3-methylanilino)-9-(oxepan-2-yl)-9H-purine, 2-fluoro-6-(4-hydroxy-6-methylanilino)-9-(oxetan-2-yl)-9H_purine, 2-fluoro-6-(4-hydroxy-6-methylanilino)-9-(tetrahydrofuran-2-yl)-9H-purine, 2-fluoro-6-(4-hydroxy-6-methylanilino)-9-(tetrahydro-2H-pyran-2-yl)-9H-purine, 2-fluoro-6-(4-hydroxy-6-methylanilino)-9-(oxepan-2-yl)-9H-purine, 2-fluoro-6-(4-hydroxy-2-methoxyanilino)-9-(oxetan-2-yl)-9H-purine, 2-fluoro-6-(4-hydroxy-2-methoxyanilino)-9-(tetrahydrofuran-2-yl)-9H-purine, 2-fluoro-6-(4-hydroxy-2-methoxyanilino)-9-(tetrahydro-2H-pyran-2-yl)-9H-purine, 2-fluoro-6-(4-hydroxy-2-methoxyanilino)-9-(oxepan-2-yl)-9H-purine, 2-fluoro-6-(4-hydroxy-3-methoxyanilino)-9-(oxetan-2-yl)-9H-purine, 2-fluoro-6-(4-hydroxy-3-methoxyanilino)-9-(tetrahydrofuran-2-yl)-9H-purine, 2-fluoro-6-(4-hydroxy-3-methoxyanilino)-9-(tetrahydro-2H-pyran-2-yl)-9H-purine, 2-fluoro-6-(4-hydroxy-3-methoxyanilino)-9-(oxepan-2-yl)-9H-purine, 6-allylamino-9-(oxetan-2-yl)-9H-purine, 6-allylamino-9-(tetrahydrofuran-2-yl, tetrahydro-2H-pyran-2-yl)-9H-purine, 6-allylamino-9-(oxepan-2-yl)-9H-purine, 6-isopentenylamino-9-(oxetan-2-yl)-9H-purine, 6-isopentenylamino-9-(tetrahydrofuran-2-yl)-9H-purine, 6-isopentenylamino-9-(tetrahydro-2H-pyran-2-yl)-9H-purine, 6-isopentenylamino-9-(oxepan-2-yl)-9H-purine, 2-fluoro-6-[(3-methylbut-2-en-1-yl)amino]-9-(oxetan-2-yl)-9H-purine, 2-fluoro-6-[(3-methylbut-2-en-1-yl)amino]-9-(tetrahydrofuran-2-yl)-9H-purine, 2-fluoro-6-[(3-methylbut-2-en-1-yl)amino]-9-(tetrahydro-2*H*-pyran-2-yl, oxepan-2-yl)-9*H*-purine; 2-fluoro-6-[(3-methylbut-3-en-1-yl)amino]-9-(oxetan-2-yl)-9H-purine, 2-fluoro-6-[(3-methylbut-3-en-1-yl)amino]-9-(tetrahydrofuran-2-yl)-9H-purine, 2-fluoro-6-[(3-methylbut-3-en-1-yl)amino]-9-(tetrahydro-2*H*-pyran-2-yl)-9H-purine, 2-fluoro-6-[(3-methylbut-3-en-1-yl)amino]-9-(oxepan-2-yl)-9*H*-purine; 2-fluoro-6-(*E*)-[(4-hydroxy-3-methylbut-2-en-1-yl)amino]-9-(oxetan-2-yl)-9H-purine, 2-fluoro-6-(*E*)-[(4-hydroxy-3-methylbut-2-en-1-yl)amino]-9-(tetrahydrofuran-2-yl)-9H-purine, 2-fluoro-6-(*E*)-[(4-hydroxy-3-methylbut-2-en-1-yl)amino]-9-(tetrahydro-2*H*-pyran-2-yl)-9H-purine, 2-fluoro-6-(*E*)-[(4-hydroxy-3-methylbut-2-en-1-yl)amino]-9-(oxepan-2-yl)-9*H-*purine; 2-fluoro-6-(*Z*)-[(4-hydroxy-3-methylbut-2-en-1-yl)amino]-9-(oxetan-2-yl)-9H-purine, 2-fluoro-6-(*Z*)-[(4-hydroxy-3-methylbut-2-en-1-yl)amino]-9-(tetrahydrofuran-2-yl)-9H-purine, 2-fluoro-6-(Z)-[(4-hydroxy-3-methylbut-2-en-1-yl)amino]-9-(tetrahydro-2*H*-pyran-2-yl)-9H-purine, 2-fluoro-6-(Z)-[(4-hydroxy-3-methylbut-2-en-1-yl)amino]-9-(oxepan-2-yl)-9*H*-purine; 2-fluoro-6-[(4-hydroxy-3-methylbutyl)amino]-9-(oxetan-2-yl)-9H-purine, 2-fluoro-6-[(4-hydroxy-3-methylbutyl)amino]-9-(tetrahydrofuran-2-yl)-9H-purine, 2-fluoro-6-[(4-hydroxy-3-methylbutyl)amino]-9-(tetrahydro-2*H*-pyran-2-yl)-9H-purine, 2-fluoro-6-[(4-hydroxy-3-methylbutyl)amino]-9-(oxepan-2-yl)-9H-purine, 2-fluoro-6-(Z)-(1'-methyl-4-hydroxy-3-methylbut-2-en-1-ylamino)-9-(oxetan-2-yl)-9H-purine, 2-fluoro-6-(*Z*)-(1'-methyl-4-hydroxy-3-methylbut-2-en-1-ylamino)-9-(tetrahydrofuran-2-yl)-9H-purine, 2-fluoro-6-(*Z*)-(1'-methyl-4-hydroxy-3-methylbut-2-en-1-ylamino)-9-(tetrahydro-2H-pyran-2-yl)-9H-purine, 2-fluoro-6-(Z)-(1'-methyl-4-hydroxy-3-methylbut-2-en-1-ylamino)-9-(oxepan-2-yl)-9*H-*purine, 2-fluoro-6-(E)-(1'-methyl-4-hydroxy-3-methylbut-2-en-1-ylamino)-9-(oxetan-2-yl)-9H-purine, 2-fluoro-6-(E)-(1'-methyl-4-hydroxy-3-methylbut-2-en-1-ylamino)-9-(tetrahydrofuran-2-yl)-9H-purine, 2-fluoro-6-(E)-(1'-methyl-4-hydroxy-3-methylbut-2-en-1-ylamino)-9-(tetrahydro-2*H*-pyran-2-yl)-9H-purine, 2-fluoro-6-(E)-(1'-methyl-4-hydroxy-3-methylbut-2-en-1-ylamino)-9-(oxepan-2-yl)-9*H*-purine, 6-(1'-methyl-4-hydroxy-3-methylbutylamino)-9-(oxetan-2-yl)-9H-purine, 6-(1'-methyl-4-hydroxy-3-methylbutylamino)-9-(tetrahydrofuran-2-yl)-9H-purine, 6-(1'-methyl-4-hydroxy-3-methylbutylamino)-9-(tetrahydro-2H-pyran-2-yl)-9H-purine, 6-(1'-methyl-4-hydroxy-3-methylbutylamino)-9-(oxepan-2-yl)-9H-purine and the acceptable salts thereof with alkali metals, ammonium or amines.

The most preferred 2-fluoro-N⁶-substituted-9-(2-oxacycloalkyl)-9H-purine-6-amine derivatives of the general formula I are selected from the group comprising: 2-fluoro-6-furfurylamino-9-(oxetan-2-yl)-9*H-*purine, 2-fluoro-6-(3-methoxyfurfurylamino)-9-(oxetan-2-yl)-9*H*-purine, 2-fluoro-6-benzylamino-9-(oxetan-2-yl)-9*H*-purine; 2-fluoro-6-[(2-hydroxybenzyl)amino]-9-(oxetan-2-yl)-9*H*-purine, 2-fluoro-6-[(3-hydroxybenzyl)amino]-9-(oxetan-2-yl)-9*H-*purine, 2-fluoro-6-[(4-hydroxybenzyl)amino]-9-(oxetan-2-yl)-9*H*-purine, 2-fluoro-6-[(2-methoxybenzyl)amino]-9-(oxetan-2-yl)-9*H*-purine, 2-fluoro-6-[(3-methoxybenzyl)amino]-9-(oxetan-2-yl)-9*H*-purine, 2-fluoro-6-[(4-methoxybenzyl)amino]-9-(oxetan-2-yl)-9*H*-purine, 2-fluoro-6-[(2-fluorobenzyl)amino]-9-(oxetan-2-yl)-9*H*-purine; 2-fluoro-6-[(3-fluorobenzyl)amino]-9-(oxetan-2-yl)-9*H*-purine, 2-fluoro-6-[(4-fluorobenzyl)amino]-9-(oxetan-2-yl)-9*H-*purine, 2-fluoro-6-[(2-chlorobenzyl)amino]-9-(oxetan-2-yl)-9*H*-purine, 2-fluoro-6-[(3-chlorobenzyl)amino]-9-(oxetan-2-yl)-9*H*-purine, 2-fluoro-6-(3-methoxyanilino)-9-(oxetan-2-yl)-9H-purine, 2-fluoro-6-[(3-methylbut-2-en-1-yl)amino]-9-(oxetan-2-yl)-9H-purine, 2-fluoro-6-(E)-[(4-hydroxy-3-methylbut-2-en-1-yl)amino]-9-(oxetan-2-yl)-9H-purine, 2-fluoro-6-(Z)-[(4-hydroxy-3-methylbut-2-en-1-yl)amino]-9-(oxetan-2-yl)-9H-purine, 2-fluoro-6-[(4-hydroxy-3-methylbutyl)amino]-9-(oxetan-2-yl)-9H-purine, 2-fluoro-6-(3-methoxyfurfurylamino)-9-(tetrahydrofuran-2-yl)-9H-purine, 2-fluoro-6-benzylamino-9-(tetrahydrofuran-2-yl)-9H-purine; 2-fluoro-6-[(2-hydroxybenzyl)amino]-9-(tetrahydrofuran-2-yl)-9H-purine, 2-fluoro-6-[(3-hydroxybenzyl)amino]-9-(tetrahydrofuran-2-yl)-9H-purine, 2-fluoro-6-[(4-hydroxybenzyl)amino]-9-(tetrahydrofuran-2-yl)-9H-purine; 2-fluoro-6-[(2-methoxybenzyl)amino]-9-(tetrahydrofuran-2-yl)-9H-purine, 2-fluoro-6-[(3-methoxybenzyl)amino]-9-(tetrahydrofuran-2-yl)-9H-purine, 2-fluoro-6-[(4-methoxybenzyl)amino]-9-(tetrahydrofuran-2-yl)-9H-purine, 2-fluoro-6-[(2-fluorobenzyl)amino]-9-(tetrahydrofuran-2-yl)-9H-purine, 2-fluoro-6-[(3-fluorobenzyl)amino]-9-(tetrahydrofuran-2-yl)-9H-purine, 2-fluoro-6-[(4-fluorobenzyl)amino]-9-(tetrahydrofuran-2-yl)-9H-purine; 2-fluoro-6-[(2-chlorobenzyl)amino]-9-(tetrahydrofuran-2-yl)-9H-purine, 2-fluoro-6-[(3-chlorobenzyl)amino]-9-(tetrahydrofuran-2-yl)-9H-purine, 2-fluoro-6-(3-methoxyanilino)-9-(tetrahydrofuran-2-yl)-9H-purine, 2-fluoro-6-[(3-methylbut-2-en-1-yl)amino]-9-(tetrahydrofuran-2-yl)-9H-purine, 2-fluoro-6-(E)-[(4-hydroxy-3-methylbut-2-en-1-yl)amino]-9-(tetrahydrofuran-2-yl)-9H-purine, 2-fluoro-6-(Z)-[(4-hydroxy-3-methylbut-2-en-1-yl)amino]-9-(tetrahydrofuran-2-yl)-9H-purine, 2-fluoro-6-[(4-hydroxy-3-methylbutyl)amino]-9-(tetrahydrofuran-2-yl)-9H-purine, 2-fluoro-6-(3-methoxyfurfurylamino)-9-(tetrahydro-2H-pyran-2-yl)-9H-purine; 2-fluoro-6-benzylamino-9-(tetrahydro-2H-pyran-2-yl)-9H-purine, 2-fluoro-6-[(2-hydroxybenzyl)amino]-9-(tetrahydro-2H-pyran-2-yl)-9H-purine, 2-fluoro-6-[(3-hydroxybenzyl)amino]-9-(tetrahydro-2H-pyran-2-yl)-9H-purine, 2-fluoro-6-[(4-hydroxybenzyl)amino]-9-(tetrahydro-2H-pyran-2-yl)-9H-purine, 2-fluoro-6-[(2-methoxybenzyl)amino]-9-(tetrahydro-2H-pyran-2-yl)-9H-purine, 2-fluoro-6-[(3-methoxybenzyl)amino]-9-(tetrahydro-2H-pyran-2-yl)-9H-purine, 2-fluoro-6-[(4-methoxybenzyl)amino]-9-(tetrahydro-2H-pyran-2-yl)-9H-purine, 2-fluoro-6-[(2-fluorobenzyl)amino]-9-(tetrahydro-2H-pyran-2-yl)-9H-purine, 2-fluoro-6-[(3-fluorobenzyl)amino]-9-(tetrahydro-2H-pyran-2-yl)-9H-purine, 2-fluoro-6-[(4-fluorobenzyl)amino]-9-(tetrahydro-2H-pyran-2-yl)-9H-purine, 2-fluoro-6-[(2-chlorobenzyl)amino]-9-(tetrahydro-2H-pyran-2-yl)-9H-purine, 2-fluoro-6-[(3-chlorobenzyl)amino]-9-(tetrahydro-2H-pyran-2-yl)-9H-purine, 2-fluoro-6-(3-methoxyanilino)-9-(tetrahydro-2H-pyran-2-yl)-9H-purine, 2-fluoro-6-[(3-methylbut-2-en-1-yl)amino]-9-(tetrahydro-2H-pyran-2-yl)-9H-purine, 2-fluoro-6-(E)-[(4-hydroxy-3-methylbut-2-en-1-yl)amino]-9-(tetrahydro-2H-pyran-2-yl)-9H-purine, 2-fluoro-6-(Z)-[(4-hydroxy-3-methylbut-2-en-1-yl)amino]-9-(tetrahydro-2H-pyran-2-yl)-9H-purine, 2-fluoro-6-[(4-hydroxy-3-methylbutyl)amino]-9-(tetrahydro-2H-pyran-2-yl)-9H-purine, 2-fluoro-6-(3-methoxyfurfurylamino)-9-(oxepan-2-yl)-9H-purine, 2-fluoro-6-benzylamino-9-(oxepan-2-yl)-9H-purine; 2-fluoro-6-[(2-hydroxybenzyl)amino]-9-(oxepan-2-yl)-9H-purine, 2-fluoro-6-[(3-hydroxybenzyl)amino]-9-(oxepan-2-yl)-9H-purine, 2-fluoro-6-[(4-hydroxybenzyl)amino]-9-(oxepan-2-yl)-9H-purine; 2-fluoro-6-[(2-methoxybenzyl)amino]-9-(oxepan-2-yl)-9H-purine; 2-fluoro-6-[(3-methoxybenzyl)amino]-9-(oxepan-2-yl)-9H-purine, 2-fluoro-6-[(4-methoxybenzyl)amino]-9-(oxepan-2-yl)-9H-purine, 2-fluoro-6-[(2-fluorobenzyl)amino]-9-(oxepan-2-yl)-9H-purine; 2-fluoro-6-[(3-fluorobenzyl)amino]-9-(oxepan-2-yl)-9H-purine, 2-fluoro-6-[(4-fluorobenzyl)amino]-9-(oxepan-2-yl)-9H-purine, 2-fluoro-6-[(2-chlorobenzyl)amino]-9-(oxepan-2-yl)-9H-purine, 2-fluoro-6-[(3-chlorobenzyl)amino]-9-(oxepan-2-yl)-9H-purine; 2-fluoro-6-(3-methoxyanilino)-9-(oxepan-2-yl)-9H-purine, 2-fluoro-6-[(3-methylbut-2-en-1-yl)amino]-9-(oxepan-2-yl)-9H-purine, 2-fluoro-6-(E)-[(4-hydroxy-3-methylbut-2-en-1-yl)amino]-9-(oxepan-2-yl)-9H-purine, 2-fluoro-6-(Z)-[(4-hydroxy-3-methylbut-2-en-1-yl)amino]-9-(oxepan-2-yl)-9H-purine, 2-fluoro-6-[(4-hydroxy-3-methylbutyl)amino]-9-(oxepan-2-yl)-9H-purine and agriculturally acceptable salts thereof.

The 2-fluoro-*N*⁶-substituted-9-(2-oxacycloalkyl)-9*H*-purine-6-amine derivatives of the general formula **I** according to the present invention have a wide range of biological activities, including anti-senescent, cell division stimulatory, and pro-differentiation activities, which are especially useful in agricultural applications. The compounds according to the present invention (and compositions containing thereof) possess cell division stimulatory, pro-differentiating, antisenescent, phloem loading, shoot forming, and root growth stimulatory properties and minimal or zero cytoxicities from human cells comparing to their analogues known in the state of the art.

The 2-fluoro-N⁶-substituted-9-(2-oxacycloalkyl)-9H-purine-6-amine derivatives of the general formula **I** of the present invention can be used in agriculture and biotechnology. The practical use of these compounds includes inhibition, delaying, or reducing the adverse effects of senescence in plants and in tissue cultures, especially in vivo and in vitro; the uses also include improving the overall appearance and condition of the plants, in paticular plant epidermal and mesophyll cells, including senescence-related changes and other undesirable changes such as yellowing, chloroplast and chlorophyll losses, leaf losses.

The 2-fluoro-N⁶-substituted-9-(2-oxacycloalkyl)-9H-purine-6-amine derivatives of the general formula **I** can be used as growth regulators in agriculture, to increase crop production, to dispatch grain filling and to increase grain and fruit size of plants and fungi, to shorten plant seed germination period, to increase the yield and quality of agricultural products, to enhance root and shoot growth, and to increase stress resistance to environmental factors. The compounds of the invention show stimulation of plant shoot growth and development without root growth inhibition.

Particularly suitable is the use of 2-fluoro-N6-substituted-9-(2-oxacycloalkyl)-9H-purine-6-amine derivatives of the general formula **I** as plant growth regulators in the production of crops, in particular cereals (wheat, barley, rice, maize, rye, oat, sorghum, and related species), beet (sugar beet and fodded beet); pomes, drupes and soft fruits (apples, pears, plums, peaches, almonds, cherries, strawberries and blackberries); leguminous plants (beans, lentils, peas, soybeans); oil plants (rape, mustard, poppy, olives, sunflowers, coconut, Ricinus, cocoa beans, groundnuts); cucumber plants (pumpkins, cucumbers, melons); fibre plants (cotton, flax, hemp, jute); citrus fruit (oranges, lemons, grapefruit, mandarins); vegetables (spinach, cinnamomum, camphor) or plants such as tobacco, nuts, eggplants, sugar cane, tea, vine grapes, hops, bananas and natural rubber and medicinal plants, as well as ornamentals. Crops include those which have been rendered tolerant towards classes of growth factors by conventional breeding methods or genetic engineering methods.

The 2-fluoro-N6-substituted-9-(2-oxacycloalkyl)-9H-purine-6-amine derivatives of the general formula **I** can also be used as growth regulators in plant tissue cultures for stimulation of proliferation and morphogenesis.

The 2-fluoro-N6-substituted-9-(2-oxacycloalkyl)-9H-purine-6-amine derivatives of the general formula **I** can be also used as growth and differentiation factors. They can be used in modulation of a variety of conditions in plant tissue cultures, especially in *in vitro* culture. In particular, such conditions include *in vitro* processes, such as micropropagation, meristematic culture, somatic embryogenesis, androgenesis, gynogenesis, suspension and protoplast cultures. The compounds according to the present invention may also be used as stimulators of root formation in plant tissue cultures.

The present invention further provides compositions comprising one or more 2-fluoro-N⁶-substituted-9-(2-oxacycloalkyl)-9H-purine-6-amine derivatives of the general formula I, and at least one agriculturally acceptable auxiliary substance.

The compounds of the general formula I can be formulated in the preparations alone or, preferably, together with the auxiliary substances conventionally employed in the art of preparations. To this end they are conveniently formulated as concentrates of active compounds as well as suspensions and dispersions, preferentially isotonic water solutions, suspensions and dispersion, diluted emulsions, soluble powders, dusts, granulates, creams, gels, oil suspensions and also encapsulations, e.g. polymeric substances. As with the type of the preparation, the methods of application, such as spraying, atomizing, dusting, scattering, coating or pouring, are chosen in accordance with the intended objectives and the prevailing circumstances. The preparations may be sterilized and/or contain further excipients of neutral nature such as preservatives, stabilizers, wetting agents or emulgators, solubilizing agents, as well as fertilizers, micronutrient donors or other formulations for obtaining special effects.

The compounds of the formula **I** can be mixed with other growth regulators, resulting in synergistic activities.

### PREPARATIONS AND FORMULATIONS

The preparations comprising the compounds (active ingredients) of formula I and, where appropriate, one or more solid or liquid auxiliary substances, are prepared in a manner known per se, e.g. by intimately mixing and/or grinding the active ingredients with the auxiliary substances, e.g. solvents or solid carriers. In addition, surface-active compounds (surfactants) may also be used in the preparation of the formulations. Depending on the nature of the compound of formula I to be formulated, suitable surface- active compounds are non-ionic, cationic and/or anionic surfactants and surfactant mixtures having good emulsifying, dispersing and wetting properties. Examples of suitable anionic, non-ionic and cationic surfactants are listed, for example, in WO 97/34485. Also suitable in the preparation of the growth regulator compositions according to the invention are the surfactants conventionally used in formulation technology, which are described, inter alia, in "McCutcheon's Detergents and Emulsifiers Annual" MC Publishing Corp., Ridgewood New Jersey, 1981, Stache, H., "Tensid- Taschenbuch", Carl Hanser Verlag, MunichNienna, 1981 and M. and J. Ash, "Encyclopedia of Surfactants", Voll-lll, Chemical Publishing Co., New York, 1980-81.

The growth regulatory preparations usually contain from 0.1 to 99 % by weight, especially from 0.1 to 95 % by weight, of active ingredient mixture comprising a compound of formula I, from 1 to 99.9% by weight of a solid or liquid adjuvant, and from 0.1 to 25 % by weight, especially from 0.1 to 25 % by weight, of a surfactant.

Whereas commercial products are usually formulated as concentrates, the end user will normally employ dilute formulations. The composition can thus contain also further additives, such as stabilizers, e.g., plant oils or epoxidized plant oils (epoxidized palm oil 0;1, rapeseed or olive oil), defoamers, e.g., silicon oil, preservatives, wetting agents or emulgators, viscosity agent, binders, glues, and also fertilizers and other active additives. Preferred formulations have especially the following compositions: (% = percent by weight)

### Emulsifiable concentrates:

| | |
|---|---|
| active ingredient mixture: | 1 to 90 %, preferably 5 to 20 % |
| surfactant: | 1 to 30 %, preferably 10 to 20 % |
| liquid carrier: | 5 to 94 %, preferably 70 to 85 % |

### Dusts:

| | |
|---|---|
| active ingredient mixture: | 0.1 to 10 %, preferably 0.1 to 5 % |
| solid carrier: | 99.9 to 90 %, preferably 99.9 to 99 % |

### Suspension concentrates:

| | |
|---|---|
| active ingredient mixture: | 5 to 75 %, preferably 10 to 50 % |
| water: | 94 to 24 %, preferably 88 to 30 % |
| surfactant: | 1 to 40 %, preferably 2 to 30 % |

### Wettable powders:

| | |
|---|---|
| active ingredient mixture: | 0.5 to 90 %, preferably 1 to 80 % |
| surfactant: | 0.5 to 20 %, preferably 1 to 15 % |
| solid carrier: | 5 to 95 %, preferably 15 to 90 % |

### Granules:

| | |
|---|---|
| active ingredient mixture: | 0.1 to 30 %, preferably 0.1 to 15 % |
| solid carrier: | 99.5 to 70 %, preferably 97 to 85 % |

The compositions may also comprise further ingredients, such as stabilisers, e.g. vegetable oils or epoxidised vegetable oils (epoxidised coconut oil, rapeseed oil or soybean oil), anti-foams, e.g. silicone oil, preservatives, viscosity regulators, binders, tackifiers, and also fertilisers or other active ingredients. For the use of growth factors of formula I, or of compositions comprising them, various methods and techniques come into consideration, such as, for example, the following:

### i) Seed dressing

a) Dressing of the seeds with a wettable powder formulation of a compound of the general formula I by shaking in a vessel until uniformly distributed over the seed surface (dry dressing). In that procedure approximately from 1 to 500 g of compound of the general formula I (4 g to 2 kg of wettable powder) are used per 100 kg of seed.
b) Dressing of the seeds with an emulsifiable concentrate of a compound of formula I according to method a) (wet dressing).
c) Dressing by immersing the seeds for from 1 to 72 hours in a liquor comprising from 100 to 1000 ppm of a compound of formula I and optionally subsequently drying the seeds (immersion dressing).

Dressing the seed or treating the germinated seedling are naturally the preferred methods of application, because treatment with the active ingredients is directed entirely at the target crop. Generally from 1 to 1000 g of antidote, preferably from 5 to 250 g of antidote, are used per 100 kg of seed, but depending on the methodology, which also enables the addition of other active ingredients or micronutrients: the concentration limits indicated can be varied up or down (repeat dressing).

### ii) Application as a tank mixture

A liquid formulation of a mixture of antidote and growth regulator is used (ratio by weight of the one to the other from 10:1 to 1:100), the rate of application of growth regulator being from 0.005 to 5.0 kg per hectare. Such tank mixtures are applied before or after sowing.

### iii) Application to the seed furrow

The compounds of formula I are introduced into the open, sown seed furrow in the form of an emulsifiable concentrate, wettable powder or granules. Once the seed furrow has been covered over, the growth regulator is applied in the usual manner in the pre-emergence process.

### iv) Controlled release of active ingredient

The compounds of formula I are applied in solution to mineral granule carriers or polymerised granules (urea/formaldehyde) and dried. If desired, it is also possible to apply a coating that allows the active ingredient to be released in metered amounts over a specific period of time (coated granules).

### Brief description of Drawings

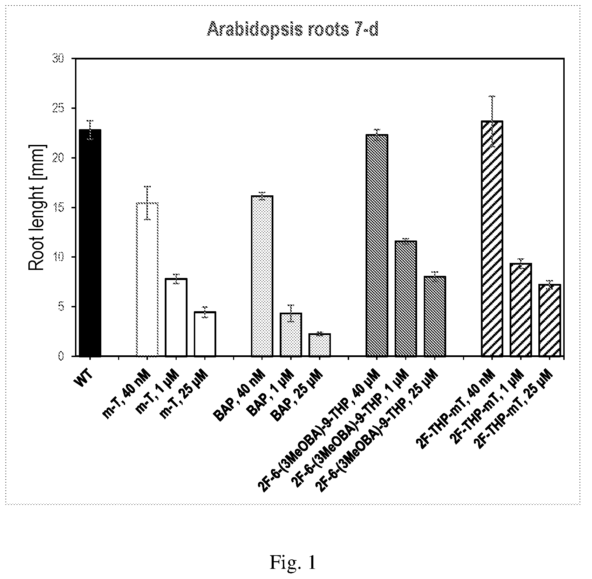

Fig. 1 displays the inhibition of roots of Arabidopsis thaliana 7-day plants of known cytokinins BAP and m-T with new cytokinin derivatives 2-fluoro-6-[(3-methoxylbenzyl)amino]-9-(tetrahydro-2H-pyran-2-yl)-9H-purine (compound **67**) and 2-fluoro-6-[(3-hydroxybenzyl)amino]-9-(tetrahydro-2H-pyran-2-yl)-9H-purine (compound **65**) at three different concentrations used (1,25 and 40 microM) compared to WT

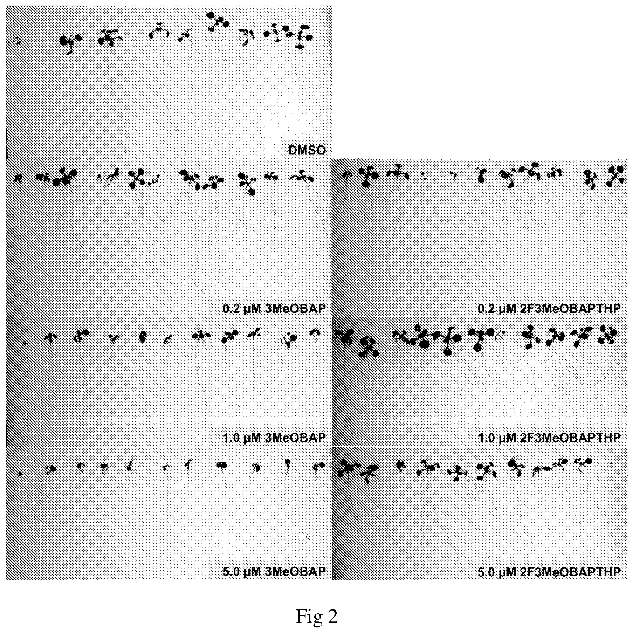

Fig 2 displays the phenotypic changes observed on *Arabidopsis* plants grown in MS agar medium supplemented with a compound of formula I. (0.2 µM - 5 µM). DMSO - control, dimethyl sulfoxide; 3MeOBAP - 6-(3-methoxybenzyl)aminopurine; 2F3MeOBAPTHP - 2-fluoro-6-[(3-methoxylbenzyl)amino]-9-(tetrahydro-2H-pyran-2-yl)-9H-purine (compound **68**)**.**

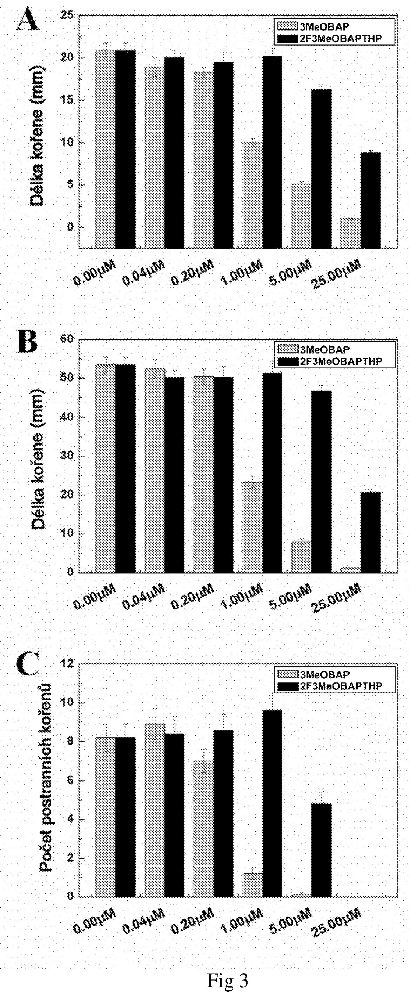

Fig. 3 shows the primary root length and the number of lateral roots of *Arabidopsis* seedlings grown on medium with applied compounds of formula I. The root length was determined on 7-day (A) and 14-day-old seedlings, the number of lateral roots formed in 14-day-old seedlings (C). Each value is the average of at least 90 plants grown on 9 independent vertical square petri dishes.

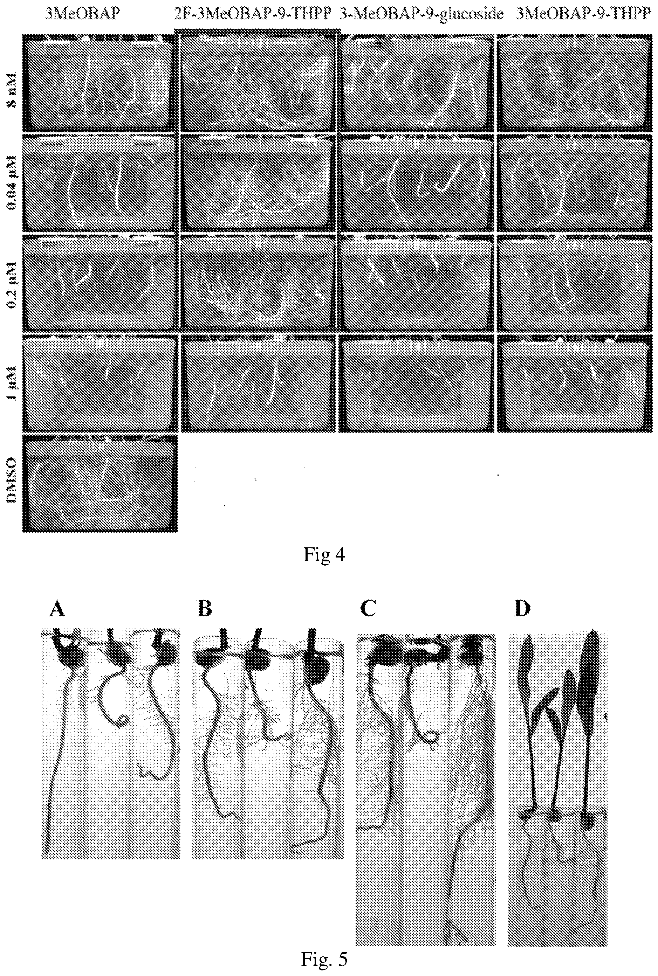

Fig. 4 shows maize phenotype after exogenous application of compounds of formula I. 8 nM tested compound were added to the nutrient solution to 3 days-old seedlings. From left to right: 3-MeOBAP - 6-(3-methoxybenzyl)aminopurine; 2F3MeOBAPTHP - 2-fluoro-6-[(3-methoxylbenzyl)amino]-9-(tetrahydro-2H-pyran-2-yl)-9H-purine (compound **67**); 2FBAPTHP - 2-fluor-6-[benzylamino]-9-(tetrahydro-2H-pyran-2-yl)-9H-purine (compound **64**) a 3-MeOBAP-9-glucoside. Approximately 50 seedlings were cultivated in one liter of nutrient solution with added compound.
Fig. 5 shows maize phenotype after exogenous application of compounds of formula I. 8 nM tested compound were added to the nutrient solution to 3 days-old seedlings. From left to right: DMSO; 3-MeOBAP - 6-(3-methoxybenzyl)aminopurine; 2F3MeOBAPTHP - 2-fluoro-6-[(3-methoxylbenzyl)amino]-9-(tetrahydro-2H-pyran-2-yl)-9H-purine (compound **67**). A - 6 days-old seedling roots; B - 7 days-old seedling roots; C - 8 days-old seedling roots; D - 7 days-old seedlings. Ten seedlings were cultivated in one liter of nutrient solution with added cytokinin.

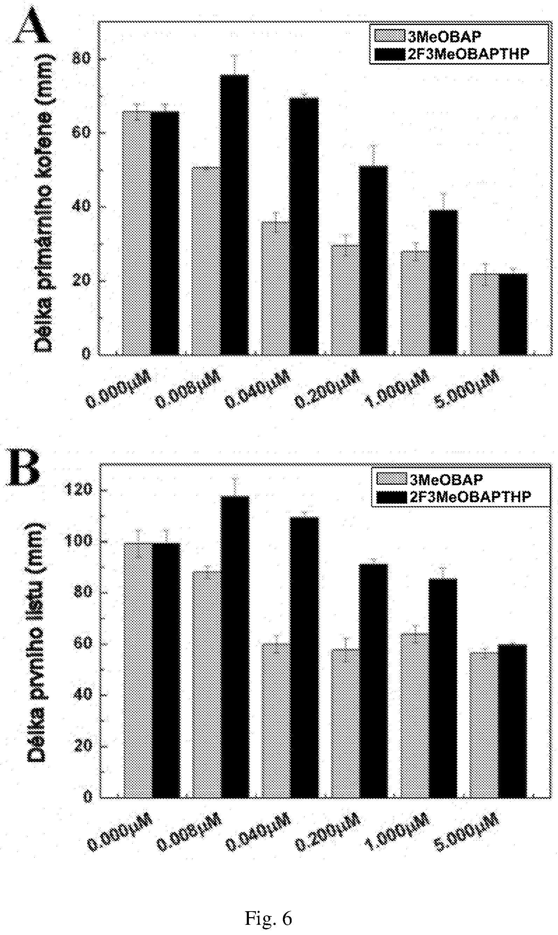

Fig. 6 shows the length of the primary root (A) and the first leaf (B) of 7-day-old maize discovered. Zea mays plants cultured for 5 days in nutrient solution with applied compounds. Each value is the average of at least 120 seedlings grown in 12 independent hydroponic boxes.

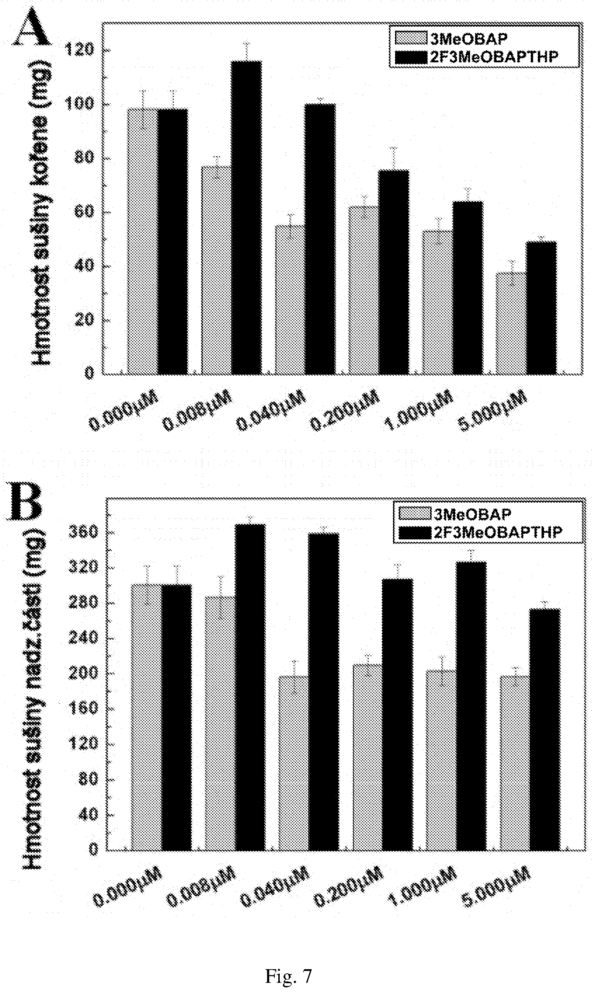

Fig. 7 shows the effect of new 2-fluoro-N⁶-substituted-9-(2-oxacycloalkyl)-9H-purin-6-amine derivatives of general formula **I** in comparison with classical cytokinin (precursor molecule 3-MeOBAP - 6-(3-methoxybenzyl)aminopurine) for the production of maize biomass. After 5 days of culturing in nutrient solution with applied cytokinins, the dry matter of the 7-day plant root system (A) and the aboveground part (B) was determined. Each value is the average of the plant mass evaluated from 120 seedlings grown in 12 independent hydroponic boxes.

### Examples

The following examples serve to illustrate the invention without limiting the scope thereof. Unless otherwise stated, all percentages and the like amounts are based on weight.

The starting materials may be obtained from commercial sources (Sigma, Aldrich, Fluka, etc.) or can be prepared as described below. Thin-layer chromatography was carried out on Silica 60 F₂₅₄ plates (Merck) using CHCl₃/MeOH as a developing system and the spots were detected by UV light (254 and 365 nm) and/or 6% (v/v) vanilline in absolute EtOH containing 1 % (v/v) of H₂SO₄. The column chromatography purification of intermediates was carried out by silica Davisil 40-63 micron (Grace Davision). Elemental analysis was determined using Flash EA 1112 analyzer (Thermo Scientific). The chromatographic purity and molecular mass of prepared compounds was determined using an Alliance 2695 separation module (Waters) linked simultaneously to a DAD detector PDA 996 (Waters) and a Q-Tof micro (Waters) benchtop quadrupole orthogonal acceleration time-of-flight tandem mass spectrometer. Samples were dissolved in DMSO and diluted to a concentration of 10 µg/ml in initial mobile phase (90% 15 mM ammonium formate, pH 4.0 (A) + 10% methanol (B)). Samples (10 µl) were injected on a RP-column Symmetry C18 (150 mm x 2.1 mm x 3.5 µm, Waters) and separated at a flow rate of 0.2 ml/min with the following binary gradient: 0 min, 10% B; 0-24 min, a linear gradient to 90% B, 10 min, followed by 10 min isocratic elution of 90% B. At the end of the gradient, the column was re-equilibrated to initial conditions (15 mM formic acid adjusted to pH 4.0 by ammonium hydroxide was used as solvent (A) and methanol as the organic modifier (solvent B)). The eluent was introduced into the DAD (scanning range 210-400 nm, with 1.2 nm resolution) and an ESI source (source temperature 110 °C, capillary voltage +3.0 kV, cone voltage +20 V, desolvation temperature 250 °C). Nitrogen was used both as desolvation gas (500 l/h) as well as cone gas (50 l/h). The data was obtained in positive (ESI+) ionization mode in the 50-1000 *m*/*z* range.

¹H and ¹³C NMR spectra were recorded on Jeol ECA-500 operating at a frequency of 500 MHz (¹H) and 126 MHz (¹³C). Samples were prepared by dissolving substances in DMSO-*d*₆ and chemical shifts were calibrated to residual solvent peak (DMSO, 2.49 ppm for proton) and DMSO-*d*₆ (39.5 ppm for carbon).

2-Fluoro-*N*⁶-substituted-9-(2-oxacycloalkyl)-9*H*-purine-6-amine derivatives were prepared in two steps, starting from 6-chloro2-fluoro-9*H*-purine. Firstly, (2-oxacycloalkyl) substituents were introduced to the *N*9-position of purine moiety by hydroamination of corresponding α,β-unsaturated *O*-heterocycles by 6-chloro2-fluoro-9*H*-purine in ethyl acetate catalyzed by trifluoroacetic acid according to protocol used for the synthesis of 2,6-dichloro-9*H*-tetrahydropyran-2-yl purine in Szučová et al. 2009 Bioorg. Med. Chem. 17, 1938-1947. 2,3,4,5-tetrahydrooxepin was prepared by *endo* cycloisomeration of 5-hexyn-1-ol catalyzed by tetradentate nitrogen-phosphorous mixed ligand ruthenium complex following methodology published in Liu et al. (2010) Chem. Eur. J. 16, 7889-7897 and Mitchell et al. (1973) J. Chem. Soc., Dalton Trans. (8), 846-854.

The intermediates were further subjected to the nucleophilic substitution at C⁶ position with appropriate side-chain amines and triethylamine in alcohols, preferably in n-propanol or n-butanol; reaction temperatures are kept in range 60 - 90 °C.

### Modification of purines at N9-position:

### A) Hydroamination of α,β-unsaturated O-heterocycles

### Example 1 6-Chloro-2-fluoro-9-(tetrahydrofuran-2-yl)-9H-purine

To a suspension of 6-chloro-2-fluoro-9*H*-purine (1 eq., 5 g) and 2,3-dihydrofuran (2.5 eq.) under Ar atmosphere in dry ethyl acetate (40 ml) was trifluoroacetic acid (2.5 eq) dropwise added and resulting solution was stirred at room temperature for 2 hours. After cooling in ice bath pH of the reaction mixture was adjusted to 7-8 by conc. NH₃/water (2:3, v/v) and the layers were separated. The water phase was reextracted by ethyl acetate (5x 25 ml). Combined organic layers were washed with brine (2x 25 ml), dried (Na₂SO₄) and evaporated under reduced pressure. The product as a pale yellow solid was obtained by crystallization from petroleum ether. Pale yellow solid, chemical formula: C₉H₈ClFN₄O, yield (%): 75. HPLC-UV/VIS retention time, purity (min., %): 21.07, 97.0. ESI⁺-MS *m*/*z* (rel. int. %): 243.0 (100.0 %), 245.0 (32.0 %), 244.0 (10.0 %). ¹H NMR (500 MHz, DMSO-d₆) δ (ppm): 2.01-2.08 (m, 1H), 2.11-2.20 (m, 1H), 2.41-2.46 (m, 2H), 3.93 (q, *J* = 7.5 Hz, 1H), 4.18 (td, *J =* 7.9, *J'* = 5.7 Hz, 1H), 6.33 (dd, *J* = 5.8*,* 4.3 Hz, 1H), 8.82 (s, 1H). ¹³C NMR (126 MHz, DMSO-d₆) δ (ppm):

### Example 2 6-Chloro-2-fluoro-9-(tetrahydro-2H-pyran-2-yl)-9H-purine

To a suspension of 6-chloro-2-fluoro-9*H*-purine (1 eq., 5 g) and 3,4-dihydro-2*H*-pyran (2 eq.) under Ar atmosphere in dry ethyl acetate (50 ml) was trifluoroacetic acid (1.8 eq.) dropwise added and resulting solution was stirred at room temperature for 2 hours. After cooling in ice bath pH of the reaction mixture was adjusted to 7-8 by conc. NH₃/water (2:3, v/v). Resulting solid was filtered, washed with cold water and dried at room temperature. Second portion of product was obtained by extraction of filtrate ans washings by ethyl acetate (3x 25 ml). The combined organic layers were washed with brine (2x 15 ml) dried (Na₂SO₄) and concentrated under reduced pressure to give a yellow gel. The solid material was obtained after crystallization from diethyl ether. White solid, chemical formula: C₁₀H₁₀ClFN₄O, yield (%): 78. HPLC-UV/VIS retention time, purity (min., %): 22.87, 98.8. ESI⁺-MS *m*/*z* (rel. int. %): 257.0 (100.0 %), 259.0 (32.0 %), 258.0 (11.0 %). ¹H NMR (500 MHz, DMSO-d₆) δ (ppm): 1.53-1.57 (m, 2H), 1.62-1.74 (m, 1H), 1.96-1.98 (m, 2H), 2.22-2.24 (m, 1H), 3.69-3.72 (m, 1H), 3.97-3.98 (m, 1H), 5.67 (dd, *J* = 10.9, *J'* = 2.3 Hz, 1H), 8.89 (s, 1H). ¹³C NMR (126 MHz, DMSO-d₆) δ (ppm): 22.3, 24.9, 30.1, 67.9, 82.2, 130.2, 146.7, 150.9, 153.4, 155.8.

### Substitution of purines at C6-position:

### Synthesis of 2-fluoro-6-substituted-9-(2-oxacycloalkyl)-9H-purine derivatives - general procedure

Mixture of 6-chloro-2-fluoro-9-(2-oxacycloalkyl)-9*H*-purine (1 eq.), side-chain amine (1.2 eq.) and triethylamine (2.5 eq. and in case of salts 3.5 eq., respectively) were heated at 60 °C in propanol (9 ml) for 6 hours. After evaporation under reduced pressure the residue was treated with ice cold water (15 ml). Method **A**) If a solid material was obtained - the solid was filtered, washed well with ice cold water and dried.

Method **B**) If no solid material was obtained - the mixture was extracted by ethyl acetate (5x 10 ml). Combined organic layers were washed using brine (2x 10 ml), dried (Na₂SO₄) and concentrated under reduced pressure.

The crude material was purified by silica column chromatography using chloroform/methanol as a mobile phase starting from pure chloroform with methanol gradient.

### Example 3 2-Fluoro-6-furfurylamino-9-(tetrahydrofuran-2-yl)-9H-purine

It was prepared from 2-fluoro-6-chloro-9-(tetrahydrofuran-2-yl)-9*H*-purine and furfurylamine, work-up using A. White solid, chemical formula: C₁₄H₁₄FN₅O₂, yield (%): 65. HPLC-UV/VIS retention time, purity (min., %): 24.43, 99.8. ESI⁺-MS *m*/*z* (rel. int. %): 304.1 (100.0%), 305.1 (15.4%). ¹H NMR (500 MHz, DMSO-d₆) δ (ppm): 1.91-1.96 (m, 1H), 2.09-2.17 (m, 1H), 2.37-2.40 (m, 2H), 3.87-3.91 (m, 1H), 4.01 (td, *J =* 7.5, *J'* = 3.2 Hz, 1H), 4.68 (d, *J* = 5.5 Hz, 2H), 6.16 (t, *J =* 5.5 Hz, 1H), 6.22 (d, *J =* 3.2 Hz, 1H), 7.54 (d, *J =* 5.5 Hz, 1H), 8.31 (s, 1H), 9.07 (t, *J =* 5.5 Hz, 1H). ¹³C NMR (126 MHz, DMSO-d₆) δ (ppm): 23.18, 31.13, 38.97, 68.62, 94.59, 101.74, 106.45, 119.92, 136.43, 145.93, 150.11, 150.27, 151.05, 153.08, 156.29, 156.48.

### Example 4 2-Fluoro-6-[(5-methylfurfuryl)amino]-9-(tetrahydrofuran-2-yl)-9H-purine

It was prepared from 2-fluoro-6-chloro-9-(tetrahydrofuran-2-yl)-9*H*-purine and 5-methylfurfurylamine, work-up using **B.** White solid, chemical formula: C₁₅H₁₆FN₅O₂, yield (%): 66. HPLC-UV/VIS retention time, purity (min., %): 26.05, 99.9. ESI⁺-MS *m*/*z* (rel. int. %): 318.1 (100.0%), 319.1 (18.1%). ¹H NMR (500 MHz, DMSO-d₆) δ (ppm): 1.96-2.02 (m, 1H), 2.13-2.16 (m, 1H), 2.36-2.42 (m, 2H), 3.62 (s, 3H), 4.07-4.10 (m, 1H), 4.63 (d, *J =* 6.5 Hz, 2H), 6.11 (t, *J =* 6.0 Hz, 1H), 6.24 (td, *J* = 5.5 Hz, *J' =* 2.1 Hz, 1H), 8.31 (s, 1H), 9.11 (t, *J =* 6.5 Hz, 1H). ¹³C NMR (126 MHz, DMSO-d₆) δ (ppm): 12.92, 22.14, 34.65, 42.11, 72.15, 89.37, 102.19, 108.82, 118.60, 137.41, 147.39, 150.07, 150.23, 152.90, 154.66, 156.34, 156.51.

### Example 5 2-Fluoro-6-benzylamino-9-(tetrahydrofuran-2-yl)-9H-purine

It was prepared from 2-fluoro-6-chloro-9-(tetrahydrofuran-2-yl)-9H-purine and benzylamine, work-up using **B.** White solid, chemical formula: C₁₆H₁₆FN₅O, yield (%): 65. HPLC-UV/VIS retention time, purity (min., %): 26.39, 99.9. ESI⁺-MS *m*/*z* (rel. int. %): 314.1 (100.0%), 315.1 (17.5%). ¹H NMR (500 MHz, DMSO-d₆) δ (ppm): 1.98-2.05 (m, 1H), 2.12-2.16 (m, 1H), 2.38-2.39 (m, 2H), 4.08-4.12 (m, 1H), 4.59 (bs, 2H), 6.15 (t, *J =* 6.0 Hz, 1H), 7.21 (t, *J =* 7.5 Hz, 1H), 7.25-7.31 (m, 4H), 8.32 (s, 1H), 9.11 (bs, 1H). ¹³C NMR (126 MHz, DMSO-d₆) δ (ppm): 24.08, 30.86, 44.16, 67.47, 83.82, 122.58, 125.19, 128.41, 133.14, 140.16, 141.68, 147.92, 150.12, 152.68, 153.02, 156.34, 156.51.

### Example 6 2-Fluoro-6-[(3-hydroxybenzyl)amino]-9-(tetrahydrofuran-2-yl)-9H-purine

It was prepared from 2-fluoro-6-chloro-9-(tetrahydrofuran-2-yl)-9*H*-purine and 3-(aminomethyl)phenol, work-up using **A.** White solid, chemical formula: C₁₆H₁₆FN₅O₂, yield (%): 94. HPLC-UV/VIS retention time, purity (min., %): 23.33, 99.2. ESI⁺-MS *m*/*z* (rel. int. %): 330.1 (100.0%), 331.1 (21.2%). ¹H NMR (500 MHz, DMSO-d₆) δ (ppm): 1.96-2.02 (m, 1H), 2.13-2.16 (m, 1H), 2.36 (dd, *J =* 12.5, 7.0 Hz, 2H), 3.87 (q, *J* = 7.5 Hz, 1H), 4.10 (dd, *J =* 13.5, *J' =* 7.5 Hz, 1H), 4.56 (d, *J =* 6.0 Hz, 2H), 5.55 (d, *J =* 12.5, *J' =* 2.50 Hz, 1H), 6.64 (d, *J =* 8.5 Hz, 1H), 6.71 (d, *J =* 8.00, 1H), 6.82 (s, 1H), 7.13 (t, *J =* 8.5 Hz, 1H), 8.34 (s, 1H), 8.92 (t, *J* = 6.0 Hz, 1H), 9.32 (bs, 1H). ¹³C NMR (126 MHz, DMSO-d₆) δ (ppm): 23.92, 30.86, 41.85, 69.47, 83.92, 111.68, 112.65, 118.22, 119.11, 127.43, 140.67, 141.28, 149.0, 150.07, 150.23, , 152.79, 156.32, 156.49, 156.82.

### Example 7 2-Fluoro-6-[(4-hydroxybenzyl)amino]-9-(tetrahydrofuran-2-yl)-9H-purine

It was prepared from 2-fluoro-6-chloro-9-(tetrahydrofuran-2-yl)-9*H*-purine and 4-(aminomethyl)phenol, work-up using **A.** White solid, chemical formula: C₁₆H₁₆FN₅O₂ yield (%): 62. HPLC-UV/VIS retention time, purity (min., %): 23.02, 97.6. ESI⁺-MS *m*/*z* (rel. int. %): 330.1 (100.0%), 331.1 (19.0%). ¹H NMR (500 MHz, DMSO-d₆) δ (ppm): 1.98-2.02 (m, 1H), 2.11-2.17 (m, 1H), 2.35-2.40 (m, 2H), 3.75 (q, *J =* 7.5 Hz, 1H), 4.11 (t, *J* = 7.5 Hz, 1H), 4.53 (d, *J =* 5.5 Hz, 2H), 6.14 (t, *J =* 5.5 Hz, 1H), 6.58 (d, *J =* 8.0 Hz, 2H), 6.82 (d, *J =* 8.0 Hz, 2H), 8.33 (s, 1H), 9.11 (t, *J =* 5.5 Hz, 1H), 10.11 (bs, 1H). ¹³C NMR (126 MHz, DMSO-d₆) δ (ppm): 21.25, 29.85, 43.53, 67.14, 85.6, 113.2, 120.40, 125.97, 128.42, 140.06, 148.27, 150.01, 150.17, 152.96, 155.8, 156.43, 157.36.

### Example 8 2-Fluoro-6-[(2-methoxybenzyl)amino]-9-(tetrahydrofuran-2-yl)-9H-purine

It was prepared from 2-fluoro-6-chloro-9-(tetrahydrofuran-2-yl)-9H-purine and 2-methoxybenzylamine, work-up using **B.** White solid, chemical formula: C₁₇H₁₈FN₅O₂, yield (%): 45. HPLC-UV/VIS retention time, purity (min., %): 26.97, 98.7. ESI⁺-MS *m*/*z* (rel. int. %): 344.1 (100.0%), 345.2 (18.7%). ¹H NMR (500 MHz, DMSO-d₆) δ (ppm): 1.93-1.98 (m, 1H), 2.13-2.16 (m, 1H), 2.37-2.40 (m, 2H), 3.67 (s, 3H), 4.01-4.03 (m, 1H), 4.12-4.17 (m, 1H), 4.59 (d, *J =* 6.0 Hz, 2H), 6.19 (t, *J =* 6.5 Hz, 1H), 6.74 (t, *J =* 7.0 Hz, 1H), 7.06 (d, *J =* 7.5 Hz, 1H), 7.32 (d, *J =* 7.5 Hz, 1H), 7.44 (t, *J =* 7.0 Hz, 1H), 8.28 (s, 1H), 9.12 (t, *J* = 5.5 Hz, 1H).

¹³C NMR (126 MHz, DMSO-d₆) δ (ppm): 22.88, 25.01, 30.37, 43.68, 55.31, 68.17, 81.59, 112.52, 112.97, 117.95, 119.84, 129.66, 139.88, 141.23, 150.05, 150.21, 156.34, 156.51, 158.41, 159.77, 160.03.

### Example 9 2-Fluoro-6-[(4-methoxybenzyl)amino]-9-(tetrahydrofuran-2-yl)-9H-purine

It was prepared from 2-fluoro-6-chloro-9-(tetrahydrofuran-2-yl)-9*H*-purine and 4-methoxybenzylamine, work-up using **B**. Pale yellow solid, chemical formula: C₁₇H₁₈FN₅O₂, yield (%): 50. HPLC-UV/VIS retention time, purity (min., %): 26.17, 98.3. ESI⁺-MS *m*/*z* (rel. int. %): 344.1 (100.0%), 345.2 (18.4%). ¹H NMR (500 MHz, DMSO-d₆) δ (ppm): 1.96-2.00 (m, 1H), 2.15-2.19 (m, 1H), 2.37-2.39 (m, 2H), 3.68 (s, 3H), 4.93 (q, *J =* 7.5 Hz, 1H), 4.03-4.09 (m, 1H), 4.68 (d, *J =* 6.5 Hz, 2H), 6.25 (t, *J =* 6.5 Hz, 1H), 6.72 (d, *J* = 8.5 Hz, 2H), 7.26 (d, *J* = 8.5 Hz, 2H), 8.19 (s, 1H), 8.92 (t, *J* = 6.5 Hz, 1H). ¹³C NMR (126 MHz, DMSO-d₆) δ (ppm): 22.85, 30.10, 38.15, 54.92, 69.15, 81.32, 109.08, 116.22, 128.4, 128.7, 131.1, 139.5, 149.1, 150.05, 150.19, 156.32, 156.46, 159.4, 167.09.

### Example 10 2-Fluoro-6-[(4-hydroxy-3-methoxybenzyl)amino]-9-(tetrahydrofuran-2-yl)-9H-purine

It was prepared from 2-fluoro-6-chloro-9-(tetrahydrofuran-2-yl)-9*H*-purine and 4-(aminomethyl)-2-methoxyphenol hydrochloride, work-up using **A.** White solid, chemical formula: C₁₇H₁₈FN₅O₃, yield (%): 58. HPLC-UV/VIS retention time, purity (min., %): 23.27, 99.9. ESI⁺-MS *m*/*z* (rel. int. %): 360.1 (100.0%), 361.1 (20.3%). ¹H NMR (500 MHz, DMSO-d₆) δ (ppm): 1.94-1.98 (m, 1H), 2.17-2.22 (m, 1H), 2.34-2.38 (m, 2H), 3.74 (s, 3H), 3.93 (q, *J* = 7.5 Hz, 1H), 4.18 (t, *J =* 7.5 Hz, 1H), 4.53 (d, *J =* 6.0 Hz, 2H), 6.27 (t, *J* = 6.0 Hz, 1H), 6.74 (d, *J =* 8.0 Hz, 1H), 6.85 (d, *J =* 8.0 Hz, 1H), 7.11 (s, 1H), 8.32 (s, 1H), 8.72 (bs, 1H), 9.12 (bs, 1H). ¹³C NMR (126 MHz, DMSO-d₆) δ (ppm): 23.11, 28.45, 41.08, 56.12, 65.19, 85.28, 114.40, 116.22, 120.09, 122.27, 130.50, 137.14, 142.30, 148.18, 149.1, 150.02, 150.18, 156.25, 156.43, 158.86, 160.92.

### Example 11 2-Fluoro-6-furfurylamino-9-(tetrahydro-2H-pyran-2-yl)-9H-purine

It was prepared from 2-fluoro-6-chloro-9-(tetrahydro-2*H*-pyran-2-yl)-9*H*-purine and furfurylamine, work-up using **A.** White solid, chemical formula: C₁₅H₁₆FN₅O₂, yield (%): 63. HPLC-UV/VIS retention time, purity (min., %): 25.57, 99.9. ESI⁺-MS *m*/*z* (rel. int. %): 318.1 (100.0%), 319.1 (18.1%). ¹H NMR (500 MHz, DMSO-d₆) δ (ppm): 1.54-1.58 (m, 2H), 1.65-1.69 (m, 1H), 1.93-1.96 (m, 2H), 2.18-2.21 (m, 1H), 3.68-3.72 (m, 1H), 3.95-3.94 (m, 1H), 4.62 (d, *J =* 4.0 Hz, 2H), 5.55-5.57 (m, 1H), 6.32 (d, *J =* 3.2 Hz, 1H), 6.41 (t, *J =* 3.5 Hz, 1H), 7.51 (d, *J =* 2.5 Hz, 1H), 8.42 (s, 1H), 9.15 (t, *J =* 5.5 Hz, 1H). ¹³C NMR (126 MHz, DMSO-*d*₆) δ (ppm): 22.42, 23.81, 31.13, 35.58, 66.12, 81.46, 105.53, 112.02, 119.41, 143.16, 144.07, 149.02, 150.10, 150.21, 156.35, 156.57, 157.12, 158.44, 164.08.

### Example 12 2-Fluoro-6-[(5-methylfurfuryl)amino]-9-(tetrahydro-2H-pyran-2-yl)-9H-purine

It was prepared from 2-fluoro-6-chloro-9-(tetrahydro-2*H*-pyran-2-yl)-9*H*-purine and 5-methylfurfurylamine, work-up using **B.** White solid, chemical formula: C₁₆H₁₈FN₅O₂, yield (%): 66. HPLC-UV/VIS retention time, purity (min., %): 26.98, 99.9. ESI⁺-MS *m*/*z* (rel. int. %): 332.1 (100.0%), 333.1 (17.6%). ¹H NMR (500 MHz, DMSO-*d*₆) δ (ppm): 1.52-1.57 (m, 2H), 1.64-1.70 (m, 1H), 1.92-1.96 (m, 2H), 2.11-2.21 (m, 4H), 3.68-3.71 (m, 1H), 4.02 (dd, *J =* 10.5, 1.5 Hz, 1H), 4.62 (d, *J =* 5.0 Hz, 2H), 5.68-5.72 (m, 1H), 5.83 (s, 1H), 6.15 (d, *J =* 3.5 Hz, 1H), 8.36 (s, 1H), 9.06 (t, *J =* 5.0 Hz, 1H). ¹³C NMR (126 MHz, DMSO-*d*₆) δ (ppm): 13.4, 24.25, 26.43, 31.12, 35.13, 68.11, 82.39, 105.28, 108.20, 117.55, 140.11, 147.13, 150.12, 150.29, 156.33, 156.52, 158.02, 162.74.

### Example 13 2-Fluoro-6-benzylamino-9-(tetrahydro-2H-pyran-2-yl)-9H-purine

It was prepared from 2-fluoro-6-chloro-9-(tetrahydro-2*H*-pyran-2-yl)-9*H-*purine and benzylamine, work-up using **A.** White solid, chemical formula: C₁₇H₁₈FN₅O, yield (%): 77. HPLC-UV/VIS retention time, purity (min., %): 27.28, 99.9. ESI⁺-MS *m*/*z* (rel. int. %): 328.2 (100.0%), 329.2 (20.3%). ¹H NMR (500 MHz, DMSO-*d*₆) δ (ppm): 1.50-1.54 (m, 2H), 1.56-1.59 (m, 1H), 1.88-1.91 (m, 2H), 2.11-2.19 (m, 1H), 3.60-3.62 (m, 1H), 3.90 (d, J=10.0, 1H), 5.33 (d, *J =* 5.0 Hz, 2H), 5.39 (dd, *J =* 10.0, *J' =* 2.5 Hz, 1H), 7.19-7.38 (m, 5H), 8.33 (s, 1H), 8.56 (t, *J =* 5.0 Hz, 1H). ¹³C NMR (126 MHz, DMSO-d₆) δ (ppm): 22.13, 24.93, 29.28, 46.41, 54.28, 79.52, 125.92, 127.22, 128.52, 144.14, 149.56, 150.05, 150.21, 156.24, 156.41, 157.11, 158.24, 160.85.

### Example 14 2-Fluoro-6-[(4-methylbenzyl)amino]-9-(tetrahydro-2H-pyran-2-yl)-9H-purine

It was prepared from 2,6-dichloro-9-(tetrahydro-2*H*-pyran-2-yl)-9*H*-purine and 4-methylbenzylamine, work-up using **B.** White solid, chemical formula: C₁₈H₂₀FN₅O, yield (%): 79. HPLC-UV/VIS retention time, purity (min., %): 28.63, 98.5. ESI⁺-MS *m*/*z* (rel. int. %): 342.2 (100.0%), 343.2 (19.7%). ¹H NMR (500 MHz, DMSO-*d*₆) δ (ppm): 1.58-1.62 (m, 2H), 1.62-1.68 (m, 1H), 1.84-1.89 (m, 2H), 2.11-2.24 (m, 1H), 2.36 (s, 3H), 3.54-3.59 (m, 1H), 4.06 (d, *J* = 10.5 Hz, 1H), 4.64 (d, *J* = 6.5 Hz, 2H), 5.52 (dd, *J = 10.0, J' =* 2.0 Hz, 1H), 6.96 (d, *J =* 7.0 Hz, 2H), 7.24 (d, *J =* 7.0 Hz, 2H), 8.33 (s, 1H), 8.84 (t, *J =* 6.5 Hz, 1H). ¹³C NMR (126 MHz, DMSO-d₆) δ (ppm): 19.85, 22.45, 26.84, 32.19, 39.63, 51.22, 64.95, 78.14, 114.13, 126.22, 129.80, 141.74, 150.12, 150.29, 156.33, 156.52, 157.01, 157.22, 162.48.

### Example 15 2-Fluoro-6-{[4-(trifluoromethyl)benzyl]amino}-9-(tetrahydro-2H-pyran-2-yl)-9H-purine

It was prepared from 2,6-dichloro-9-(tetrahydro-2*H*-pyran-2-yl)-9*H*-purine and 4-(trifluoromethyl)benzylamine, work-up using **A.** White solid, chemical formula: C₁₈H₁₇F₄N₅O, yield (%): 53. HPLC-UV/VIS retention time, purity (min., %): 29.12, 99.9. ESI⁺-MS *m*/*z* (rel. int. %): 396.1 (100.0%), 397.1 (19.7%). ¹H NMR (500 MHz, DMSO-*d*₆) δ (ppm): 1.52-1.58 (m, 2H), 1.64-1.68 (m, 1H), 1.91-1.95 (m, 2H), 2.16-2.24 (m, 1H), 3.64-3.68 (m, 1H), 4.02 (d, *J =* 10.5 Hz, 1H), 4.76 (d, *J =* 5.5 Hz, 2H), 5.53-5.55 (m, 1H), 7.79 (d, *J =* 8.5 Hz, 2H), 8.07 (d, *J =* 8.5 Hz, 2H), 8.36 (s, 1H), 9.17 (t, *J =* 5.5 Hz, 1H). ¹³C NMR (126 MHz, DMSO-d₆) δ (ppm): 21.59, 23.55, 31.14, 40.64, 66.29, 81.87, 120.45, 122.4, 124.9, 127.5, 127.8, 139.7, 144.1, 149.4, 153.2, 154.9.

### Example 16 2-Fluoro-6-[(3-hydroxybenzyl)amino]-9-(tetrahydro-2H-pyran-2-yl)-9H-purine

It was prepared from 2,6-dichloro-9-(tetrahydro-2*H*-pyran-2-yl)-9*H*-purine and 3-(aminomethyl)phenol, work-up using **B.** White solid, chemical formula: C₁₇H₁₈FN₅O₂, yield (%): 43. HPLC-UV/VIS retention time, purity (min., %): 24.43, 99.8. ESI⁺-MS *m*/*z* (rel. int. %): 344.2 (100.0%), 345.3 (22.0%). ¹H NMR (500 MHz, DMSO-*d*₆) δ (ppm): 1.54-1.57 (m, 2H), 1.67-1.76 (m, 1H), 1.92-1.96 (m, 2H), 2.19-2.27 (m, 1H), 3.64-3.70 (m, 1H), 3.99 (d, *J* = 11.0 Hz, 1H), 4.56 (d, *J* = 6.5 Hz, 2H), 5.53 (dd, *J =* 11.0, *J' =* 2.50 Hz, 1H), 6.61 (dd, *J* = 8.0, *J' =* 2.0 Hz, 1H), 6.71 (s, 1H), 6.73 (d, *J* = 7.50 Hz, 1H), 7.09 (t, *J* = 8.0 Hz, 1H), 8.36 (s, 1H), 8.92 (t, *J =* 6.5 Hz, 1H), 9.35 (bs, 1H). ¹³C NMR (126 MHz, DMSO-*d*₆) δ (ppm): 22.88, 25.01, 30.38, 43.57, 46.11, 68.18, 81.49, 114.32, 117.89, 118.05, 129.80, 139.82, 141.14, 150.03, 150.19, 156.37, 156.53, 157.88, 158.46, 160.08.

### Example 17 2-Fluoro-6-[(4-hydroxybenzyl)amino]-9-(tetrahydro-2H-pyran-2-yl)-9H-purine

It was prepared from 2,6-dichloro-9-(tetrahydro-2*H*-pyran-2-yl)-9*H*-purine and 4-(aminomethyl)phenol, work-up using **B.** White solid, chemical formula: C₁₇H₁₈FN₅O₂, yield (%): 61. HPLC-UV/VIS retention time, purity (min., %): 23.98, 98.0. ESI⁺-MS *m*/*z* (rel. int. %): 344.1 (100.0%), 345.1 (18.6%). ¹H NMR (500 MHz, DMSO-d₆) δ (ppm): 1.52-1.56 (m, 2H), 1.61-1.64 (m, 1H), 1.88-1.93 (m, 2H), 2.11-2.24 (m, 1H), 3.55-3.62 (m, 1H), 3.91 (d, *J* = 10.5 Hz, 1H), 4.59 (d, *J* = 5.5 Hz, 2H), 5.39 (dd, *J =* 10.5, *J' =* 2.0 Hz, 1H), 6.63 (d, *J* = 8.8 Hz, 2H), 7.69 (d, *J* = 8.8 Hz, 2H) 8.32 (s, 1H), 8.91 (t, *J* = 5.5 Hz, 1H), 9.06 (bs, 1H). ¹³C NMR (126 MHz, DMSO-d₆) δ (ppm): 21.12, 25.06, 31.12, 43.70, 67.82, 81.59, 112.52, 115.15, 122.22, 132.57, 136.13, 150.05, 150.21, 156.34, 156.52, 157.22, 158.21, 160.45.

### Example 18 2-Fluoro-6-[(3-methoxybenzyl)amino]-9-(tetrahydro-2H-pyran-2-yl)-9H-purine

It was prepared from 2,6-dichloro-9-(tetrahydro-2*H*-pyran-2-yl)-9*H*-purine and 3-methoxybenzylamine, work-up using **B.** White solid, chemical formula: C₁₈H₂₀FN₅O₂, yield (%): 55. HPLC-UV/VIS retention time, purity (min., %): 27.10, 98.0. ESI⁺-MS *m*/*z* (rel. int. %): 358.2 (100.0%), 359.2 (23.0%). ¹H NMR (500 MHz, DMSO-*d*₆) δ (ppm): 1.50-1.54 (m, 2H), 1.59-1.63 (m, 1H), 1.87-1.92 (m, 2H), 2.14-2.22 (m, 1H), 3.57-3.66 (m, 1H), 3.68 (s, 3H), 3.95 (d, *J* = 11.0 Hz, 1H), 4.57 (d, *J* = 6.0 Hz, 2H), 5.48 (dd, *J = 10.5, J' =* 2.0 Hz, 1H), 6.76 (dd, *J* = 8.0, *J' =* 2.0 Hz, 1H), 6.85-6.89 (m, 2H), 7.18 (t, *J* = 7.50 Hz, 1H), 8.32 (s, 1H), 8.91 (t, *J =* 6.0 Hz, 1H). ¹³C NMR (126 MHz, DMSO-d₆) δ (ppm): 22.88, 25.01, 30.37, 43.68, 55.31, 68.17, 81.59, 112.52, 112.97, 117.95, 119.84, 129.66, 139.88, 141.23, 150.05, 150.21, 156.34, 156.51, 158.41, 159.77, 160.03.

### Example 19 2-Fluoro-6-[(4-methoxybenzyl)amino]-9-(tetrahydro-2H-pyran-2-yl)-9H-purine

It was prepared from 2,6-dichloro-9-(tetrahydro-2*H*-pyran-2-yl)-9*H*-purine and 4-methoxybenzylamine, work-up using **B.** White solid, chemical formula: C₁₈H₂₀FN₅O₂, yield (%): 62. HPLC-UV/VIS retention time, purity (min., %): 27.10, 98.0. ESI⁺-MS *m*/*z* (rel. int. %): 358.2 (100.0%), 359.2 (21.5%). ¹H NMR (500 MHz, DMSO-*d*₆) δ (ppm): 1.51-1.55 (m, 2H), 1.57-1.62 (m, 1H), 1.87-1.92 (m, 2H), 2.15-2.21 (m, 1H), 3.59-3.65 (m, 1H), 3.67 (s, 3H), 3.94 (d, *J* = 10.5 Hz, 1H), 5.36 (d, *J* = 5.5 Hz, 2H), 5.44 (dd, *J = 10.5, J'* = 2.0 Hz, 1H), 6.86 (d, *J =* 8.5 Hz, 1H), 7.28 (d, *J =* 8.5 Hz, 2H), 8.33 (s, 1H), 8.77 (t, *J =* 5.5 Hz, 1H). ¹³C NMR (126 MHz, DMSO-d₆) δ (ppm): 22.45, 25.06, 30.16, 42.39, 47.05, 55.31, 81.28, 129.9, 131.01, 148.78, 150.14, 150.24, 152.81, 141.23, 150.05, 150.21, 156.24, 156.41, 161.29.

**Table 1. Examples of 9-(oxetan-2-yl) purine derivatives**

| | | **PURINE SUBSTITUENT** | **CHN ANALYSES** | **MS ANALYSIS** |
|---|---|---|---|---|
| **Comp.** | **C2** | **C6** | **[%C, %H, %N]** | **[M+H]⁺** |
| **1** | F | furfurylamino | 54.0, 4.2, 24.2 | 290 |
| **2** | F | benzylamino | 60.2, 4.7, 23.4 | 300 |
| **3** | F | (3-methylbut-2-en-1-yl)amino | 56.3, 5.8, 25.3 | 278 |
| **4** | F | (*E*)-(4-hydroxy-3-methylbut-2-en-1-yl)amino | 53.2, 5.5, 23.9 | 294 |
| **5** | F | (*Z*)-(4-hydroxy-3-methylbut-2-en-1-yl)amino | 53.2, 5.5, 23.9 | 294 |

**Table 2. Examples of 9-(tetrahydrofuran-2-yl) purine derivatives**

| | | **PURINE SUBSTITUENT** | **CHN ANALYSES** | **MS ANALYSIS** |
|---|---|---|---|---|
| **Comp.** | **C2** | **C6** | **[%C, %H, %N]** | **[M+H]⁺** |
| **6** | F | (E)-(4-hydroxy-2-methylbut-2-en-1-ylamino) | 54.7, 5.9, 22.8 | 308 |
| **7** | F | (Z)-(4-hydroxy-3-methylbut-2-en-1-ylamino) | 54.7, 5.9, 22.8 | 308 |
| **8** | F | (E)-(4-hydroxy-3-methylbut-2-en-1-ylamino) | 54.7, 5.9, 22.8 | 308 |
| **9** | F | (Z)-(4-hydroxy-1,3-dimethylbut-2-en-1-ylamino) | 56.1, 6.3, 21.8 | 322 |
| **10** | F | (E)-(4-hydroxy-1,3-dimethylbut-2-en-1-ylamino) | 56.1, 6.3, 21.8 | 322 |
| **11** | F | 4-hydroxy-3-methylbutylamino | 54.4, 6.5, 22.6 | 310 |
| **12** | F | isopentenylamino | 57.7, 6.2, 24.0 | 292 |
| **13** | F | 3-methylbut-2-en-1-ylamino | 57.7, 6.2, 24.0 | 292 |
| **14** | F | 2-hydroxybenzylamino | 58.3, 4.9, 21.3 | 330 |
| **15** | F | 3-hydroxybenzylamino | 58.3, 4.9, 21.3 | 330 |
| **16** | F | 4- hydroxybenzylamino | 58.3, 4.9, 21.3 | 330 |
| **17** | F | 2-methoxybenzylamino | 59.5, 5.3, 20.4 | 344 |
| **18** | F | 3-methoxybenzylamino | 59.5, 5.3, 20.4 | 344 |
| **19** | F | 4-methoxybenzylamino | 59.5, 5.3, 20.4 | 344 |
| **20** | F | 2-hydroxy-3-methoxybenzylamino | 56.8, 5.1, 19.5 | 360 |
| **21** | F | 2-hydroxy-4-methoxybenzylamino | 56.8, 5.1, 19.5 | 360 |
| **22** | F | 2-hydroxy-5-methoxybenzylamino | 56.8, 5.1, 19.5 | 360 |
| **23** | F | 2-hydroxy-3-chlorobenzylamino | 52.8, 4.2, 19.3 | 364 |
| **24** | F | 2-hydroxy-5-chlorobenzylamino | 52.8, 4.2, 19.3 | 364 |
| **25** | F | 2-hydroxy-3-fluorobenzylamino | 55.3, 4.4, 20.2 | 348 |
| **26** | F | 2-hydroxy-5-fluorobenzylamino | 55.3, 4.4, 20.2 | 348 |
| **27** | F | 3-hydroxy-5-chlorobenzylamino | 52.8, 4.2, 19.3 | 364 |
| **28** | F | 3-hydroxy-5-fluorobenzylamino | 55.3, 4.4, 20.2 | 348 |
| **29** | F | 2,3-dihydroxybenzylamino | 55.7, 4.7, 20.3 | 346 |
| **30** | F | 2,4-dihydroxybenzylamino | 55.7, 4.7, 20.3 | 346 |
| **31** | F | 2,5-dihydroxybenzylamino | 55.7, 4.7, 20.3 | 346 |
| **32** | F | 2,6-dihydroxybenzylamino | 55.7, 4.7, 20.3 | 346 |
| **33** | F | 3,4-dihydroxybenzylamino | 55.7, 4.7, 20.3 | 346 |
| **34** | F | 3,5-dihydroxybenzylamino | 55.7, 4.7, 20.3 | 346 |
| **35** | F | 2,3-dimethoxybenzylamino | 57.9, 5.4, 18.8 | 374 |
| **36** | F | 2,4-dimethoxybenzylamino | 57.9, 5.4, 18.8 | 374 |
| **37** | F | 2,5-dimethoxybenzylamino | 57.9, 5.4, 18.8 | 374 |
| **38** | F | 2,6-dimethoxybenzylamino | 57.9, 5.4, 18.8 | 374 |
| **39** | F | 3,4-dimethoxybenzylamino | 57.9, 5.4, 18.8 | 374 |
| **40** | F | 3,5-dimethoxybenzylamino | 57.9, 5.4, 18.8 | 374 |
| **41** | F | 4-hydroxy-3,5-dimethoxybenzylamino | 55.5, 5.2, 18.0 | 390 |
| **42** | F | 4-hydroxy-2,6-dimethoxybenzylamino | 55.5, 5.2, 18.0 | 390 |
| **43** | F | 4-hydroxy-3-methoxybenzylamino | 56.8, 5.1, 19.5 | 360 |
| **44** | F | 3-hydroxy-5-methoxybenzylamino | 56.8, 5.1, 19.5 | 360 |
| **45** | F | 2,3,4-trimethoxybenzylamino | 56.6, 5.5, 17.4 | 404 |
| **46** | F | 2,4,5-trimethoxybenzylamino | 56.6, 5.5, 17.4 | 404 |
| **47** | F | 2,3,4-trihydroxybenzylamino | 53.2, 4.5, 19.4 | 362 |
| **48** | F | 2,4,5-trihydroxybenzylamino | 53.2, 4.5, 19.4 | 362 |
| **49** | F | 2-hydroxy-3-methylbenzylamino | 59.5, 5.3, 20.4 | 344 |
| **50** | F | 2-hydroxy-5-methylbenzylamino | 59.5, 5.3, 20.4 | 344 |
| **51** | F | 4-hydroxy-3-methylbenzylamino | 59.5, 5.3, 20.4 | 344 |
| **52** | F | 3-hydroxy-5-methylbenzylamino | 59.5, 5.3, 20.4 | 344 |
| **53** | F | 3-hydroxyfurfurylamino | 52.7, 4.4, 21.9 | 320 |
| **54** | F | 4-hydroxyfurfurylamino | 52.7, 4.4, 21.9 | 320 |
| **55** | F | 5-hydroxyfurfurylamino | 52.7, 4.4, 21.9 | 320 |
| **56** | F | allylamino | 54.7, 5.4, 26.6 | 264 |

**Table 3. Examples of prepared 9-(tetrahydro-2H-pyran-2-yl) purine derivatives**

| **Comp.** | **C2** | **PURINE SUBSTITUENT** | **CHN ANALYSES** | **MS ANALYSIS** |
|---|---|---|---|---|
| | | **C6** | **[%C, %H, %N]** | **[M+H]⁺** |
| **57** | F | furfurylamino | 56.8, 5.1, 22.1 | 318 |
| **58** | F | (3-methylfurfuryl)amino | 58.0, 5.5, 21.1 | 332 |
| **59** | F | (4-methylfurfuryl)amino | 58.0, 5.5, 21.1 | 332 |
| **60** | F | (5-methylfurfuryl)amino | 58.0, 5.5, 21.1 | 332 |
| **61** | F | (3-methoxyfurfuryl)amino | 55.3, 5.2, 20.2 | 348 |
| **62** | F | (4-methoxyfurfuryl)amino | 55.3, 5.2, 20.2 | 348 |
| **63** | F | (5-methoxyfurfuryl)amino | 55.3, 5.2, 20.2 | 348 |
| **64** | F | benzylamino | 62.4, 5.5, 21.4 | 328 |
| **65** | F | (3-hydroxybenzyl)amino | 59.5, 5.3, 20.4 | 344 |
| **66** | F | (4-hydroxybenzyl)amino | 59.5, 5.3, 20.4 | 344 |
| **67** | F | (3-methoxybenzyl)amino | 60.5, 5.6, 19.6 | 358 |
| **68** | F | (4-methoxybenzyl)amino | 60.5, 5.6, 19.6 | 358 |
| **69** | F | (3-methylbut-2-en-1-yl)amino | 59.0, 6.6, 22.9 | 306 |
| **70** | F | (*E*)-(4-hydroxy-3-methylbut-2-en-1-yl)amino | 56.1, 6.3, 21.8 | 322 |
| **71** | F | (*Z*)-(4-hydroxy-3-methylbut-2-en-1-yl)amino | 56.1, 6.3, 21.8 | 322 |
| **72** | F | (4-hydroxy-3-methylbutyl)amino | 55.7, 6.9, 21.7 | 324 |

**Table 4. Examples of prepared 9-(oxepan-2-yl) purine derivatives**

| **Comp.** | **C2** | **PURINE SUBSTITUENT** | **CHN ANALYSES** | **MS ANALYSIS** |
|---|---|---|---|---|
| | | **C6** | **[%C, %H, %N]** | |
| **73** | F | furfurylamino | 58.0, 5.5, 21.1 | 332 |
| **74** | F | (3-methylfurfuryl)amino | 59.1, 5.8, 20.3 | 346 |
| **75** | F | (3-methoxyfurfuryl)amino | 56.5, 5.6, 19.4 | 362 |
| **76** | F | benzylamino | 63.3, 5.9, 20.5 | 342 |
| **77** | F | (3-hydroxybenzyl)amino | 60.5, 5.6, 19.6 | 358 |
| **78** | F | (3-methoxybenzyl)amino | 61.4, 6.0, 18.9 | 372 |
| **79** | F | (3-methylbut-2-en-1-yl)amino | 60.2, 6.9, 21.9 | 320 |
| **80** | F | (*E*)-(4-hydroxy-3-methylbut-2-en-1-yl)amino | 57.3, 6.6, 20.9 | 336 |
| **81** | F | (*Z*)-(4-hydroxy-3-methylbut-2-en-1-yl)amino | 57.3, 6.6, 20.9 | 336 |
| **82** | F | (4-hydroxy-3-methylbutyl)amino | 57.0, 7.2, 20,8 | 338 |

### Example 20 In vitro cytotoxic activity of novel compounds

Low cytotoxicity of the compounds is the major property determining their agricultural use. One of the parameters used, as the basis for cytotoxicity assays, is the metabolic activity of viable cells. For example, a microtiter assay, which uses the Calcein AM, is now widely used to quantitate cell proliferation and cytotoxicity. For instance, this assay is used in drug screening programs and in chemosensitivity testing. Because only metabolically active cells cleave Calcein AM, these assays detect viable cells exclusively. The quantity of reduced Calcein AM corresponds to the number of vital cells in the culture. Mouse fibroblasts NIH3T3; mouse immortalized bone marrow macrophages B2.4 and B10A.4, and BJ (human foreskin fibroblasts) were used for routine screening of compounds. The cells were maintained in Nunc/Corning 80 cm² plastic tissue culture flasks and cultured in cell culture medium (DMEM with 5 g/l glucose, 2 mM glutamine, 100 U/ml penicillin, 100 µg/ml streptomycin, 10% fetal calf serum and sodium bicarbonate). The cell suspensions that were prepared and diluted according to the particular cell type and the expected target cell density (2.500-30.000 cells per well based on cell growth characteristics) were added by pipette (80 µl) into 96/well microtiter plates. Inoculates were allowed a pre-incubation period of 24 hours at 37 °C and 5% CO₂ for stabilisation. Four-fold dilutions of the intended test concentration were added at time zero in 20 µl aliquots to the microtiter plate wells. Usually, the compound tested was evaluated at six 4-fold dilutions. In routine testing, the highest well concentration was 166.7 µM, but it can be the matter of change dependent on the agent. All compound concentrations were examined in duplicates. Incubations of cells with the tested compounds lasted for 72 hours at 37 °C, in 5% CO₂ atmosphere and 100% humidity. At the end of the incubation period, the cells were assayed by using Calcein AM. Ten microliters of the stock solution were pipetted into each well and incubated for 1 hour. Fluorescence (FD) was measured with the Labsystem FIA Reader Fluoroscan Ascent (UK). The tumour cell survival (GI₅₀) was calculated using the following equitation: TCS=(FD_{drug exposed well} / mean FD_{control wells}) x 100%. The IC₅₀ value, the compound concentration lethal to 50 % of the cells, was calculated from the obtained dose response curves (Table 5). Zero cytotoxicity is a basic prerequisite for the use of these substances in cosmetical applications. To assess the antitumor activity, the toxicity of new derivatives on panels containing cell lines of different histogenic and species origin was tested (Table 5). It has been shown that for all tested tumor lines the action of the new compounds was comparable, whereas non-malignant cell lines, NIH3T3 fibroblasts and normal human lymphocytes were resistant to this effect. The compounds listed in Table 5 can be divided into 2 groups.

The first group contains "classical cytokinins" represented by 6-substituted purines (their effects are already known). The second group includes novel 2-fluoro-N6-substituted-9-(2-oxacycloalkyl)-9H-purine-6-amine derivatives. These results suggest that substitution at the 2-position of the purine skeleton by fluorine generally results in a decrease in cytotoxic activity compared to "classical cytokinin" analogues. As shown in Table 5, GI50 for NIH3T3 fibroblasts and normal human lymphocytes was always greater than 166.7 µM. The new derivatives show zero toxicity for both normal and tumor cells at concentrations of about 166.7 µM and are therefore much more suitable for agricultural and cosmetic applications than "classical cytokinins" (6-substituted purine derivatives). Low cytotoxicity (high IC₅₀ value) of the compounds is the basic prerequisite for cosmetic and medical applications. Zero cytotoxic activity was found for 2-fluoro-N⁶-substituted-9-(2-oxacycloalkyl)-9H-purine-6-amine derivatives in comparison to classical cytokinins known in prior art (such as kinetin, isopentenyladenine, etc.).

**Table 5: Cytotoxicity of novel compounds for different normal cell lines tested / IC₅₀ (µmol/L)**

| **Compound** | **B10A.4** | **B10A.4** | **BJ** | **NIH-3T3** |
|---|---|---|---|---|
| **Kinetin** | >166.7 | 164.1 | 147.5 | 132.8 |
| **isopentenyladenine** | >166.7 | 146.9 | 134.1 | 123.7 |
| **6-benzyladenine** | >166.7 | 138.9 | 128.7 | 112.6 |
| ***trans-zeatin*** | >166.7 | >166.7 | >166.7 | 154.9 |
| ***meta-*topolin** | >166.7 | 178.4 | 165.7 | 167.2 |
| ***ortho*-topolin** | 87.5 | 94.3 | 107.8 | 94.1 |
| **Adenine** | >166.7 | >166.7 | >166.7 | >166.7 |
| **6** | >166.7 | | >166.7 | >166.7 |
| **11** | 146.2 | | >166.7 | 138.7 |
| **13** | >166.7 | >166.7 | >166.7 | >166.7 |
| **14** | >166.7 | >166.7 | >166.7 | >166.7 |
| **15** | >166.7 | | >166.7 | >166.7 |
| **16** | >166.7 | | >166.7 | >166.7 |
| **17** | >166.7 | | >166.7 | >166.7 |
| **18** | >166.7 | | >166.7 | >166.7 |
| **20** | 124.1 | 126.3 | 136.7 | >141.2 |
| **21** | 146.8 | | 132.1 | 158.2 |
| **29** | >166.7 | | >166.7 | >166.7 |
| **33** | >166.7 | | >166.7 | >166.7 |
| **34** | >166.7 | | >166.7 | >166.7 |
| **36** | >166.7 | | >166.7 | >166.7 |
| **37** | >166.7 | | >166.7 | >166.7 |
| **38** | >166.7 | | >166.7 | >166.7 |
| **39** | >166.7 | | >166.7 | >166.7 |
| **40** | >166.7 | | >166.7 | >166.7 |
| **41** | >166.7 | | >166.7 | >166.7 |
| **42** | >166.7 | | >166.7 | >166.7 |
| **43** | >166.7 | | >166.7 | >166.7 |
| **44** | >166.7 | | >166.7 | >166.7 |
| **49** | 156.3 | | 161.2 | >166.7 |
| **50** | >166.7 | | >166.7 | >166.7 |
| **51** | >166.7 | | >166.7 | >166.7 |
| **52** | >166.7 | | >166.7 | >166.7 |
| **53** | >166.7 | | >166.7 | >166.7 |
| **55** | >166.7 | | >166.7 | >166.7 |
| **57** | 148.7 | | 132.3 | 138.5 |
| **60** | >166.7 | | >166.7 | >166.7 |
| **63** | >166.7 | | >166.7 | >166.7 |
| **64** | 121.2 | | 124.4 | 127.6 |
| **65** | >166.7 | | >166.7 | >166.7 |
| **66** | >166.7 | | >166.7 | >166.7 |
| **67** | >166.7 | | >166.7 | >166.7 |
| **68** | >166.7 | | >166.7 | >166.7 |
| **69** | 146.5 | | 152.8 | 141.9 |
| **70** | >166.7 | | >166.7 | >166.7 |
| **71** | >166.7 | | >166.7 | >166.7 |
| **72** | >166.7 | | >166.7 | >166.7 |

### Example 21 Ames Test

A number of tests related to the safety of new derivatives (compounds No. **6**, **15, 17, 18, 27, 28, 57, 64, 65, 67, 68, 71, 73)** have been carried out using conventionally accepted protocols and procedures. The results of these studies are summarized herein. Tests were carried out using DMSO as solvent and dose levels of new compounds at 2.5, 5.0, 15, 50, 500, 1500, and 5000 µg/plate based upon standard protocol and procedures (Ames et al., Mutation Research, 31, 347-364, 1975; Maron et al., Mutation Research, 113, 173-215, 1983). Using *Salmonella typhimurium* histidin auxotrophs TA98 and TA100 in the presence and absence of Aroclor-induced rat liver S9, no positive mutagenic responses were observed with tetrahydropyranyl derivatives at the levels tested.

### Example 22 Arabidopsis root growth inhibition test

*Arabidopsis thaliana* seeds (ecotype Columbia) were sterilized with 70% ethanol and 0.1% Triton X-100 and then washed 4 times with sterile water. The seeds were transferred to Petri dishes on a solid medium consisting of MS (2.2 g l⁻¹), sucrose (10 g l⁻¹), agar (8 g l⁻¹), and supplemented with cytokinins or DMSO control (0.1% DMSO). After incubation for 3 days in darkness at 4°C, the seeds were germinated and grown horizontally in an environmental chamber at 22/20 °C under an 8 h light (120 µmol photons m⁻² s⁻¹)/16 h dark cycle for 4 days. 7-d-old seedling were used for measurements of the primary root length. At least 35 plants were used for each treatment and each treatment was repeated in at least 5 individual Petri dishes. The length of the primary root was evaluated using Scion Image software (Scion Corporation, Frederick, MD, USA). All measured parameters were statistically evaluated between untreated and treated plants according to Student's unpaired t-test. WT treated with BAP significantly inhibited root growth when compared to WT, followed by m-topolin which is a precursor of the most effective compound in the assay (it means that did not caused the inhibition of root) 2-F-THP-mT (compound **65**). Compared to BAP and WT, 2-F-6-(3MeOBA)-9-THP (compound **67**) also did not cause significant root inhibition which is also shown in
Graph at Fig. 1.

**Tab. Inhibition of the primary root growth in 7-d-old Arabidopsis plants. 100% = WT plants, DMSO treated.**

| | Tested compound | Tested concentration (µmol.l⁻¹) | Primary root length 7-d, WT = 100% |
|---|---|---|---|
| 1. | *m*-topolin | 0.04 | 67.1 (±7.2) |
| 2. | *m*-topolin | 1 | 33.9 (±2.0) |
| 3. | *m*-topolin | 25 | 19.3 (±2.3) |
| 4. | BAP | 0.04 | 70.2 (±1.5) |
| 5. | BAP | 1 | 18.8 (±3.6) |
| 6. | BAP | 25 | 9.8 (±0.8) |
| 7. | 2F-6-(3MeOBA)-9-THP **(67)** | 0.04 | 96.9 (±2.4) |
| 8. | 2F-6-(3MeOBA)-9-THP **(67)** | 1 | 50.3 (±1.1) |
| 9. | 2F-6-(3MeOBA)-9-THP **(67)** | 25 | 34.9 (±2.1) |
| 10. | 2F-THP-mT **(65)** | 0.04 | 102.9 (±11.0) |
| 11. | 2F-THP-mT **(65)** | 1 | 40.6 (±2.1) |
| 12. | 2F-THP-mT **(65)** | 25 | 31.3 (±1.8) |

### Example 23 Effect on the root proliferation of Arabidopsis thaliana

*Arabidopsis thaliana* seeds (ecotype Columbia) were sterilized with 70% ethanol and 0.1% Triton X-100 and then washed with 70% ethanol containing 0.01% Triton X-100. Then the seeds were transferred to vertical square petri dishes (11 seeds per dish) on a solid medium consisting of MS (2.15 g/l), MES (0.5 g/l), sucrose (1 g/l), agar (11 g/l), buffered to pH 5.8, and various concentrations of tested compounds. The compounds were dissolved in DMSO, thus 0.05% DMSO was used as a control solution. After incubation for 3 days in darkness at 4°C, the seeds were germinated and grown in an environmental chamber (fluorescence light intensity 150 µE m⁻² s⁻¹, humidity 55%, 16 h day/8 h night, 22°C). After 7 and 14 days, the length of the primary root was evaluated using Scion Image software (Scion Corporation, Frederick, MD, USA). The number of lateral roots was scored under a stereomicroscope. Application of compound **67** into the MS media does not have negative effect on elongation of primary root in a range of concentration 0.04 to 5 µM. On the contrary, classical cytokinin 6-(3-methoxybenzylamino)purine (precursor cytokinin for **67**) known from prior art significantly inhibits root elongation and branching already in micromolar concentration (Fig. 2, Fig. 3AB). In addition, compounds with tetrahydropyranyl protection at N9 slightly increase the number of emerged lateral roots (Fig 3C). Arabidopsis seedlings grown on MS media with added N9 protected cytokinins do not show distinguishable changes (reduction) in number and size of emerged leaves in contrast to cytokinin with free N9 group. The adenine derivatives known in the art as growth regulators inhibit *Arabidopsis* root growth and branching in more then 10 nanomolar concentration (10⁻⁸ mol.l⁻¹ kinetin); 9-tetrahydropyranyl derivatives show this negative effect in several orders of magnitude higher concentrations or not at all (Table 6).

**Table 6: The inhibitory effect of novel 2-fluoro-N6-substituted-9-(2-oxacycloalkyl)-9H-purine-6-amine derivatives on the growth of Arabidopsis roots - number of lateral roots (% of control). Kinetin (a classical cytokinin) activity at 10⁻⁸ mol.l⁻¹ was 100%.**

| In the tables provided below, R6 is the substituent at N6 and R9 is the substituent at N9. | | | | |
|---|---|---|---|---|
| No. | Tested compound | | Concentration with highest activity (mol.l⁻¹) | Activity (%) [10⁻⁸ mol.l⁻¹ Kin = 100%] |
| | R6 | R9 | | |
| **6** | (E)-(4-hydroxy-2-methylbut-2-en-1-ylamino) | tetrahydrofuran-2-yl | 10⁻⁵ | 48.5 (± 6) |
| **15** | 3-hydroxybenzylamino | tetrahydrofuran-2-yl | 10⁻⁵ | 24.8 (± 4) |
| **17** | 2-methoxybenzylamino | tetrahydrofuran-2-yl | 10⁻⁵ | 30.7 (± 7) |
| **18** | 3-methoxybenzylamino | tetrahydrofuran-2-yl | 10⁻⁵ | 28.6 (± 2) |
| **27** | 3-hydroxy-5-chlorobenzylamino | tetrahydrofuran-2-yl | 10⁻⁵ | 45.7 (± 6) |
| **28** | 3-hydroxy-5-fluorobenzylamino | tetrahydrofuran-2-yl | 10⁻⁵ | 49.3 (± 9) |
| **57** | furfurylamino | tetrahydrofuran-2-yl | 10⁻⁶ | 68.4 (± 8) |
| **64** | benzylamino | tetrahydrofuran-2-yl | 10⁻⁶ | 61.2 (± 3) |
| **65** | 3-hydroxybenzylamino | tetrahydropyran-2-yl | 10⁻⁵ | 32.6 (± 6) |
| **67** | 3-methoxybenzylamino | tetrahydropyran-2-yl | 10⁻⁵ | 35.8 (± 6) |
| **67** | 4-methoxybenzylamino | tetrahydropyran-2-yl | 10⁻⁵ | 29.4 (± 4) |
| **71** | (E)-(4-hydroxy-3-methylbut-2-en-1-yl)amino | tetrahydropyran-2-yl | 10⁻⁶ | 71.8 (± 7) |
| **72** | (4-hydroxy-3-methylbutyl)amino | tetrahydropyran-2-yl | 10⁻⁶ | 68.5 (± 2) |

### Example 24 Effect on development of Zea mays seedlings

Maize seeds (*Zea mays* cv. CELLUX) were imbibed in tap water and germinated on a wetted filter paper. After 3 days, the germinated seedlings were transferred to aerated hydroponic tanks filled with Hoagland's nutrient solution. The plants were grown in an environmental chamber with 16 h light period (250 µE m⁻² s⁻¹) at 27°C and 8 h dark period at 20°C. Tested compounds diluted in DMSO were added to nutrient solution immediately when seedlings were nested in the tanks or after two additional days of cultivation. Control plants were grown in parallel with addition of corresponding amount of DMSO (up to 0.01%). Root and leaf tissues of ten plants were harvested at different time points after the application of the compounds, immediately frozen in liquid nitrogen and used for cytokinin content and transcript level analysis. Length of primary root and the first leaf were determined with ruler on sets of twelve plants, each cultivated in the independent tank (for every cytokinin application repeated ten times). Dry mass of root system and the aerial part was determined after cutting off the kernel and desiccation in dryer for two days at 60°C. Tetrahydropyranyl substituted 2F-3MeOBAPTHP (compound **67**) and closely related 2F-BAPTHP (compound **64**) together with its free base (6-(3-methoxybenzylamino)purine, 3MeOBAP) were added to Hoagland nutrition solution to observe their effect on maize seedlings development. Similarly as on *Arabidopsis* plantlets, addition of compound **13,** up to 1 µM concentration, does not inhibit root elongation and lateral root proliferation (Fig. 4). Positive effect of tetrahydropyranyl group at N9 of 2F-3MeOBAP on lateral root proliferation and elongation of primary root was observed in the nanomolar concentrations (8 to 40 nM) when less seedlings were grown in the nutrient solution (10 seedlings per 1 liter of solution; Fig. 4) and up to 1 micromolar concentration when more seedlings were in the solution (approx. 50 seedlings per 1 liter; Fig. 5). Positive effect of compound **67** on cell division and organ growth was observed in both the root system as well as on the aerial part of seedlings (Table 7 and 8). It is expressed as an increase in the dry mass of roots and leaves and total length of primary root and the first emerged leaf (Fig. 6, 7). The classical cytokinin growth regulators (kinetin, BAP, 3MeOBAP, 2F-3MeOBAP, etc.) known in the art inhibit maize root growth and branching in high nanomolar concentrations; the 2-fluoro-N⁶-substituted-9-(2-oxacycloalkyl)-9H-purine-6-amine derivatives derivatives show only slight inhibitory effect and in several orders of magnitude higher concentrations. Moreover 2-fluoro-N⁶-substituted-9-(2-oxacycloalkyl)-9H-purine-6-amine derivatives show slight stimulatory effect on root and shoot mass production in nanomolar concentration; the adenine-based growth regulators known in the prior art do not show this effect at all.

**Table 7: The effect of novel 2-fluoro-N6-substituted-9-(2-oxacycloalkyl)-9H-purine-6-amine derivatives on the growth of Arabidopsis roots - dry root mass (%) relative to control (4 x 10⁻⁸ mol.l⁻¹ Kin = 100%).**

| No. | Tested compound | | Concentration with highest activity (mol.1⁻¹) | Root dry mass (%) [4 x 10⁻⁸ mol.l⁻¹ Kin = 100%] |
|---|---|---|---|---|
| | R6 | R9 | | |
| **6** | (E)-(4-hydroxy-2-methylbut-2-en-1-ylamino) | tetrahydrofuran-2-yl | 10⁻⁷ | 167.2 (± 14) |
| **15** | 3-hydroxybenzylamino | tetrahydrofuran-2-yl | 10⁻⁷ | 201.4 (± 22) |
| **17** | 2-methoxybenzylamino | tetrahydrofuran-2-yl | 10⁻⁷ | 241.5 (± 19) |
| **18** | 3-methoxybenzylamino | tetrahydrofuran-2-yl | 10⁻⁷ | 240.6 (± 22) |
| **27** | 3-hydroxy-5-chlorobenzylamino | tetrahydrofuran-2-yl | 10⁻⁷ | 194.5 (± 18) |
| **28** | 3-hydroxy-5-fluorobenzylamino | tetrahydrofuran-2-yl | 10⁻⁷ | 190.2 (± 20) |
| **57** | furfurylamino | tetrahydrofuran-2-yl | 10⁻⁷ | 183.9 (± 24) |
| **64** | benzylamino | tetrahydrofuran-2-yl | 10⁻⁷ | 168.9 (± 16) |
| **65** | 3-hydroxybenzylamino | tetrahydropyran-2-yl | 10⁻⁷ | 187.3 (± 18) |
| **67** | 2-methoxybenzylamino | tetrahydropyran-2-yl | 10⁻⁷ | 243.7 (± 16) |
| **67** | 3-methoxybenzylamino | tetrahydropyran-2-yl | 10⁻⁷ | 261.2 (± 20) |
| **71** | (E)-(4-hydroxy-3-methylbut-2-en-1-yl)amino | tetrahydropyran-2-yl | 10⁻⁷ | 172.5 (± 14) |
| **72** | (4-hydroxy-3-methylbutyl)amino | tetrahydropyran-2-yl | 10⁻⁷ | 161.3 (± 21) |

**Table 8: The stimulatory effect of novel compounds on the growth of Arabidopsis shoots - dry shoot mass (%) relative to control (10⁻⁶ mol.1⁻¹ Kin = 100%)..**

| No. | Tested compound | | Concentration with highest activity (mol.1⁻¹) | Shoot dry mass (%) [10⁻⁶ mol.l⁻¹ Kin = 100%] |
|---|---|---|---|---|
| | R6 | R9 | | |
| **6** | (E)-(4-hydroxy-2-methylbut-2-en-1-ylamino) | tetrahydrofuran-2-yl | 10⁻⁶ | 121.2 (± 6) |
| **15** | 3-hydroxybenzylamino | tetrahydrofuran-2-yl | 10⁻⁶ | 124.3 (± 8) |
| **17** | 2-methoxybenzylamino | tetrahydrofuran-2-yl | 10⁻⁶ | 139.8 (± 7) |
| **18** | 3-methoxybenzylamino | tetrahydrofuran-2-yl | 10⁻⁶ | 148.2 (± 8) |
| **27** | 3-hydroxy-5-chlorobenzylamino | tetrahydrofuran-2-yl | 10⁻⁶ | 116.3 (± 7) |
| **28** | 3-hydroxy-5-fluorobenzylamino | tetrahydrofuran-2-yl | 10⁻⁶ | 111.8 (± 6) |
| **57** | furfurylamino | tetrahydropyran-2-yl | 10⁻⁶ | 112.8 (± 11) |
| **64** | benzylamino | tetrahydropyran-2-yl | 10⁻⁶ | 108.9 (± 8) |
| **65** | 3-hydroxybenzylamino | tetrahydropyran-2-yl | 10⁻⁶ | 121.6 (± 7) |
| **67** | 3-methoxybenzylamino | tetrahydropyran-2-yl | 10⁻⁶ | 127.6 (± 9) |
| **68** | 4-methoxybenzylamino | tetrahydropyran-2-yl | 10⁻⁶ | 118.6 (± 9) |
| **71** | (E)-(4-hydroxy-3-methylbut-2-en-1-yl)amino | tetrahydropyran-2-yl | 10⁻⁶ | 111.1 (± 6) |
| **72** | (4-hydroxy-3-methylbutyl)amino | tetrahydropyran-2-yl | 10⁻⁶ | 104.8 (± 7) |

### Example 24 Senescence inhibition by novel compounds tested on sinter wheat leaf segments

Seeds of winter wheat, *Triticum aestivum* cv. Hereward, were washed under running water for 24 hours and then sown on vermiculite soaked with the Knop's solution. They were placed in the grow chamber at 25 °C with a 16/8 h light period at 50 µmol.m⁻².s⁻¹. After 7 days, the first leaf was fully developed and the second leaf had started to grow. A tip section of the first leaf, approximately 35 mm long, was removed from 5 seedlings and trimmed slightly to a combined weight of 100 mg. The basal ends of the five leaf tips were placed in the wells of a microtiter polystyrene plate containing 150 µl of the solution of the tested compound each. The entire plate was inserted into a plastic box lined with paper tissues soaked with distilled water to prevent leaf sections from drying out. After 96 h incubation in the dark at 25 °C, the leaves were removed and chlorophyll was extracted by heating at 80 °C for 10 min in 5 ml of 80 % ethanol (v/v). The sample volume was then restored to 5 ml by the addition of 80 % ethanol (v/v). The absorbance of the extract was recorded at 665 nm. In addition, chlorophyll extracts from fresh leaves and leaf tips incubated in deionised water were measured. From the obtained data, the concetration with the highest activity was selected for each compound tested. Relative activity of the compound at this concentration was calculated (Table 7). The activity obtained for 10⁻⁴ M 6-furfurylamino-9-tetrahydropyranylpurine (KTHP) was postulated as 100%. The values shown are means of five replicates and the whole experiment was repeated twice. The compounds to be tested were dissolved in dimethylsulfoxide (DMSO) and the solution brought up to 10⁻³ M with distilled water. This stock solution was further diluted with distilled water to a concentration ranging from 10⁻⁸ M to 10⁻⁴ M. The final concentration of DMSO did not exceed 0.2 % and therefore did not affect the biological activity in the assay system used. The compounds listed in Table 7 can be divided into 2 groups. The first group contains "classical 6,9-disubstituted purines (KTHP as a control)" known in prior art. The results show that the substitution in position 2 by fluorine of the purine skeleton generally led to an increase of the antisenescent activity in comparison to the corresponding "classical cytokinin" analogues.

**Table 9: The effect of novel 2-fluoro-N6-substituted-9-(2-oxacycloalkyl)-9H-purine-6-amine derivatives on the retention of chlorophyll in excised wheat leaf tips (standard deviations of the mean for 10 replicate determinations are shown)**

| No. | Tested compound | | Concentration with highest activity (mol.l⁻¹) | Activity (%) [10⁻⁴mol.l⁻¹ KTHP = 100%] |
|---|---|---|---|---|
| | R6 | R9 | | |
| **KTHP** | furfurylamino | tetrahydropyran-2-yl | 10⁻⁴ | 100 (± 8.6) |
| **6** | (E)-(4-hydroxy-3-methylbut-2-en-1-ylamino) | tetrahydrofuran-2-yl | 10⁻⁴ | 138.2 (± 17) |
| **11** | 4-hydroxy-3-methylbutylamino | tetrahydrofuran-2-yl | 10⁻⁴ | 125 (± 12) |
| **13** | 3-methylbut-2-en-1-ylamino | tetrahydrofuran-2-yl | 10⁻⁴ | 109.8 (± 13) |
| **14** | 2-hydroxybenzylamino | tetrahydrofuran-2-yl | 10⁻⁴ | 53.6 (± 7) |
| **15** | 3-hydroxybenzylamino | tetrahydrofuran-2-yl | 10⁻⁴ | 133.1 (± 15) |
| **16** | 4-hydroxybenzylamino | tetrahydrofuran-2-yl | 10⁻⁴ | 110.1 (± 19) |
| **17** | 2-methoxybenzylamino | tetrahydrofuran-2-yl | 10⁻⁴ | 104.3 (± 19) |
| **18** | 3-methoxybenzylamino | tetrahydrofuran-2-yl | 10⁻⁴ | 175.8 (± 10) |
| **20** | 2-hydroxy-3-methoxybenzylamino | tetrahydrofuran-2-yl | 10⁻⁴ | 50 (± 5) |
| **21** | 2-hydroxy-4-methoxybenzylamino | tetrahydrofuran-2-yl | 10⁻⁴ | 35 (± 9.5) |
| **29** | 2,5-dihydroxybenzylamino | tetrahydrofuran-2-yl | 10⁻⁴ | 15 (±5) |
| **33** | 3,4-dihydroxybenzylamino | tetrahydrofuran-2-yl | 10⁻⁴ | 71.3 (± 17) |
| **34** | 3,5-dihydroxybenzylamino | tetrahydrofuran-2-yl | 10⁻⁴ | 145 (± 12) |
| **36** | 2,4-dimethoxybenzylamino | tetrahydrofuran-2-yl | 10⁻⁴ | 125.6 (± 15) |
| **37** | 2,5-dimethoxybenzylamino | tetrahydrofuran-2-yl | 10⁻⁴ | 121.8 (± 17) |
| **38** | 2,6-dimethoxybenzylamino | tetrahydrofuran-2-yl | 10⁻⁴ | 128 (± 16) |
| **39** | 3,4-dimethoxybenzylamino | tetrahydrofuran-2-yl | 10⁻⁴ | 126 (± 21) |
| **40** | 3,5-dimethoxybenzylamino | tetrahydrofuran-2-yl | 10⁻⁴ | 163 (± 8) |
| **41** | 4-hydroxy-3,5-dimethoxybenzylamino | tetrahydrofuran-2-yl | 10⁻⁴ | 42 (± 13) |
| **42** | 4-hydroxy-2,6-dimethoxybenzylamino | tetrahydrofuran-2-yl | 10⁻⁴ | 22 (± 4) |
| **43** | 4-hydroxy-3-methoxybenzylamino | tetrahydrofuran-2-yl | 10⁻⁴ | 55 (± 18) |
| **44** | 3-hydroxy-5-methoxybenzylamino | tetrahydrofuran-2-yl | 10⁻⁴ | 47 (± 11) |
| **49** | 2-hydroxy-3-methylbenzylamino | tetrahydrofuran-2-yl | 10⁻⁴ | 16.4 (± 3) |
| **50** | 2-hydroxy-5-methylbenzylamino | tetrahydrofuran-2-yl | 10⁻⁴ | 82 (± 12) |
| **51** | 4-hydroxy-3-methylbenzylamino | tetrahydrofuran-2-yl | 10⁻⁴ | 19 (± 2) |
| **52** | 3-hydroxy-5-methylbenzylamino | tetrahydrofuran-2-yl | 10⁻⁴ | 23 (± 1) |
| **53** | 3-hydroxyfurfurylamino | tetrahydrofuran-2-yl | 10⁻⁴ | 45 (± 13) |
| **55** | 3-hydroxyfurfurylamino | tetrahydrofuran-2-yl | 10⁻⁴ | 101 (± 17) |
| **57** | furfurylamino | tetrahydropyran-2-yl | 10⁻⁴ | 111 (± 14) |
| **60** | (5-methylfurfuryl)amino | tetrahydropyran-2-yl | 10⁻⁴ | 123 (± 7) |
| **63** | (5-methoxyfurfuryl)amino | tetrahydropyran-2-yl | 10⁻⁴ | 97 (± 5) |
| **64** | benzylamino | tetrahydropyran-2-yl | 10⁻⁴ | 110 (± 12) |
| **65** | 3-hydroxybenzylamino | tetrahydropyran-2-yl | 10⁻⁴ | 128 (± 19) |
| **66** | 4-hydroxybenzylamino | tetrahydropyran-2-yl | 10⁻⁴ | 74 (± 4) |
| **67** | 3-methoxybenzylamino | tetrahydropyran-2-yl | 10⁻⁴ | 195 (± 16) |
| **68** | 4-methoxybenzylamino | tetrahydropyran-2-yl | 10⁻⁴ | 155 (± 14) |
| **69** | (3-methylbut-2-en-1-yl)amino | tetrahydropyran-2-yl | 10⁻⁴ | 108 (± 13) |
| **70** | (E)-(4-hydroxy-3-methylbut-2-en-1-ylamino) | tetrahydropyran-2-yl | 10⁻⁴ | 126 (± 12) |
| **71** | (Z)-(4-hydroxy-3-methylbut-2-en-1-ylamino) | tetrahydropyran-2-yl | 10⁻⁴ | 113 (± 18) |
| **72** | (4-hydroxy-3-methylbutyl)amino | tetrahydropyran-2-yl | 10⁻⁴ | 103 (± 7) |

### Example 25 The effect of novel 2-fluoro-N⁶-substituted-9-(2-oxacycloalkyl)-9H-purine-6-amine derivative in field trials with vegetables

The compound 2-fluoro-6-[(3-methoxylbenzyl)amino]-9-(tetrahydro-2*H*-pyran-2-yl)-9*H*-purine ("tested compound") was used for hydroponically grown vegetables and for sweet peppers, cucumbers and tomatoes grown in soil conditions (in open 15 l pots in open greenhouse) in the years 2010, 2014, 2015 and 2017 and the results are shown in Table 10. Besides, plants were treated with a commercial fertilizer (type Kristalon) with optimal ratio of N,P,K and micronutrients. Dosage of 2-fluoro-6-[(3-methoxylbenzyl)amino]-9-(tetrahydro-2*H*-pyran-2-yl)-9*H*-purine was in the range between 10 - 100 nM, but usually 50 or 100 nM from re-planting to the end of vegetation once a week 500 ml to each plant (Table 10). The application of the compound was performed 1-3 times per week (according to the cultivation system - field or hydroponic). Stock solution was prepared in the concentration range 1-5 µM, then dosing into hydroponic system, watering with fertilizer, or in concentration 100-1000 mM in final fertilizer for use in soil conditions. Number of replicates was 10 / 12 plants from each variant (including control).

The used treatment (tested compound) had a positive effect on fertility term elongation of vegetables (longer harvest period), on increasing number of fruits, and protection against abiotic stress. In case of cucumbers, fruit weight harvested per plant varied from 99,6 / 110,0 %, number of fruits were 102,3 / 106,8 % compared to control. Fruit weight from 1 plant varied from 100,0 to 124,7 % and number of fruits varied from 92,9 to 143,2 %, compared to control, after application of the tested compound to sweet peppers. In most trials, the size of fruits increased after the application of the tested compound.

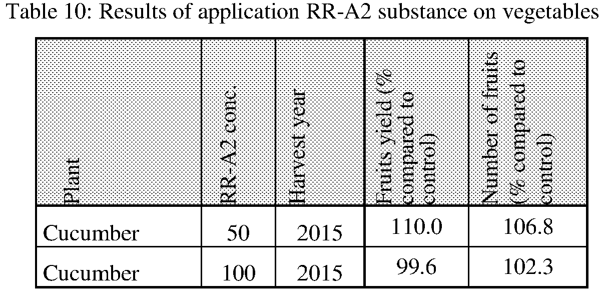

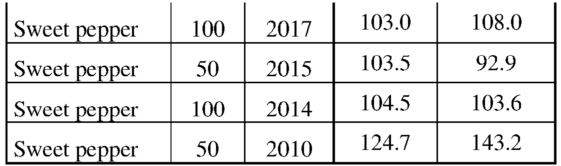

### Example 26 Formulations

The growth regulatory formulations usually contain from 0.1 to 99 % by weight, especially from 0.1 to 95 % by weight, of active ingredient mixture comprising a compound of formula I, from 1 to 99.9% by weight of a solid or liquid formula- tion adjuvant, and from ° to 25 % by weight, especially from 0.1 to 25 % by weight, of a surfactant. Whereas commercial products are usually formulated as concentrates, the end user will normally employ dilute formulations. The compositions may also comprise further ingredients, such as stabilisers, e.g. vegetable oils or epoxidised vegetable oils (epoxidised coconut 0;1, rapeseed *oil* or soybean oil), antifoams, e.g. silicone oil, preservatives, viscosity regulators, binders, tackifiers, and also fertilisers or other active ingredients. Preferred formulations have especially the following compositions: (% = percent by weight)

| A1. Emulsifiable concentrates | a) | b) | c) | d) |
|---|---|---|---|---|
| active ingredient mixture | 5% | 10% | 25% | 50% |
| calcium dodecylbenzenesulfonate | 6% | 8% | 6% | 8% |
| castor oil polyglycol ether (36 mol of ethylene oxide) | 4% | - | 4% | 4% |
| octylphenol polyglycol ether (7-8 mol of ethylene oxide) | - | 4 % | - | 2 % |
| cyclohexanone | - | - | 10 % | 20 % |
| arom. hydrocarbon mixture | 85 % | 78 % | 55 % | 16 % |
| C₉-C₁₂ | | | | |

Emulsions of any desired concentration can be obtained from such concentrates by dilution with water.

| A2. Solutions | a) | b) | c) | d) |
|---|---|---|---|---|
| active ingredient mixture | 5% | 10 % | 50% | 90% |
| 1-methoxy-3-( 3-methoxy-propoxy)-propane | | 20 % | 20% | - |
| polyethylene glycol MW 400 | 20% | 10 % | - | - |
| N-methyl-2-pyrrolidone | | - | 30% | 10% |
| arom. hydrocarbon mixture | 75 % | 60 % | - | - |
| C₉-C₁₂ | | | | |

The solutions are suitable for use in the form of microdrops.

| A3. Wettable powders | a) | b) | c) | d) |
|---|---|---|---|---|
| active ingredient mixture | 5% | 25 % | 50 *%* | 80 % |
| sodium lignosulfonate | 4% | - | 3% | - |
| sodium lauryl sulfate | 2 % | 3% | - | 4 % |
| sodium diisobutylnaphthalenesulfonate | - | 6% | 5 % | 6 % |
| octylphenol polyglycol ether (7-8 mol of ethylene oxide) | - | 1% | 2 % | - |
| highly dispersed silicic acid | 1 % | 3% | 5 % | 10 % |
| kaolin | 88 % | 62 % | 35 % | - |

The active ingredient is mixed thoroughly with the adjuvants and the mixture is thoroughly ground in a suitable mill, affording wettable powders which can be diluted with water to give suspensions of any desired concentration.

| A4. Coated granules | a) | b) | c) |
|---|---|---|---|
| active ingredient mixture | 0.1 % | 5% | 15% |
| highly dispersed silicic acid 0.9 % | 2 % | 2 % | |
| inorganic carrier (.AE 0.1 -1 mm) | 99.0 % | 93 % | 83 % |
| e.g. CaCO₃ or SiO₂ | | | |

The active ingredient is dissolved in methylene chloride and applied to the carrier by spraying, and the solvent is then evaporated off in vacuo.

| A5. Coated granules | a) | b) | c) |
|---|---|---|---|
| active ingredient mixture | 0.1 % | 5% | 15 % |
| polyethylene glycol MW 200 1.0 % | 2% | 3% | |
| highly dispersed silicic acid 0.9 % | 1% | 2% | |
| inorganic carrier (AE 0.1 -1 mm) | 98.0 % | 92% | 80 % |
| e.g. CaCO₃ or SiO₂ | | | |

The finely ground active ingredient is uniformly applied, in a mixer, to the carrier moistened with polyethylene glycol. Non-dusty coated granules are obtained in this manner.

| A6. Extruder granules | a) | b) | c) | d) |
|---|---|---|---|---|
| active ingredient mixture | 0.1 % | 3% | 5 % | 15 % |
| sodium lignosulfonate | 1.5 % | 2% | 3% | 4% |
| carboxymethylcellulose | 1.4 % | 2% | 2% | 2% |
| kaolin | 97.0 % | 93 % | 90 % | 79 % |

The active ingredient is mixed and ground with the adjuvants, and the mixture is moistened with water. The mixture is extruded and then dried in a stream of air.

| A7. Dusts | a) | b) | c) |
|---|---|---|---|
| active ingredient mixture | 0.1 % | 1 % | 5 % |
| talcum | 39.9 % | 49 % | 35 % |
| kaolin | 60.0 % | 50% | 60 % |

Ready-to-use dusts are obtained by mixing the active ingredient with the carriers and grinding the mixture in a suitable mill.

| A8. Suspension concentrates | a) | b) | c) | d) |
|---|---|---|---|---|
| active ingredient mixture | 3% | 10% | 25% | 50% |
| ethylene glycol | 5 % | 5% | 5 % | 5% |
| nonylphenol polyglycol ether - (15 mol of ethylene oxide) | 1 % | 2% | - | |
| sodium lignosulfonate | 3 % | 3% | 4 % | 5% |
| carboxymethylcellulose | 1 % | 1% | 1 % | 1% |
| 37 % aqueous formaldehyde solution | 0.2 % | 0.2 % | 0.2 % | 0.2% |
| silicone oil emulsion | 0.8 % | 0.8 % | 0.8 % | 0.8% |
| water | 87 % | 79 % | 62 % | 38% |

The finely ground active ingredient is intimately mixed with the adjutants, giving a suspension concentrate from which suspensions of any desired concentration can be obtained by dilution with water.

## Claims

1. 2-Fluoro-N⁶-substituted-9-(2-oxacycloalkyl)-9H-purine-6-amine derivative of the general formula **I,** 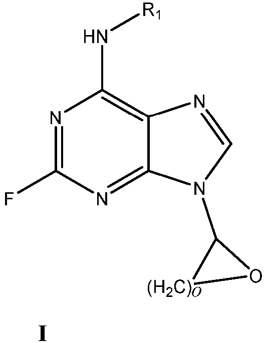 wherein
*o* is an integer in the range of from 2 to 5, wherein one hydrogen in at least one methylene group (CH₂) of the substituent on N9 is optionally replaced by methyl and/or methoxy group;
R¹ is selected from the group containing:
- furfuryl,
- furfuryl substituted with at least one methyl or methoxy group,
- benzyl,
- benzyl substituted by at least one substituent independently selected from the group comprising methyl, trifluoromethyl, hydroxy, methoxy, trifluoromethoxy, halogen, amino, methoxycarbonyl and acetoxy;
- 3-methylbut-2-en-1-yl,
- 3-methylbut-3-en-1-yl,
- 4-hydroxy-3-methylbut-2-en-1-yl, and
- 4-hydroxy-3-methylbutyl;
or agriculturally acceptable salts thereof.

2. 2-Fluoro-N⁶-substituted-9-(2-oxacycloalkyl)-9H-purine-6-amine derivative according to claim 1, wherein R¹ is selected from the group comprising furfuryl, 3-methylfurfuryl, 4-methylfurfuryl, 5-methylfurfuryl, 3-methoxyfurfuryl, 4-methoxyfurfuryl, 5-methoxyfurfuryl, benzyl, 2-methylbenzyl, 3-methylbenzyl, 4-methylbenzyl, 2-(trifluoromethyl)benzyl, 3-(trifluoromethyl)benzyl, 4-(trifluoromethyl)benzyl, 2-hydroxybenzyl, 3-hydroxybenzyl, 4-hydroxybenzylamino, 2-methoxybenzyl, 3-methoxybenzyl, 4-methoxybenzyl, 2-(trifluoromethoxy)benzyl, 3-(trifluoromethoxy)benzyl, 4-(trifluoromethoxy)benzyl, 2-fluorobenzyl, 3-fluorobenzyl, 4-fluorobenzyl, 2-chlorobenzyl, 3-chlorobenzyl, 4-chlorobenzyl, 2-bromobenzyl, 3-bromobenzyl, 4-bromobenzyl, 2-iodobenzyl, 3-iodobenzyl, 4-iodobenzyl, 2-aminobenzyl, 3-aminobenzyl, 4-aminobenzyl, 2-(methoxykarbonyl)benzyl, 3-(methoxykarbonyl)benzyl, 4-(methoxykarbonyl)benzyl, 2-acetoxybenzyl, 3-acetoxybenzyl, 4-acetoxybenzyl, 2,3-dihydroxybenzyl, 2,5-dihydroxybenzyl, 3,4-dihydroxybenzyl, 3,5-dihydroxybenzyl, 2,3-dimethoxybenzyl, 2,5-dimethoxybenzyl, 3,4-dimethoxybenzyl, 3,5-dimethoxybenzyl, 2,6-difluorobenzyl, 3,4-difluorobenzyl, 3,5-difluorobenzyl, 2,3-dichlorobenzyl, 2,4-dichlorobenzyl, 3,4-dichlorobenzyl, 3,5-dichlorobenzyl, 2-hydroxy-3-methylbenzyl, 2-hydroxy-5-methylbenzyl, 2-hydroxy-3-methoxybenzyl, 2-hydroxy-4-methoxybenzyl, 3-hydroxy-4-methoxybenzyl, 4-hydroxy-2-methoxybenzyl, 4-hydroxy-3-methoxybenzyl, 3-fluoro-4-hydroxybenzyl, 3-chloro-4-hydroxybenzyl, 4-fluoro-3-hydroxybenzyl, 4-chloro-3-hydroxybenzyl, 2-chloro-4-fluorbenzyl, 2-chloro-6-fluorbenzyl, 3,4,5-trihydroxybenzyl, 3,4,5-trimethoxybenzyl, 2,3,4-trifluorobenzyl, 2,3,6-trifluorobenzyl, 3,4,5-trifluorobenzyl, 4-hydroxy-3,5-dimethoxybenzyl, 3-methylbut-2-en-1-yl, 3-methylbut-3-en-1-yl, 4-hydroxy-3-methylbut-2-en-1-yl, 4-hydroxy-3-methylbutyl)amino.

3. 2-Fluoro-N⁶-substituted-9-(2-oxacycloalkyl)-9H-purine-6-amine derivative according to any one of the preceding claims, wherein the substituent on N9 is selected from tetrahydro-2H-pyran-2-yl and tetrahydrofuran-2-yl, each of which may optionally be substituted by at least one methyl or methoxy group.

4. 2-fluoro-N6-substituted-9-(2-oxacycloalkyl)-9H-purine-6-amine derivative according to claim 1, selected from the group comprising 2-fluoro-6-furfurylamino-9-(oxetan-2-yl)-9*H*-purine, 2-fluoro-6-(3-methoxyfurfurylamino)-9-(oxetan-2-yl)-9*H*-purine, 2-fluoro-6-benzylamino-9-(oxetan-2-yl)-9*H*-purine; 2-fluoro-6-[(2-hydroxybenzyl)amino]-9-(oxetan-2-yl)-9*H*-purine, 2-fluoro-6-[(3-hydroxybenzyl)amino]-9-(oxetan-2-yl)-9*H*-purine, 2-fluoro-6-[(4-hydroxybenzyl)amino]-9-(oxetan-2-yl)-9*H*-purine, 2-fluoro-6-[(2-methoxybenzyl)amino]-9-(oxetan-2-yl)-9*H*-purine, 2-fluoro-6-[(3-methoxybenzyl)amino]-9-(oxetan-2-yl)-9*H*-purine, 2-fluoro-6-[(4-methoxybenzyl)amino]-9-(oxetan-2-yl)-9*H*-purine, 2-fluoro-6-[(2-fluorobenzyl)amino]-9-(oxetan-2-yl)-9*H*-purine; 2-fluoro-6-[(3-fluorobenzyl)amino]-9-(oxetan-2-yl)-9*H-*purine, 2-fluoro-6-[(4-fluorobenzyl)amino]-9-(oxetan-2-yl)-9*H*-purine, 2-fluoro-6-[(2-chlorobenzyl)amino]-9-(oxetan-2-yl)-9*H*-purine, 2-fluoro-6-[(3-chlorobenzyl)amino]-9-(oxetan-2-yl)-9*H-*purine, 2-fluoro-6-(3-methoxyanilino)-9-(oxetan-2-yl)-9H-purine, 2-fluoro-6-[(3-methylbut-2-en-1-yl)amino]-9-(oxetan-2-yl)-9H-purine, 2-fluoro-6-(E)-[(4-hydroxy-3-methylbut-2-en-1-yl)amino]-9-(oxetan-2-yl)-9H-purine, 2-fluoro-6-(Z)-[(4-hydroxy-3-methylbut-2-en-1-yl)amino]-9-(oxetan-2-yl)-9H-purine, 2-fluoro-6-[(4-hydroxy-3-methylbutyl)amino]-9-(oxetan-2-yl)-9H-purine, 2-fluoro-6-(3-methoxyfurfurylamino)-9-(tetrahydrofuran-2-yl)-9H-purine, 2-fluoro-6-benzylamino-9-(tetrahydrofuran-2-yl)-9H-purine; 2-fluoro-6-[(2-hydroxybenzyl)amino]-9-(tetrahydrofuran-2-yl)-9H-purine, 2-fluoro-6-[(3-hydroxybenzyl)amino]-9-(tetrahydrofuran-2-yl)-9H-purine, 2-fluoro-6-[(4-hydroxybenzyl)amino]-9-(tetrahydrofuran-2-yl)-9H-purine; 2-fluoro-6-[(2-methoxybenzyl)amino]-9-(tetrahydrofuran-2-yl)-9H-purine, 2-fluoro-6-[(3-methoxybenzyl)amino]-9-(tetrahydrofuran-2-yl)-9H-purine, 2-fluoro-6-[(4-methoxybenzyl)amino]-9-(tetrahydrofuran-2-yl)-9H-purine, 2-fluoro-6-[(2-fluorobenzyl)amino]-9-(tetrahydrofuran-2-yl)-9H-purine, 2-fluoro-6-[(3-fluorobenzyl)amino]-9-(tetrahydrofuran-2-yl)-9H-purine, 2-fluoro-6-[(4-fluorobenzyl)amino]-9-(tetrahydrofuran-2-yl)-9H-purine; 2-fluoro-6-[(2-chlorobenzyl)amino]-9-(tetrahydrofuran-2-yl)-9H-purine, 2-fluoro-6-[(3-chlorobenzyl)amino]-9-(tetrahydrofuran-2-yl)-9H-purine, 2-fluoro-6-(3-methoxyanilino)-9-(tetrahydrofuran-2-yl)-9H-purine, 2-fluoro-6-[(3-methylbut-2-en-1-yl)amino]-9-(tetrahydrofuran-2-yl)-9H-purine, 2-fluoro-6-(E)-[(4-hydroxy-3-methylbut-2-en-1-yl)amino]-9-(tetrahydrofuran-2-yl)-9H-purine, 2-fluoro-6-(Z)-[(4-hydroxy-3-methylbut-2-en-1-yl)amino]-9-(tetrahydrofuran-2-yl)-9H-purine, 2-fluoro-6-[(4-hydroxy-3-methylbutyl)amino]-9-(tetrahydrofuran-2-yl)-9H-purine, 2-fluoro-6-(3-methoxyfurfurylamino)-9-(tetrahydro-2H-pyran-2-yl)-9H-purine; 2-fluoro-6-benzylamino-9-(tetrahydro-2H-pyran-2-yl)-9H-purine, 2-fluoro-6-[(2-hydroxybenzyl)amino]-9-(tetrahydro-2H-pyran-2-yl)-9H-purine, 2-fluoro-6-[(3-hydroxybenzyl)amino]-9-(tetrahydro-2H-pyran-2-yl)-9H-purine, 2-fluoro-6-[(4-hydroxybenzyl)amino]-9-(tetrahydro-2H-pyran-2-yl)-9H-purine, 2-fluoro-6-[(2-methoxybenzyl)amino]-9-(tetrahydro-2H-pyran-2-yl)-9H-purine, 2-fluoro-6-[(3-methoxybenzyl)amino]-9-(tetrahydro-2H-pyran-2-yl)-9H-purine, 2-fluoro-6-[(4-methoxybenzyl)amino]-9-(tetrahydro-2H-pyran-2-yl)-9H-purine, 2-fluoro-6-[(2-fluorobenzyl)amino]-9-(tetrahydro-2H-pyran-2-yl)-9H-purine, 2-fluoro-6-[(3-fluorobenzyl)amino]-9-(tetrahydro-2H-pyran-2-yl)-9H-purine, 2-fluoro-6-[(4-fluorobenzyl)amino]-9-(tetrahydro-2H-pyran-2-yl)-9H-purine, 2-fluoro-6-[(2-chlorobenzyl)amino]-9-(tetrahydro-2H-pyran-2-yl)-9H-purine, 2-fluoro-6-[(3-chlorobenzyl)amino]-9-(tetrahydro-2H-pyran-2-yl)-9H-purine, 2-fluoro-6-(3-methoxyanilino)-9-(tetrahydro-2H-pyran-2-yl)-9H-purine, 2-fluoro-6-[(3-methylbut-2-en-1-yl)amino]-9-(tetrahydro-2H-pyran-2-yl)-9H-purine, 2-fluoro-6-(E)-[(4-hydroxy-3-methylbut-2-en-1-yl)amino]-9-(tetrahydro-2H-pyran-2-yl)-9H-purine, 2-fluoro-6-(Z)-[(4-hydroxy-3-methylbut-2-en-1-yl)amino]-9-(tetrahydro-2H-pyran-2-yl)-9H-purine, 2-fluoro-6-[(4-hydroxy-3-methylbutyl)amino]-9-(tetrahydro-2H-pyran-2-yl)-9H-purine, 2-fluoro-6-(3-methoxyfurfurylamino)-9-(oxepan-2-yl)-9H-purine, 2-fluoro-6-benzylamino-9-(oxepan-2-yl)-9*H-*purine; 2-fluoro-6-[(2-hydroxybenzyl)amino]-9-(oxepan-2-yl)-9H-purine, 2-fluoro-6-[(3-hydroxybenzyl)amino]-9-(oxepan-2-yl)-9H-purine, 2-fluoro-6-[(4-hydroxybenzyl)amino]-9-(oxepan-2-yl)-9H-purine; 2-fluoro-6-[(2-methoxybenzyl)amino]-9-(oxepan-2-yl)-9H-purine; 2-fluoro-6-[(3-methoxybenzyl)amino]-9-(oxepan-2-yl)-9H-purine, 2-fluoro-6-[(4-methoxybenzyl)amino]-9-(oxepan-2-yl)-9H-purine, 2-fluoro-6-[(2-fluorobenzyl)amino]-9-(oxepan-2-yl)-9H-purine; 2-fluoro-6-[(3-fluorobenzyl)amino]-9-(oxepan-2-yl)-9H-purine, 2-fluoro-6-[(4-fluorobenzyl)amino]-9-(oxepan-2-yl)-9H-purine, 2-fluoro-6-[(2-chlorobenzyl)amino]-9-(oxepan-2-yl)-9H-purine, 2-fluoro-6-[(3-chlorobenzyl)amino]-9-(oxepan-2-yl)-9H-purine; 2-fluoro-6-(3-methoxyanilino)-9-(oxepan-2-yl)-9H-purine, 2-fluoro-6-[(3-methylbut-2-en-1-yl)amino]-9-(oxepan-2-yl)-9H-purine, 2-fluoro-6-(E)-[(4-hydroxy-3-methylbut-2-en-1-yl)amino]-9-(oxepan-2-yl)-9H-purine, 2-fluoro-6-(Z)-[(4-hydroxy-3-methylbut-2-en-1-yl)amino]-9-(oxepan-2-yl)-9H-purine, 2-fluoro-6-[(4-hydroxy-3-methylbutyl)amino]-9-(oxepan-2-yl)-9H-purine and agriculturally acceptable salts thereof.

5. Use of 2-fluoro-N6-substituted-9-(2-oxacycloalkyl)-9H-purine-6-amine derivative according to any one of claims 1 to 4 as cell division stimulatory, pro-differentiating, antisenescent, phloem loading, shoot forming, and root growth stimulatory agent in plants, in particular in crop plants.

6. Use of 2-fluoro-N6-substituted-9-(2-oxacycloalkyl)-9H-purine-6-amine derivative according to any one of claims 1 to 4 in agriculture and biotechnology, for inhibition, delaying, or reducing the adverse effects of senescence, improving the overall appearance and condition of the plants, in paticular plant epidermal and mesophyll cells, and for inhibition, delaying or reducing yellowing, chloroplast and/or chlorophyll losses and leaf losses.

7. Use of 2-fluoro-N6-substituted-9-(2-oxacycloalkyl)-9H-purine-6-amine derivative according to any one of claims 1 to 4 as growth regulators in agriculture, to increase crop production, to dispatch grain filling, to increase grain and fruit size of plants and fungi, to shorten plant seed germination period, to increase the yield and quality of agricultural products, to enhance root and shoot growth, and/or to increase stress resistance to environmental factors.

8. Use of 2-fluoro-N6-substituted-9-(2-oxacycloalkyl)-9H-purine-6-amine derivative according to any one of claims 1 to 4 in plant tissue cultures as growth regulators, for stimulation of proliferation and morphogenesis and/or as root formation stimulators.

9. Use of 2-fluoro-N6-substituted-9-(2-oxacycloalkyl)-9H-purine-6-amine derivative according to any one of claims 1 to 4 in plant tissue cultures, for modulation of micropropagation, meristematic culture, somatic embryogenesis, androgenesis, gynogenesis, suspension and protoplast cultures.

10. Composition, **characterized in that** it comprises one or more 2-fluoro-N⁶-substituted-9-(2-oxacycloalkyl)-9H-purine-6-amine derivatives according to any one of claims 1 to 4, and and at least one agriculturally acceptable auxiliary substance.

## Patentansprüche

1. 2-Fluor-N6-substituiertes 9-(2-Oxacycloalkyl)-9H-purin-6-amin-derivat der allgemeinen Formel I, 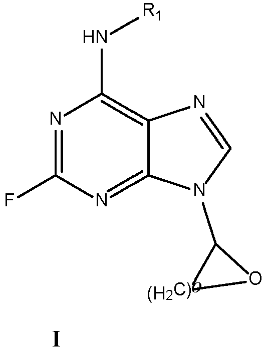 wobei
o eine ganze Zahl im Bereich von 2 bis 5 ist, wobei ein Wasserstoffatom in mindestens einer Methylengruppe (CH₂) des Substituenten an N9 optional durch eine Methyl- und/oder Methoxygruppe ersetzt sein kann;
R¹ ausgewählt ist aus der Gruppe, die enthält:
- Furfuryl,
- Furfuryl substituiert mit mindestens einer Methyl- oder Methoxygruppe,
- Benzyl,
- Benzyl substituiert mit mindestens einem Substituenten, unabhängig ausgewählt aus der Gruppe, die besteht aus Methyl, Trifluormethyl, Hydroxy, Methoxy, Trifluormethoxy, Halogen, Amino, Methoxycarbonyl und Acetoxy;
- 3-Methylbut-2-en-1-yl,
- 3-Methylbut-3-en-1-yl,
- 4-Hydroxy-3-methylbut-2-en-1-yl, und
- 4-Hydroxy-3-methylbutyl;
oder landwirtschaftlich verträgliche Salze davon.

2. 2-Fluor-N6-substituiertes 9-(2-Oxacycloalkyl)-9H-purin-6-amin-derivat nach dem Anspruch 1, worin R¹ ausgewählt ist aus der Gruppe enthaltend Furfuryl, 3-Methylfurfuryl, 4-Methylfurfuryl, 5-Methylfurfuryl, 3-Methoxyfurfuryl, 4-Methoxyfurfuryl, 5-Methoxyfurfuryl, Benzyl, 2-Methylbenzyl, 3-Methylbenzyl, 4-Methylbenzyl, 2-(Trifluormethyl)benzyl, 3-(Trifluormethyl)benzyl, 4-(Trifluormethyl)benzyl, 2-Hydroxybenzyl, 3-Hydroxybenzyl, 4-Hydroxybenzylamino, 2-Methoxybenzyl, 3-Methoxybenzyl, 4-Methoxybenzyl, 2-(Trifluormethoxy)benzyl, 3-(Trifluormethoxy)benzyl, 4-(Trifluormethoxy)benzyl, 2-Fluorbenzyl, 3-Fluorbenzyl, 4-Fluorbenzyl, 2-Chlorbenzyl, 3-Chlorbenzyl, 4-Chlorbenzyl, 2-Bromobenzyl, 3-Bromobenzyl, 4-Bromobenzyl, 2-Jodbenzyl, 3-Jodbenzyl, 4-Jodbenzyl, 2-Aminobenzyl, 3-Aminobenzyl, 4-Aminobenzyl, 2-(Methoxykarbonyl)benzyl, 3-(Methoxykarbonyl)benzyl, 4-(Methoxykarbonyl)benzyl, 2-Acetoxybenzyl, 3-Acetoxybenzyl, 4-Acetoxybenzyl, 2,3-Dihydroxybenzyl, 2,5-Dihydroxybenzyl, 3,4-Dihydroxybenzyl, 3,5-Dihydroxybenzyl, 2,3-Dimethoxybenzyl, 2,5-Dimethoxybenzyl, 3,4-Dimethoxybenzyl, 3,5-Dimethoxybenzyl, 2,6-Difluorbenzyl, 3,4-Difluorbenzyl, 3,5-Difluorbenzyl, 2,3-Dichlorbenzyl, 2,4-Dichlorbenzyl, 3,4-Dichlorbenzyl, 3,5-Dichlorbenzyl, 2-Hydroxy-3-methylbenzyl, 2-Hydroxy-5-methylbenzyl, 2-Hydroxy-3-methoxybenzyl, 2-Hdroxy-4-methoxybenzyl, 3-Hydroxy-4-methoxybenzyl, 4-Hydroxy-2-methoxybenzyl, 4-Hydroxy-3-methoxybenzyl, 3-Fluor-4-hydroxybenzyl, 3-Chlor-4-hydroxybenzyl, 4-Fluor-3-hydroxybenzyl, 4-Chlor-3-hydroxybenzyl, 2-Chlor-4-fluorbenzyl, 2-Chlor-6-fluorbenzyl, 3,4,5-Trihydroxybenzyl, 3,4,5-Trimethoxybenzyl, 2,3,4-Trifluorbenzyl, 2,3,6-Trifluorbenzyl, 3,4,5-Trifluorbenzyl, 4-Hydroxy-3,5-dimethoxybenzyl, 3-Methylbut-2-en-1-yl, 3-Methylbut-3-en-1-yl, 4-Hydroxy-3-methylbut-2-en-1-yl, 4-Hydroxy-3-methylbutyl)amino.

3. 2-Fluor-N6-substituiertes 9-(2-Oxacycloalkyl)-9H-purin-6-amin-derivat nach einem der vorhergehenden Ansprüche, wobei der Substituent an N9 ausgewählt ist aus Tetrahydro-2H-pyran-2-yl und Tetrahydrofuran-2-yl, die jeweils optional mit mindestens einer Methyl- oder Methoxygruppe substituiert sein können.

4. 2-Fluor-N6-substituiertes 9-(2-Oxacycloalkyl)-9H-purin-6-amin-derivat nach Anspruch 1, ausgewählt aus der Gruppe enthaltend 2-Fluor-6-furfurylamino-9-(oxetan-2-yl)-9*H*-purin, 2-Fluor-6-(3-methoxyfurfurylamino)-9-(oxetan-2-yl)-9*H*-purin, 2-Fluor-6-benzylamino-9-(oxetan-2-yl)-9*H-*purin; 2-Fluor-6-[(2-hydroxybenzyl)amino]-9-(oxetan-2-yl)-9*H*-purin, 2-Fluor-6-[(3-hydroxybenzyl)amino]-9-(oxetan-2-yl)-9*H*-purin, 2-Fluor-6-[(4-hydroxybenzyl)amino]-9-(oxetan-2-yl)-9*H*-purin, 2-Fluor-6-[(2-methoxybenzyl)amino]-9-(oxetan-2-yl)-9*H*-purin, 2-Fluor-6-[(3-methoxybenzyl)amino]-9-(oxetan-2-yl)-9*H*-purin, 2-Fluor-6-[(4-methoxybenzyl)amino]-9-(oxetan-2-yl)-9*H*-purin, 2-Fluor-6-[(2-fluorbenzyl)amino]-9-(oxetan-2-yl)-9*H*-purin; 2-Fluor-6-[(3-fluorbenzyl)amino]-9-(oxetan-2-yl)-9*H*-purin, 2-Fluor-6-[(4-fluorbenzyl)amino]-9-(oxetan-2-yl)-9*H-*purin, 2-Fluor-6-[(2-chlorbenzyl)amino]-9-(oxetan-2-yl)-9*H*-purin, 2-Fluor-6-[(3-chlorbenzyl)amino]-9-(oxetan-2-yl)-9*H*-purin, 2-Fluor-6-(3-methoxyanilino)-9-(oxetan-2-yl)-9H-purin, 2-Fluor-6-[(3-methylbut-2-en-1-yl)amino]-9-(oxetan-2-yl)-9H-purin, 2-Fluor-6-(E)-[(4-hydroxy-3-methylbut-2-en-1-yl)amino]-9-(oxetan-2-yl)-9H-purin, 2-Fluor-6-(Z)-[(4-hydroxy-3-methylbut-2-en-1-yl)amino]-9-(oxetan-2-yl)-9H-purin, 2-Fluor-6-[(4-hydroxy-3-methylbutyl)amino]-9-(oxetan-2-yl)-9H-purin, 2-Fluor-6-(3-methoxyfurfurylamino)-9-(tetrahydrofuran-2-yl)-9H-purin, 2-Fluor-6-benzylamino-9-(tetrahydrofuran-2-yl)-9H-purin; 2-Fluor-6-[(2-hydroxybenzyl)amino]-9-(tetrahydrofuran-2-yl)-9H-purin, 2-Fluor-6-[(3-hydroxybenzyl)amino]-9-(tetrahydrofuran-2-yl)-9H-purin, 2-Fluor-6-[(4-hydroxybenzyl)amino]-9-(tetrahydrofuran-2-yl)-9H-purin; 2-Fluor-6-[(2-methoxybenzyl)amino]-9-(tetrahydrofuran-2-yl)-9H-purin, 2-Fluor-6-[(3-methoxybenzyl)amino]-9-(tetrahydrofuran-2-yl)-9H-purin, 2-Fluor-6-[(4-methoxybenzyl)amino]-9-(tetrahydrofuran-2-yl)-9H-purin, 2-Fluor-6-[(2-fluorbenzyl)amino]-9-(tetrahydrofuran-2-yl)-9H-purin, 2-Fluor-6-[(3-fluorbenzyl)amino]-9-(tetrahydrofuran-2-yl)-9H-purin, 2-Fluor-6-[(4-fluorbenzyl)amino]-9-(tetrahydrofuran-2-yl)-9H-purin; 2-Fluor-6-[(2-chlorbenzyl)amino]-9-(tetrahydrofuran-2-yl)-9H-purin, 2-Fluor-6-[(3-chlorbenzyl)amino]-9-(tetrahydrofuran-2-yl)-9H-purin, 2-Fluor-6-(3-methoxyanilino)-9-(tetrahydrofuran-2-yl)-9H-purin, 2-Fluor-6-[(3-methylbut-2-en-1-yl)amino]-9-(tetrahydrofuran-2-yl)-9H-purin, 2-Fluor-6-(E)-[(4-hydroxy-3-methylbut-2-en-1-yl)amino]-9-(tetrahydrofuran-2-yl)-9H-purin, 2-Fluor-6-(Z)-[(4-hydroxy-3-methylbut-2-en-1-yl)amino]-9-(tetrahydrofuran-2-yl)-9H-purin, 2-Fluor-6-[(4-hydroxy-3-methylbutyl)amino]-9-(tetrahydrofuran-2-yl)-9H-purin, 2-Fluor-6-(3-methoxyfurfurylamino)-9-(tetrahydro-2H-pyran-2-yl)-9H-purin; 2-Fluor-6-benzylamino-9-(tetrahydro-2H-pyran-2-yl)-9H-purin, 2-Fluor-6-[(2-hydroxybenzyl)amino]-9-(tetrahydro-2H-pyran-2-yl)-9H-purin, 2-Fluor-6-[(3-hydroxybenzyl)amino]-9-(tetrahydro-2H-pyran-2-yl)-9H-purin, 2-Fluor-6-[(4-hydroxybenzyl)amino]-9-(tetrahydro-2H-pyran-2-yl)-9H-purin, 2-Fluor-6-[(2-methoxybenzyl)amino]-9-(tetrahydro-2H-pyran-2-yl)-9H-purin, 2-Fluor-6-[(3-methoxybenzyl)amino]-9-(tetrahydro-2H-pyran-2-yl)-9H-purin, 2-Fluor-6-[(4-methoxybenzyl)amino]-9-(tetrahydro-2H-pyran-2-yl)-9H-purin, 2-Fluor-6-[(2-fluorbenzyl)amino]-9-(tetrahydro-2H-pyran-2-yl)-9H-purin, 2-Fluor-6-[(3-fluorbenzyl)amino]-9-(tetrahydro-2H-pyran-2-yl)-9H-purin, 2-Fluor-6-[(4-fluorbenzyl)amino]-9-(tetrahydro-2H-pyran-2-yl)-9H-purin, 2-Fluor-6-[(2-chlorbenzyl)amino]-9-(tetrahydro-2H-pyran-2-yl)-9H-purin, 2-fluor-6-[(3-chlorbenzyl)amino]-9-(tetrahydro-2H-pyran-2-yl)-9H-purin, 2-Fluor-6-(3-methoxyanilino)-9-(tetrahydro-2H-pyran-2-yl)-9H-purin, 2-Fluor-6-[(3-methylbut-2-en-1-yl)amino]-9-(tetrahydro-2H-pyran-2-yl)-9H-purin, 2-Fluor-6-(E)-[(4-hydroxy-3-methylbut-2-en-1-yl)amino]-9-(tetrahydro-2H-pyran-2-yl)-9H-purin, 2-Fluor-6-(Z)-[(4-hydroxy-3-methylbut-2-en-1-yl)amino]-9-(tetrahydro-2H-pyran-2-yl)-9H-purin, 2-Fluor-6-[(4-hydroxy-3-methylbutyl)amino]-9-(tetrahydro-2H-pyran-2-yl)-9H-purin, 2-Fluor-6-(3-methoxyfurfurylamino)-9-(oxepan-2-yl)-9H-purin, 2-Fluor-6-benzylamino-9-(oxepan-2-yl)-9H-purin; 2-Fluor-6-[(2-hydroxybenzyl)amino]-9-(oxepan-2-yl)-9H-purin, 2-Fluor-6-[(3-hydroxybenzyl)amino]-9-(oxepan-2-yl)-9H-purin, 2-Fluor-6-[(4-hydroxybenzyl)amino]-9-(oxepan-2-yl)-9H-purin; 2-Fluor-6-[(2-methoxybenzyl)amino]-9-(oxepan-2-yl)-9H-purin; 2-Fluor-6-[(3-methoxybenzyl)amino]-9-(oxepan-2-yl)-9H-purin, 2-Fluor-6-[(4-methoxybenzyl)amino]-9-(oxepan-2-yl)-9H-purin, 2-Fluor-6-[(2-fluorbenzyl)amino]-9-(oxepan-2-yl)-9H-purin; 2-Fluor-6-[(3-fluorbenzyl)amino]-9-(oxepan-2-yl)-9H-purin, 2-Fluor-6-[(4-fluorbenzyl)amino]-9-(oxepan-2-yl)-9H-purin, 2-Fluor-6-[(2-chlorbenzyl)amino]-9-(oxepan-2-yl)-9H-purin, 2-Fluor-6-[(3-chlorbenzyl)amino]-9-(oxepan-2-yl)-9H-purin; 2-Fluor-6-(3-methoxyanilino)-9-(oxepan-2-yl)-9H-purin, 2-Fluor-6-[(3-methylbut-2-en-1-yl)amino]-9-(oxepan-2-yl)-9H-purin, 2-Fluor-6-(E)-[(4-hydroxy-3-methylbut-2-en-1-yl)amino]-9-(oxepan-2-yl)-9H-purin, 2-Fluor-6-(Z)-[(4-hydroxy-3-methylbut-2-en-1-yl)amino]-9-(oxepan-2-yl)-9H-purin, 2-Fluor-6-[(4-hydroxy-3-methylbutyl)amino]-9-(oxepan-2-yl)-9H-purin und oder landwirtschaftlich verträgliche Salze davon.

5. Verwendung des 2-Fluor-N6-substituierten 9-(2-Oxacycloalkyl)-9H-purin-6-amin-derivats nach einem der Ansprüche 1 bis 4 als zellteilungsstimulierendes, differenzierungsförderndes, antiseneszentes, phloembildendes, sprossbildendes und wurzelwachstumsstimulierendes Mittel in Pflanzen, insbesondere in Kulturpflanzen.

6. Verwendung des 2-Fluor-N6-substituierten 9-(2-Oxacycloalkyl)-9H-purin-6-amin-derivats nach einem der Ansprüche 1 bis 4 in der Landwirtschaft und Biotechnologie zur Hemmung, Verzögerung oder Verringerung der negativen Auswirkungen der Seneszenz, zur Verbesserung des Gesamterscheinungsbildes und -zustands der Pflanzen, insbesondere in Epidermis- und Mesophyllzellen der Pflanzen, sowie zur Hemmung, Verzögerung oder Verringerung der Vergilbung, des Chlorplast- und/oder Chlorophyllverlusts und des Blattverlusts.

7. Verwendung von 2-Fluor-N6-substituiertem 9-(2-Oxacycloalkyl)-9H-purin-6-amin-derivaten nach einem der Ansprüche 1 bis 4 als Wachstumsregulatoren in der Landwirtschaft, zur Steigerung der Ernteerträge, zur Förderung der Kornfüllung, zur Erhöhung der Korn- und Fruchtgröße von Pflanzen und Pilzen, zur Verkürzung der Keimzeit von Pflanzensamen, zur Steigerung von Ertrag und Qualität landwirtschaftlicher Produkte, zur Förderung des Wurzel- und Sprosswachstums und/oder zur Erhöhung der Stressresistenz gegenüber Umweltfaktoren.

8. Verwendung von 2-Fluor-N6-substituiertem 9-(2-Oxacycloalkyl)-9H-purin-6-amin-derivaten nach einem der Ansprüche 1 bis 4 in Pflanzengewebekulturen als Wachstumsregulatoren, zur Stimulierung der Proliferation und Morphogenese und/oder als Wurzelbildungsstimulatoren.

9. Verwendung des 2-Fluor-N6-substituierten 9-(2-Oxacycloalkyl)-9H-purin-6-amin-derivats nach einem der Ansprüche 1 bis 4 in Pflanzengewebekulturen zur Modulation der Mikropropagation, Meristemkultur, somatischen Embryogenese, Androgenese, Gynogenese, Suspensions- und Protoplastenkulturen.

10. Zusammensetzung, **dadurch gekennzeichnet, dass** sie ein oder mehrere 2-Fluor-N6-substituierte 9-(2-Oxacycloalkyl)-9H-purin-6-amin-derivate nach einem der Ansprüche 1 bis 4 und mindestens einen landwirtschaftlich verträglichen Hilfsstoff enthält.

## Revendications

1. Dérivé de 2-fluoro-N6-substitué-9-(2-oxacycloalkyl)-9H-purine-6-amine de formule générale I, 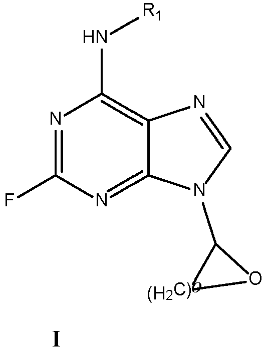 où
*o* est un nombre entier compris entre 2 et 5, où un hydrogène dans au moins un groupe méthylène (CH₂) du substituant sur N9 est éventuellement remplacé par un groupe méthyle et/ou méthoxy;
R' est choisi dans le groupe comprenant:
- furfuryle,
- furfuryle substitué par au moins un groupe méthyle ou méthoxy,
- benzyle,
- benzyle substitué par au moins un substituant choisi indépendamment dans le groupe comprenant méthyle, trifluorométhyle, hydroxy, méthoxy, trifluorométhoxy, halogène, amino, méthoxycarbonyle et acétoxy;
- 3-méthylbut-2-én-1-yle,
- 3-méthylbut-3-én-1-yle,
- 4-hydroxy-3-méthylbut-2-én-1-yle, et
- 4-hydroxy-3-méthylbutyle;
ou leurs sels acceptables en agriculture.

2. Dérivé de 2-fluoro-N6-substitué-9-(2-oxacycloalkyl)-9H-purine-6-amine selon la revendication 1, où R' est choisi dans le group comprenant furfuryle, 3-méthylfurfuryle, 4-méthylfurfuryle, 5-méthylfurfuryle, 3-méthoxyfurfuryle, 4-méthoxyfurfuryle, 5-méthoxyfurfuryle, benzyle, 2-méthylbenzyle, 3-méthylbenzyle, 4-méthylbenzyle, 2-(trifluorométhyl)benzyle, 3-(trifluorométhyl)benzyle, 4-(trifluorométhyl)benzyle, 2-hydroxybenzyle, 3-hydroxybenzyle, 4-hydroxybenzylamino, 2-méthoxybenzyle, 3-méthoxybenzyle, 4-méthoxybenzyle, 2-(trifluorométhoxy)benzyle, 3-(trifluorométhoxy)benzyle, 4-(trifluorométhoxy)benzyle, 2-fluorobenzyle, 3-fluorobenzyle, 4-fluorobenzyle, 2-chlorobenzyle, 3-chlorobenzyle, 4-chlorobenzyle, 2-bromobenzyle, 3-bromobenzyle, 4-bromobenzyle, 2-iodobenzyle, 3-iodobenzyle, 4-iodobenzyle, 2-aminobenzyle, 3-aminobenzyle, 4-aminobenzyle, 2-(méthoxycarbonyl)benzyle, 3-(méthoxycarbonyl)benzyle, 4-(méthoxycarbonyl)benzyle, 2-acétoxybenzyle, 3-acétoxybenzyle, 4-acétoxybenzyle, 2,3-dihydroxybenzyle, 2,5-dihydroxybenzyle, 3,4-dihydroxybenzyle, 3,5-dihydroxybenzyle, 2,3-diméthoxybenzyle, 2,5-diméthoxybenzyle, 3,4-diméthoxybenzyle, 3,5-diméthoxybenzyle, 2,6-difluorobenzyle, 3,4-difluorobenzyle, 3,5-difluorobenzyle, 2,3-dichlorobenzyle, 2,4-dichlorobenzyle, 3,4-dichlorobenzyle, 3,5-dichlorobenzyle, 2-hydroxy-3-méthylbenzyle, 2-hydroxy-5-méthylbenzyle, 2-hydroxy-3-méthoxybenzyle, 2-hydroxy-4-méthoxybenzyle, 3-hydroxy-4-méthoxybenzyle, 4-hydroxy-2-méthoxybenzyle, 4-hydroxy-3-méthoxybenzyle, 3-fluoro-4-hydroxybenzyle, 3-chloro-4-hydroxybenzyle, 4-fluoro-3-hydroxybenzyle, 4-chloro-3-hydroxybenzyle, 2-chloro-4-fluorbenzyle, 2-chloro-6-fluorbenzyle, 3,4,5-trihydroxybenzyle, 3,4,5-triméthoxybenzyle, 2,3,4-trifluorobenzyle, 2,3,6-trifluorobenzyle, 3,4,5-trifluorobenzyle, 4-hydroxy-3,5-diméthoxybenzyle, 3-méthylbut-2-én-1-yle, 3-méthylbut-3-én-1-yle, 4-hydroxy-3-méthylbut-2-én-1-yle, 4-hydroxy-3-méthylbutyle.

3. Dérivé de 2-fluoro-N6-substitué-9-(2-oxacycloalkyl)-9H-purine-6-amine selon l'une quelconque des revendications précédentes, où le substituant sur N9 est choisi parmi le tétrahydro-2H-pyran-2-yle et le tétrahydrofuran-2-yle, chacun pouvant être éventuellement substitué par au moins un groupe méthyle ou méthoxy.

4. Dérivé de 2-fluoro-N6-substitué-9-(2-oxacycloalkyl)-9H-purine-6-amine selon la revendication 1, choisi dans le groupe comprenant 2-fluoro-6-furfurylamino-9-(oxétan-2-yl)-9*H*-purine, 2-fluoro-6-(3-méthoxyfurfurylamino)-9-(oxétan-2-yl)-9*H*-purine, 2-fluoro-6-benzylamino-9-(oxétan-2-yl)-9*H-*purine; 2-fluoro-6-[(2-hydroxybenzyl)amino]-9-(oxétan-2-yl)-9*H*-purine, 2-fluoro-6-[(3-hydroxybenzyl)amino]-9-(oxétan-2-yl)-9*H*-purine, 2-fluoro-6-[(4-hydroxybenzyl)amino]-9-(oxétan-2-yl)-9H-purine, 2-fluoro-6-[(2-méthoxybenzyl)amino]-9-(oxétan-2-yl)-9*H*-purine, 2-fluoro-6-[(3-méthoxybenzyl)amino]-9-(oxétan-2-yl)-9*H*-purine, 2-fluoro-6-[(4-méthoxybenzyl)amino]-9-(oxétan-2-yl)-9H-purine, 2-fluoro-6-[(2-fluorobenzyl)amino]-9-(oxétan-2-y1)-9*H*-purine; 2-fluoro-6-[(3-fluorobenzyl)amino]-9-(oxétan-2-yl)-9*H*-purine, 2-fluoro-6-[(4-fluorobenzyl)amino]-9-(oxétan-2-yl)-9H-purine, 2-fluoro-6-[(2-chlorobenzyl)amino]-9-(oxétan-2-yl)-9*H*-purine, 2-fluoro-6-[(3-chlorobenzyl)amino]-9-(oxétan-2-yl)-9*H*-purine, 2-fluoro-6-(3-méthoxyanilino)-9-(oxétan-2-yl)-9H-purine, 2-fluoro-6-[(3-méthylbut-2-én-1-yl)amino]-9-(oxétan-2-yl)-9H-purine, 2-fluoro-6-(E)-[(4-hydroxy-3-méthylbut-2-én-1-yl)amino]-9-(oxétan-2-yl)-9H-purine, 2-fluoro-6-(Z)-[(4-hydroxy-3-méthylbut-2-én-1-yl)amino]-9-(oxétan-2-yl)-9H-purine, 2-fluoro-6-[(4-hydroxy-3-méthylbutyl)amino]-9-(oxétan-2-yl)-9H-purine, 2-fluoro-6-(3-méthoxyfurfurylamino)-9-(tétrahydrofuran-2-yl)-9H-purine, 2-fluoro-6-benzylamino-9-(tétrahydrofuran-2-yl)-9H-purine; 2-fluoro-6-[(2-hydroxybenzyl)amino]-9-(tétrahydrofuran-2-yl)-9H-purine, 2-fluoro-6-[(3-hydroxybenzyl)amino]-9-(tétrahydrofuran-2-yl)-9H-purine, 2-fluoro-6-[(4-hydroxybenzyl)amino]-9-(tétrahydrofuran-2-yl)-9H-purine, 2-fluoro-6-[(2-méthoxybenzyl)amino]-9-(tétrahydrofuran-2-yl)-9H-purine, 2-fluoro-6-[(3-méthoxybenzyl)amino]-9-(tétrahydrofuran-2-yl)-9H-purine, 2-fluoro-6-[(4-méthoxybenzyl)amino]-9-(tétrahydrofuran-2-yl)-9H-purine, 2-fluoro-6-[(2-fluorobenzyl)amino]-9-(tétrahydrofuran-2-yl)-9H-purine, | 2-fluoro-6-[(3-fluorobenzyl)amino]-9-(tétrahydrofuran-2-yl)-9H-purine, 2-fluoro-6-[(4-fluorobenzyl)amino]-9-(tétrahydrofuran-2-yl)-9H-purine; 2-fluoro-6-[(2-chlorobenzyl)amino]-9-(tétrahydrofuran-2-yl)-9H-purine, 2-fluoro-6-[(3-chlorobenzyl)amino]-9-(tétrahydrofuran-2-yl)-9H-purine, 2-fluoro-6-(3-méthoxyanilino)-9-(tétrahydrofuran-2-yl)-9H-purine, 2-fluoro-6-[(3-méthylbut-2-én-1-yl)amino]-9-(tétrahydrofuran-2-yl)-9H-purine, 2-fluoro-6-(E)-[(4-hydroxy-3-méthylbut-2-én-1-yl)amino]-9-(tétrahydrofuran-2-yl)-9H-purine, 2-fluoro-6-(Z)-[(4-hydroxy-3-méthylbut-2-én-1-yl)amino]-9-(tétrahydrofuran-2-yl)-9H-purine, 2-fluoro-6-[(4-hydroxy-3-méthylbutyl)amino]-9-(tétrahydrofuran-2-yl)-9H-purine, 2-fluoro-6-(3-méthoxyfurfurylamino)-9-(tétrahydro-2H-pyran-2-yl)-9H-purine; 2-fluoro-6-benzylamino-9-(tétrahydro-2H-pyran-2-yl)-9H-purine, 2-fluoro-6-[(2-hydroxybenzyl)amino]-9-(tétrahydro-2H-pyran-2-yl)-9H-purine, 2-fluoro-6-[(3-hydroxybenzyl)amino]-9-(tétrahydro-2H-pyran-2-yl)-9H-purine, 2-fluoro-6-[(4-hydroxybenzyl)amino]-9-(tétrahydro-2H-pyran-2-yl)-9H-purine, 2-fluoro-6-[(2-méthoxybenzyl)amino]-9-(tétrahydro-2H-pyran-2-yl)-9H-purine, 2-fluoro-6-[(3-méthoxybenzyl)amino]-9-(tétrahydro-2H-pyran-2-yl)-9H-purine, 2-fluoro-6-[(4-méthoxybenzyl)amino]-9-(tétrahydro-2H-pyran-2-yl)-9H-purine, 2-fluoro-6-[(2-fluorobenzyl)amino]-9-(tétrahydro-2H-pyran-2-yl)-9H-purine, 2-fluoro-6-[(3-fluorobenzyl)amino]-9-(tétrahydro-2H-pyran-2-yl)-9H-purine, 2-fluoro-6-[(4-fluorobenzyl)amino]-9-(tétrahydro-2H-pyran-2-yl)-9H-purine, 2-fluoro-6-[(2-chlorobenzyl)amino]-9-(tétrahydro-2H-pyran-2-yl)-9H-purine, 2-fluoro-6-[(3-chlorobenzyl)amino]-9-(tétrahydro-2H-pyran-2-yl)-9H-purine, 2-fluoro-6-(3-méthoxyanilino)-9-(tétrahydro-2H-pyran-2-yl)-9H-purine, 2-fluoro-6-[(3-méthylbut-2-én-1-yl)amino]-9-(tétrahydro-2H-pyran-2-yl)-9H-purine, 2-fluoro-6-(E)-[(4-hydroxy-3-méthylbut-2-én-1-yl)amino]-9-(tétrahydro-2H-pyran-2-yl)-9H-purine, 2-fluoro-6-(Z)-[(4-hydroxy-3-méthylbut-2-én-1-yl)amino]-9-(tétrahydro-2H-pyran-2-yl)-9H-purine, 2-fluoro-6-[(4-hydroxy-3-méthylbutyl)amino]-9-(tétrahydro-2H-pyran-2-yl)-9H-purine, 2-fluoro-6-(3-méthoxyfurfurylamino)-9-(oxepan-2-yl)-9H-purine, 2-fluoro-6-benzylamino-9-(oxepan-2-yl)-9H-purine; 2-fluoro-6-[(2-hydroxybenzyl)amino]-9-(oxepan-2-yl)-9H-purine, 2-fluoro-6-[(3-hydroxybenzyl)amino]-9-(oxepan-2-yl)-9H-purine, 2-fluoro-6-[(4-hydroxybenzyl)amino]-9-(oxepan-2-yl)-9H-purine; 2-fluoro-6-[(2-méthoxybenzyl)amino]-9-(oxepan-2-yl)-9H-purine; 2-fluoro-6-[(3-méthoxybenzyl)amino]-9-(oxepan-2-yl)-9H-purine, 2-fluoro-6-[(4-méthoxybenzyl)amino]-9-(oxepan-2-yl)-9H-purine, 2-fluoro-6-[(2-fluorobenzyl)amino]-9-(oxepan-2-yl)-9H-purine; 2-fluoro-6-[(3-fluorobenzyl)amino]-9-(oxepan-2-yl)-9H-purine, 2-fluoro-6-[(4-fluorobenzyl)amino]-9-(oxepan-2-yl)-9H-purine, 2-fluoro-6-[(2-chlorobenzyl)amino]-9-(oxepan-2-yl)-9H-purine, 2-fluoro-6-[(3-chlorobenzyl)amino]-9-(oxepan-2-yl)-9H-purine; 2-fluoro-6-(3-méthoxyanilino)-9-(oxepan-2-yl)-9H-purine, 2-fluoro-6-[(3-méthylbut-2-én-1-yl)amino]-9-(oxepan-2-yl)-9H-purine, 2-fluoro-6-(E)-[(4-hydroxy-3-méthylbut-2-én-1-yl)amino]-9-(oxepan-2-yl)-9H-purine, 2-fluoro-6-(Z)-[(4-hydroxy-3-méthylbut-2-én-1-yl)amino]-9-(oxepan-2-yl)-9H-purine, 2-fluoro-6-[(4-hydroxy-3-méthylbutyl)amino]-9-(oxepan-2-yl)-9H-purine et leurs sels acceptables en agriculture.

5. Utilisation du dérivé de 2-fluoro-N6-substitué-9-(2-oxacycloalkyl)-9H-purine-6-amine selon l'une quelconque des revendications 1 à 4 comme agent stimulant la division cellulaire, prodifférenciatif, antisénescent, stimulant la charge du phloème, la formation des pousses et la croissance racinaire chez les plantes, en particulier les plantes cultivées.

6. Utilisation du dérivé de 2-fluoro-N6-substitué-9-(2-oxacycloalkyl)-9H-purine-6-amine selon l'une quelconque des revendications 1 à 4 en agriculture et en biotechnologie, pour inhiber, retarder ou réduire les effets néfastes de la sénescence, améliorer l'aspect général et l'état des plantes, en particulier des cellules épidermiques et mésophylliennes, et pour inhiber, retarder ou réduire le jaunissement, les pertes de chloroplastes et/ou de chlorophylle et les pertes foliaires.

7. Utilisation du dérivé de 2-fluoro-N6-substitué-9-(2-oxacycloalkyl)-9H-purine-6-amine selon l'une quelconque des revendications 1 à 4 comme régulateur de croissance en agriculture, pour augmenter la production végétale, accélérer le remplissage des grains, augmenter la taille des grains et des fruits des plantes et des champignons, raccourcir la période de germination des graines, augmenter le rendement et la qualité des produits agricoles, favoriser la croissance des racines et des pousses, et/ou accroître la résistance au stress environnementaux.

8. Utilisation du dérivé de 2-fluoro-N6-substitué-9-(2-oxacycloalkyl)-9H-purine-6-amine selon l'une quelconque des revendications 1 à 4 dans les cultures de tissus végétaux comme régulateur de croissance, pour stimuler la prolifération et la morphogenèse et/ou stimuler la formation des racines.

9. Utilisation du dérivé de 2-fluoro-N6-substitué-9-(2-oxacycloalkyl)-9H-purine-6-amine selon l'une quelconque des revendications 1 à 4 dans des cultures de tissus végétaux, pour la modulation de la micropropagation, de la culture méristématique, de l'embryogenèse somatique, de l'androgenèse, de la gynogenèse, des cultures en suspension et des cultures de protoplastes.

10. Composition, **caractérisée en ce qu'**elle comprend un ou plusieurs dérivés de 2-fluoro-N6-substitué-9-(2-oxacycloalkyl)-9H-purine-6-amine selon l'une quelconque des revendications 1 à 4, et au moins une substance auxiliaire acceptable en agriculture.
